# EUROPEAN PATENT APPLICATION

(11) **EP 4 534 557 A1**
(43) Date of publication of application: **09.04.2025**
(21) Application number: 23811103.3
(22) Date of filing: 24.05.2023
(51) Int. Cl.: C07K 16/28, C12N 15/13, C12N 15/63, A61K 39/395, A61P 3/10, A61P 37/02, A61P 29/00, A61P 19/02

(54) **ANTI-BDCA2 ANTIBODY AND USE THEREOF**

(30) Priority: 25.05.2022 CN 202210599888; 26.10.2022 CN 202211318610
(71) Applicant: Duality Biologics (Suzhou) Co., Ltd., Suzhou, Jiangsu 215000 (CN)
(72) Inventor: LI, Xi, Shanghai 201204 (CN); HUA, Haiqing, Shanghai 201204 (CN); ZHU, Zhongyuan, Shanghai 201204 (CN); LI, Jian, Shanghai 201204 (CN)
(74) Representative: De Clercq & Partners
(86) International application number: PCT/CN2023/096076
(87) International publication number: WO 2023/227033

(57) **Abstract**

The invention provides antibodies or antigen binding fragments thereof that specifically bind to BDCA2, as well as compositions comprising the same. Also provided are nucleic acid molecules encoding the antibodies or antigen binding fragments of the invention, vectors and host cells for expressing the antibodies or antigen binding fragments of the invention, and therapeutic and diagnostic methods and uses of the antibodies or antigen binding fragments of the invention.

## Description

### Field of the Invention

The invention relates to the field of monoclonal antibodies and/or engineered antibodies. Specifically, the invention provides an antibody or antigen-binding fragment thereof specifically bind to BDCA2 and a composition comprising the antibody or antigen-binding fragment thereof. The invention further provides a nucleic acid molecule encoding the antibody or antigen-binding fragment thereof of the invention, a vector and a host cell for expressing the antibody or antigen-binding fragments thereof of the invention, and a treatment and diagnostic/detection method and use of the antibody or antigen-binding fragments thereof of the invention.

### Background of the Invention

As the medical researches progress, variety of diseases have been found to be associated with dysfunctions of immune system, and research into immune system has helped to further understand the pathogenesis and seek more optimal treatment options.

The immune system is a highly complex system composed of many cell types, including but not limited to T cells, B cells, natural killer cells, antigen presenting cells, dendritic cells, monocytes and macrophages. The interactions and responses of these cells are under control of a complex and subtle system. The cells utilize activation and inhibition mechanisms as well as feedback loops to keep the control of reaction, and prevent the negative consequences caused by uncontrolled immune response (e.g., autoimmune diseases) as much as possible. There is the involvement of a large number of signaling molecules and their interactions among the multiple signaling pathways related to immunity.

Blood dendritic cell antigen 2 (BDCA2) is a C-type lectin expressed on human plasmacytoid dendritic cell (plc.) (Dzionek et al., J. Immunol.,165:6037-6046(2000)). BDCA2 consists of a single extracellular carbohydrate recognition domain (CRD) (which belongs to the type II C-type lectin group) at its C-terminus, a transmembrane region from asparagine residue at position 45 to isoleucine residue at position 213, and a short cytoplasmic tail at its N-terminus (which does not harbor a signaling motif). BDCA2 transmits intracellular signals through an associated transmembrane adaptor, the FcεRIγ, and induces a B cell receptor (BCR)-like signaling cascade.

### Summary of the Invention

The invention provides an antibody or antigen-binding fragment thereof specifically bind to BDCA2, which has advantages of, high affinity and high specificity to human and cynomolgus BDCA2, and the like. The anti-BDCA2 antibody or antigen-binding fragment thereof provided by the invention can be used, for example, in the treatment of inflammatory conditions/diseases, either as an independent mono therapy or in combination with other therapies and/or other agents.

The anti-BDCA2 antibodies described herein inhibit the production and/or secretion of inflammatory cytokines and chemokines by human plasmacytoid dendritic cells (pDC). In addition, the anti-BDCA2 antibodies described herein may down-regulate the levels of CD32a and/or CD62L on the surface of pDCs. In addition, the anti-BDCA2 antibodies of the disclosure are capable of mediating the internalization of BDCA2 from the surface of pDCs. In addition, the anti-BDCA2 antibodies described herein may be used to deplete pDCs by ADCC or CDC and may be used to treat or prevent immune disorders, e.g., inflammatory disorders and autoimmune disorders.

In one aspect, the invention provides an anti-BDCA2 antibody or antigen-binding fragment thereof, wherein the anti-BDCA2 antibody or antigen-binding fragment thereof comprises a heavy chain variable region and/or a light chain variable region:
The heavy chain variable region comprises:
(I) HCDR1, HCDR2, and HCDR3 having amino acid sequences as set forth in SEQ ID NO: 11, SEQ ID NO: 12, and SEQ ID NO: 13, respectively; or HCDR1, HCDR2 and HCDR3 having 1, 2 or 3 amino acid differences from the amino acid sequences as set forth in SEQ ID NO: 11, SEQ ID NO: 12 and SEQ ID NO: 13, respectively; or
(II) HCDR1, HCDR2 and HCDR3 having amino acid sequences as set forth in SEQ ID NO: 19, SEQ ID NO: 20 and SEQ ID NO: 21, respectively; or HCDR1, HCDR2 and HCDR3 having 1, 2 or 3 amino acid differences from the amino acid sequences as set forth in SEQ ID NO: 19, SEQ ID NO: 20 and SEQ ID NO: 21, respectively; or
(III) HCDR1, HCDR2 and HCDR3 having amino acid sequences as set forth in SEQ ID NO: 25, SEQ ID NO: 26 and SEQ ID NO: 27, respectively; or HCDR1, HCDR2, and HCDR3 having 1, 2, or 3 amino acid differences from the amino acid sequences as set forth in SEQ ID NO: 25, SEQ ID NO: 26, and SEQ ID NO: 27, respectively; or
(IV) HCDR1, HCDR2 and HCDR3 having amino acid sequences as set forth in SEQ ID NO: 31, SEQ ID NO: 32 and SEQ ID NO: 33, respectively; or HCDR1, HCDR2, and HCDR3 having 1, 2, or 3 amino acid differences from the amino acid sequences as set forth in SEQ ID NO: 31, SEQ ID NO: 32, and SEQ ID NO: 33, respectively; or
(V) HCDR1, HCDR2 and HCDR3 having amino acid sequences as set forth in SEQ ID NO: 38, SEQ ID NO: 39 and SEQ ID NO: 40, respectively; or HCDR1, HCDR2, and HCDR3 having 1, 2, or 3 amino acid differences from the amino acid sequences as set forth in SEQ ID NO: 38, SEQ ID NO: 39, and SEQ ID NO: 40, respectively; or
(VI) HCDR1, HCDR2 and HCDR3 having amino acid sequences as set forth in SEQ ID NO: 31, SEQ ID NO: 46 and SEQ ID NO: 47, respectively; or HCDR1, HCDR2, and HCDR3 having 1, 2, or 3 amino acid differences from the amino acid sequences as set forth in SEQ ID NO: 31, SEQ ID NO: 46, and SEQ ID NO: 47, respectively; or
(VII) HCDR1, HCDR2 and HCDR3 having amino acid sequences as set forth in SEQ ID NO: 31, SEQ ID NO: 50 and SEQ ID NO: 47, respectively; or HCDR1, HCDR2, and HCDR3 having 1, 2, or 3 amino acid differences from the amino acid sequences as set forth in SEQ ID NO: 31, SEQ ID NO: 50, and SEQ ID NO: 47, respectively; or
(VIII) HCDR1, HCDR2 and HCDR3 having amino acid sequences as set forth in SEQ ID NO: 52, SEQ ID NO: 39 and SEQ ID NO: 53, respectively; or HCDR1, HCDR2, and HCDR3 having 1, 2, or 3 amino acid differences from the amino acid sequences as set forth in SEQ ID NO: 52, SEQ ID NO: 39, and SEQ ID NO: 53, respectively; or
(IX) HCDR1, HCDR2 and HCDR3 having amino acid sequences as set forth in SEQ ID NO: 25, SEQ ID NO: 26 and SEQ ID NO: 37, respectively; or HCDR1, HCDR2, and HCDR3 having 1, 2, or 3 amino acid differences from the amino acid sequences as set forth in SEQ ID NO: 25, SEQ ID NO: 26, and SEQ ID NO: 37, respectively; and/or

The light chain variable region comprises:
(I) LCDR1, LCDR2, and LCDR3 having amino acid sequences as set forth in SEQ ID NO: 14, SEQ ID NO: 15, and SEQ ID NO: 16, respectively; or LCDR1, LCDR2 and LCDR3 having 1, 2 or 3 amino acid differences from the amino acid sequences as set forth in SEQ ID NO: 14, SEQ ID NO: 15 and SEQ ID NO: 16, respectively; or
(II) LCDR1, LCDR2, and LCDR3 having amino acid sequences as set forth in SEQ ID NO: 22, SEQ ID NO: 23, and SEQ ID NO: 24, respectively; or LCDR1, LCDR2 and LCDR3 having 1, 2 or 3 amino acid differences from the amino acid sequences as set forth in SEQ ID NO: 22, SEQ ID NO: 23 and SEQ ID NO: 24, respectively; or
(III) LCDR1, LCDR2, and LCDR3 having amino acid sequences as set forth in SEQ ID NO: 28, SEQ ID NO: 29, and SEQ ID NO: 30, respectively; or LCDR1, LCDR2 and LCDR3 having 1, 2 or 3 amino acid differences from the amino acid sequences as set forth in SEQ ID NO: 28, SEQ ID NO: 29 and SEQ ID NO: 30, respectively; or
(IV) LCDR1, LCDR2, and LCDR3 having amino acid sequences as set forth in SEQ ID NO: 34, SEQ ID NO: 35, and SEQ ID NO: 36, respectively; or LCDR1, LCDR2 and LCDR3 having 1, 2 or 3 amino acid differences from the amino acid sequences as set forth in SEQ ID NO: 34, SEQ ID NO: 35 and SEQ ID NO: 36, respectively; or
(V) LCDR1, LCDR2, and LCDR3 having amino acid sequences as set forth in SEQ ID NO: 41, SEQ ID NO: 15, and SEQ ID NO: 42, respectively; or LCDR1, LCDR2 and LCDR3 having 1, 2 or 3 amino acid differences from the amino acid sequences as set forth in SEQ ID NO: 41, SEQ ID NO: 15 and SEQ ID NO: 42, respectively; or
(VI) LCDR1, LCDR2, and LCDR3 having amino acid sequences as set forth in SEQ ID NO: 34, SEQ ID NO: 48, and SEQ ID NO: 49, respectively; or LCDR1, LCDR2 and LCDR3 having 1, 2 or 3 amino acid differences from the amino acid sequences as set forth in SEQ ID NO: 34, SEQ ID NO: 48 and SEQ ID NO: 49, respectively; or
(VII) LCDR1, LCDR2, and LCDR3 having amino acid sequences as set forth in SEQ ID NO: 34, SEQ ID NO: 51, and SEQ ID NO: 49 respectively; or LCDR1, LCDR2 and LCDR3 having 1, 2 or 3 amino acid differences from the amino acid sequences as set forth in SEQ ID NO: 34, SEQ ID NO: 51 and SEQ ID NO: 49, respectively; or
(VIII) LCDR1, LCDR2, and LCDR3 having amino acid sequences as set forth in SEQ ID NO: 54, SEQ ID NO: 55, and SEQ ID NO: 42, respectively; or LCDR1, LCDR2 and LCDR3 having 1, 2 or 3 amino acid differences from the amino acid sequences as set forth in SEQ ID NO: 54, SEQ ID NO: 55 and SEQ ID NO: 42, respectively; or
(IX) LCDR1, LCDR2, and LCDR3 having amino acid sequences as set forth in SEQ ID NO: 86, SEQ ID NO: 15, and SEQ ID NO: 16, respectively; or LCDR1, LCDR2 and LCDR3 having 1, 2 or 3 amino acid differences from the amino acid sequences as set forth in SEQ ID NO: 86, SEQ ID NO: 15 and SEQ ID NO: 16, respectively.

In some embodiments, the antibody or antigen-binding fragment thereof of the invention, wherein the antibody or antigen-binding fragment thereof comprises:
(I) a heavy chain variable region comprising HCDR1, HCDR2, and HCDR3 having amino acid sequences as set forth in SEQ ID NO: 52, SEQ ID NO: 39, and SEQ ID NO: 53, respectively; and a light chain variable region comprising LCDR1, LCDR2, and LCDR3 having amino acid sequences as set forth in SEQ ID NO: 54, SEQ ID NO: 55, and SEQ ID NO: 42, respectively; or
(II) a heavy chain variable region comprising HCDR1, HCDR2, and HCDR3 having amino acid sequences as set forth in SEQ ID NO: 19, SEQ ID NO: 20, and SEQ ID NO: 21, respectively; and a light chain variable region comprising LCDR1, LCDR2, and LCDR3 having amino acid sequences as set forth in SEQ ID NO: 22, SEQ ID NO: 23, and SEQ ID NO: 24, respectively; or
(III) a heavy chain variable region comprising HCDR1 and HCDR2 having amino acid sequences as set forth in SEQ ID NO: 25, SEQ ID NO: 26, respectively, and HCDR3 as set forth in SEQ ID NO: 27 or SEQ ID NO: 37, respectively; and a light chain variable region comprising LCDR1, LCDR2, and LCDR3 having amino acid sequences as set forth in SEQ ID NO: 28, SEQ ID NO: 29, and SEQ ID NO: 30, respectively; or
(IV) a heavy chain variable region comprising HCDR1, HCDR2, and HCDR3 having amino acid sequences as set forth in SEQ ID NO: 31, SEQ ID NO: 32, and SEQ ID NO: 33, respectively; and a light chain variable region comprising LCDR1, LCDR2, and LCDR3 having amino acid sequences as set forth in SEQ ID NO: 34, SEQ ID NO: 35, and SEQ ID NO: 36, respectively; or
(V) a heavy chain variable region comprising HCDR1, HCDR2, and HCDR3 having amino acid sequences as set forth in SEQ ID NO: 38, SEQ ID NO: 39, and SEQ ID NO: 40, respectively; and a light chain variable region comprising LCDR1, LCDR2, and LCDR3 having amino acid sequences as set forth in SEQ ID NO: 41, SEQ ID NO: 15, and SEQ ID NO: 42, respectively; or
(VI) a heavy chain variable region comprising HCDR1 having amino acid sequences as set forth in SEQ ID NO: 31, HCDR2 as set forth in SEQ ID NO: 46 or SEQ ID NO: 50, and HCDR3 as set forth in SEQ ID NO:47, respectively; and a light chain variable region comprising LCDR1 having amino acid sequences as set forth in SEQ ID NO: 34, LCDR2 as set forth in SEQ ID NO: 48 or SEQ ID NO: 51, and LCDR3 as set forth in SEQ ID NO:49, respectively; or
(VII) a heavy chain variable region comprising HCDR1, HCDR2, and HCDR3 having amino acid sequences as set forth in SEQ ID NO: 11, SEQ ID NO: 12, and SEQ ID NO: 13, respectively; and a light chain variable region comprising LCDR1 having amino acid sequences as set forth in SEQ ID NO: 14 or SEQ ID NO:86, LCDR2 as set forth in SEQ ID NO: 15, and LCDR3 as set forth in SEQ ID NO: 16, respectively.

In some embodiments, the antibody or antigen-binding fragment thereof of the invention, wherein the antibody or antigen-binding fragment thereof comprises a heavy chain variable region and a light chain variable region:
(I) the heavy chain variable region comprises the amino acid sequence as set forth in SEQ ID NO: 61, or comprises an amino acid sequence having at least 95%, 96%, 97%, 98%, or 99% sequence identity to the amino acid sequence as set forth in SEQ ID NO: 61; and the light chain variable region comprising the amino acid sequence as set forth in SEQ ID NO: 62, or comprising an amino acid sequence having at least 95%, 96%, 97%, 98%, or 99% sequence identity to the amino acid sequence as set forth in SEQ ID NO: 62; or
(II) the heavy chain variable region comprises the amino acid sequence as set forth in SEQ ID NO: 65, or comprises an amino acid sequence having at least 95%, 96%, 97%, 98%, or 99% sequence identity to the amino acid sequence as set forth in SEQ ID NO: 65; and the light chain variable region comprising the amino acid sequence as set forth in SEQ ID NO: 66, or comprising an amino acid sequence having at least 95%, 96%, 97%, 98%, or 99% sequence identity to the amino acid sequence as set forth in SEQ ID NO: 66; or
(III) the heavy chain variable region comprises the amino acid sequence as set forth in SEQ ID NO: 67, or comprises an amino acid sequence having at least 95%, 96%, 97%, 98%, or 99% sequence identity to the amino acid sequence as set forth in SEQ ID NO: 67; and the light chain variable region comprising the amino acid sequence as set forth in SEQ ID NO: 68, or comprising an amino acid sequence having at least 95%, 96%, 97%, 98%, or 99% sequence identity to the amino acid sequence as set forth in SEQ ID NO: 68; or
(IV) the heavy chain variable region comprises the amino acid sequence as set forth in SEQ ID NO: 69, or comprises an amino acid sequence having at least 95%, 96%, 97%, 98%, or 99% sequence identity to the amino acid sequence as set forth in SEQ ID NO: 69; and the light chain variable region comprising the amino acid sequence as set forth in SEQ ID NO: 70, or comprising an amino acid sequence having at least 95%, 96%, 97%, 98%, or 99% sequence identity to the amino acid sequence as set forth in SEQ ID NO: 70; or
(V) the heavy chain variable region comprises the amino acid sequence as set forth in SEQ ID NO: 71, or comprises an amino acid sequence having at least 95%, 96%, 97%, 98%, or 99% sequence identity to the amino acid sequence as set forth in SEQ ID NO: 71; and the light chain variable region comprising the amino acid sequence as set forth in SEQ ID NO: 68, or comprising an amino acid sequence having at least 95%, 96%, 97%, 98%, or 99% sequence identity to the amino acid sequence as set forth in SEQ ID NO: 68; or
(VI) the heavy chain variable region comprises the amino acid sequence as set forth in SEQ ID NO: 72, or comprises an amino acid sequence having at least 95%, 96%, 97%, 98%, or 99% sequence identity to the amino acid sequence as set forth in SEQ ID NO: 72; and the light chain variable region comprising the amino acid sequence as set forth in SEQ ID NO: 73, or comprising an amino acid sequence having at least 95%, 96%, 97%, 98%, or 99% sequence identity to the amino acid sequence as set forth in SEQ ID NO: 73; or
(VII) the heavy chain variable region comprises the amino acid sequence as set forth in SEQ ID NO: 76, or comprises an amino acid sequence having at least 95%, 96%, 97%, 98%, or 99% sequence identity to the amino acid sequence as set forth in SEQ ID NO: 76; and the light chain variable region comprising the amino acid sequence as set forth in SEQ ID NO: 77, or comprising an amino acid sequence having at least 95%, 96%, 97%, 98%, or 99% sequence identity to the amino acid sequence as set forth in SEQ ID NO: 77; or
(VIII) the heavy chain variable region comprises the amino acid sequence as set forth in SEQ ID NO: 80, or comprises an amino acid sequence having at least 95%, 96%, 97%, 98%, or 99% sequence identity to the amino acid sequence as set forth in SEQ ID NO: 80; and the light chain variable region comprising the amino acid sequence as set forth in SEQ ID NO: 79, or comprising an amino acid sequence having at least 95%, 96%, 97%, 98%, or 99% sequence identity to the amino acid sequence as set forth in SEQ ID NO: 79; or
(IX) the heavy chain variable region comprises the amino acid sequence as set forth in SEQ ID NO: 82, or comprises an amino acid sequence having at least 95%, 96%, 97%, 98%, or 99% sequence identity to the amino acid sequence as set forth in SEQ ID NO: 82; and the light chain variable region comprising the amino acid sequence as set forth in SEQ ID NO: 83, or comprising an amino acid sequence having at least 95%, 96%, 97%, 98%, or 99% sequence identity to the amino acid sequence as set forth in SEQ ID NO: 83.

In some embodiments, the antibody or antigen-binding fragment thereof of the invention, wherein the antibody or antigen-binding fragment thereof comprises:
(I) a heavy chain variable region comprising the amino acid sequence as set forth in any one of SEQ ID NOs: 87-110, or comprising an amino acid sequence having at least 95%, 96%, 97%, 98%, or 99% sequence identity to the amino acid sequence as set forth in any one of SEQ ID NOs: 87-110; and a light chain variable region comprising the amino acid sequence as set forth in any one of SEQ ID NOs: 135-158, or comprising an amino acid sequence having at least 95%, 96%, 97%, 98%, or 99% sequence identity to an amino acid sequence as set forth in any one of SEQ ID NOs: 135-158; or
(II) a heavy chain variable region comprising the amino acid sequence as set forth in any one of SEQ ID NOs: 111-134, or comprising an amino acid sequence having at least 95%, 96%, 97%, 98%, or 99% sequence identity to the amino acid sequence as set forth in any one of SEQ ID NOs: 111-134; and a light chain variable region comprising the amino acid sequence as set forth in any one of SEQ ID NOs: 159-182, or comprising an amino acid sequence having at least 95%, 96%, 97%, 98%, or 99% sequence identity to an amino acid sequence as set forth in any one of SEQ ID NOs: 159-182; or
(III) a heavy chain variable region comprising the amino acid sequence as set forth in SEQ ID NO: 89, or comprising an amino acid sequence having at least 95%, 96%, 97%, 98%, or 99% sequence identity to the amino acid sequence as set forth in SEQ ID NO: 89; and a light chain variable region comprising the amino acid sequence as set forth in SEQ ID NO: 137, or comprising an amino acid sequence having at least 95%, 96%, 97%, 98%, or 99% sequence identity to an amino acid sequence as set forth in SEQ ID NO: 137; or
(IV) a heavy chain variable region comprising the amino acid sequence as set forth in SEQ ID NO: 106, or comprising an amino acid sequence having at least 95%, 96%, 97%, 98%, or 99% sequence identity to the amino acid sequence as set forth in SEQ ID NO: 106; and a light chain variable region comprising the amino acid sequence as set forth in SEQ ID NO: 154, or comprising an amino acid sequence having at least 95%, 96%, 97%, 98%, or 99% sequence identity to an amino acid sequence as set forth in SEQ ID NO: 154; or
(V) a heavy chain variable region comprising the amino acid sequence as set forth in SEQ ID NO: 114, or comprising an amino acid sequence having at least 95%, 96%, 97%, 98%, or 99% sequence identity to the amino acid sequence as set forth in SEQ ID NO: 114; and a light chain variable region comprising the amino acid sequence as set forth in SEQ ID NO: 162, or comprising an amino acid sequence having at least 95%, 96%, 97%, 98%, or 99% sequence identity to an amino acid sequence as set forth in SEQ ID NO: 162.

In some embodiments, the antibody or antigen-binding fragment thereof of the invention, wherein the antibody or antigen-binding fragment thereof comprises:
(I) a heavy chain variable region comprising the amino acid sequence as set forth in any one of SEQ ID NOs: 87-110 and a light chain variable region comprising the amino acid sequence as set forth in any one of SEQ ID NOs: 135-158; or
(II) a heavy chain variable region comprising the amino acid sequence as set forth in any one of SEQ ID NOs: 111-134 and a light chain variable region comprising the amino acid sequence as set forth in any one of SEQ ID NOs: 159-182; or
(III) a heavy chain variable region comprising the amino acid sequence as set forth in SEQ ID NO: 89 and a light chain variable region comprising the amino acid sequence as set forth in SEQ ID NO: 137; or
(IV) a heavy chain variable region comprising the amino acid sequence as set forth in SEQ ID NO: 106 and a light chain variable region comprising the amino acid sequence as set forth in SEQ ID NO: 154; or
(V) a heavy chain variable region comprising the amino acid sequence as set forth in SEQ ID NO: 114 and a light chain variable region comprising the amino acid sequence as set forth in SEQ ID NO: 162.

In some embodiments, the antibody or antigen-binding fragment thereof of the invention, wherein the antibody is selected from the group consisting of a mouse antibody, a chimeric antibody, a humanized antibody, or a fully human antibody. In some embodiments, the antibody or antigen-binding fragment thereof of the invention, wherein the antibody is a humanized antibody.

In some embodiments, the antibody or antigen-binding fragment thereof of the invention, wherein the antigen-binding fragment is selected from the group consisting of Fab, Fab', F(ab')2, Fv, scFv and sdAb.

In some embodiments, the antibody or antigen-binding fragment thereof of the invention, wherein the antibody or antigen-binding fragment thereof is of any IgG subtype, such as IgG1, IgG2, IgG3 or IgG4, preferably of IgG1 subtype.

In some embodiments, the antibody or antigen-binding fragment thereof of the invention, wherein the antibody comprises or is consisted of the sequence:
(I) a heavy chain having amino acid sequence as set forth in SEQ ID NO: 183 or having at least 95%, 96%, 97%, 98% or 99% sequence identity thereto, and a light chain having amino acid sequence as set forth in SEQ ID NO: 186 or having at least 95%, 96%, 97%, 98% or 99% sequence identity thereto; or
(II) a heavy chain having amino acid sequence as set forth in SEQ ID NO: 184 or having at least 95%, 96%, 97%, 98% or 99% sequence identity thereto, and a light chain having amino acid sequence as set forth in SEQ ID NO: 187 or having at least 95%, 96%, 97%, 98% or 99% sequence identity thereto; or
(III) a heavy chain having amino acid sequence as set forth in SEQ ID NO: 185 or having at least 95%, 96%, 97%, 98% or 99% sequence identity thereto, and a light chain having amino acid sequence as set forth in SEQ ID NO: 188 or having at least 95%, 96%, 97%, 98% or 99% sequence identity thereto.

In some embodiments, the antibody or antigen-binding fragment thereof of the invention, wherein the antibody comprises or is consisted of the sequence:
(I) a heavy chain having amino acid sequence as set forth in SEQ ID NO: 183 and a light chain having amino acid sequence as set forth in SEQ ID NO: 186; or
(II) a heavy chain having amino acid sequence as set forth in SEQ ID NO: 184 and a light chain having amino acid sequence as set forth in SEQ ID NO: 187; or
(III) a heavy chain having amino acid sequence as set forth in SEQ ID NO: 185 and a light chain having amino acid sequence as set forth in SEQ ID NO: 188.

In some embodiments, the antibody or antigen-binding fragment thereof of the invention, wherein the antibody comprises or is consisted of the sequence:
(I) a heavy chain having 1, 2, or 3 amino acid differences from amino acid sequence as set forth in SEQ ID NO: 183, and a light chain having amino acid sequence as set forth in SEQ ID NO: 186; or
(II) a heavy chain having 1, 2, or 3 amino acid differences from amino acid sequence as set forth in SEQ ID NO: 184, and a light chain having amino acid sequence as set forth in SEQ ID NO: 187; or
(III) a heavy chain having 1, 2, or 3 amino acid differences from amino acid sequence as set forth in SEQ ID NO: 185, and a light chain having amino acid sequence as set forth in SEQ ID NO: 188.

In some embodiments, the antibody or antigen-binding fragment thereof of the invention, wherein the antibody comprises or is consisted of the sequence:
(I) a heavy chain having amino acid sequence as set forth in SEQ ID NO: 191, SEQ ID NO: 192 or SEQ ID NO: 193 and a light chain having amino acid sequence as set forth in SEQ ID NO: 186; or
(II) a heavy chain having amino acid sequence as set forth in SEQ ID NO: 194, SEQ ID NO: 195 or SEQ ID NO: 196 and a light chain having amino acid sequence as set forth in SEQ ID NO: 187.

In some embodiments, the antibody or antigen-binding fragment thereof of the invention is a mouse antibody, a chimeric antibody, a humanized antibody, or a fully human antibody, or antigen-binding fragment thereof.

In some embodiments, the antibody or antigen-binding fragment thereof of the invention, wherein the antigen-binding fragment is Fab, Fab', F(ab')2, Fv, scFv and sdAb.

In some embodiments, the antibody or antigen-binding fragment thereof of the invention, wherein the antibody or antigen-binding fragment thereof is of any IgG subtype, such as IgG1, IgG2, IgG3 or IgG4, preferably of IgG1 subtype.

In another aspect, the invention provides an isolated anti-BDCA2 antibody or antigen-binding fragment thereof having one or more of the following properties:
(1) Binding to the same epitope, or a fully or partially overlapping epitope of human BDCA2 protein as that of any one of the anti-BDCA2 antibodies or antigen-binding fragments thereof of the invention;
(2) Competition for binding to an epitope of human BDCA2 protein with any one of the anti-BDCA2 antibodies or antigen-binding fragments thereof of the invention.

In another aspect, the invention provides an isolated anti-BDCA2 antibody or antigen-binding fragment thereof having one or more of the following properties:
(1) Binding to human/cynomolgus BDCA2 protein with high affinity, e.g., exhibiting a KD value of 1.0×10⁻⁶ M or less, a KD value of 5.0×10⁻⁷ M or less, a KD value of 2.5×10⁻⁷ M or less, a KD value of 1.0×10⁻⁷ M or less, a KD value of 5.0×10⁻⁸ M or less, a KD value of 2.5×10⁻⁸ M or less, a KD value of 1.0×10⁻⁸ M or less, a KD value of 5.0×10⁻⁹ M or less, a KD value of 2.5×10⁻⁹ M or less, a KD value of 1.0×10⁻⁹ M or less, a KD value of 5.0×10⁻¹⁰ M or less, a KD value of 2.5×10⁻¹⁰ M or less, a KD value of 1.0×10⁻¹⁰ M or less, a KD value of 1.0×10⁻¹⁰ M or less, a KD value of 5.0×10⁻¹⁰M or less;
(2) Inhibiting stimulation of CpG-A to the release of cytokine IFNα from human PBMC cells, e.g., such that the production of IFNα by target cells is reduced by at least 10%, at least 20%, at least 30%, at least 40%, at least 50%, at least 60%, at least 70%, at least 80%, at least 90%, or at least 95%;
(3) Promoting the internalization of BDCA2 from cell surface upon binding to BDCA2, e.g., such that at least 10%, at least 20%, at least 30%, at least 40%, at least 50%, at least 60%, at least 70%, at least 80%, at least 90%, or at least 95% of BDCA2 molecules on cell surface are internalized.

In some embodiments, the antibody of the invention is a monoclonal antibody.

The invention also provides a multi-specific antibody comprising a light chain variable region and a heavy chain variable region of the antibody or antigen-binding fragment thereof described herein.

The invention also provides a single chain antibody comprising the light chain variable region and the heavy chain variable region of the antibody or antigen-binding fragment thereof described herein.

The invention also provides an immunoconjugate comprising an antibody or antigen-binding fragment thereof as described herein conjugated to a therapeutic or diagnostic agent, preferably the therapeutic or diagnostic agent is an anti-inflammatory drug or an immunosuppressant.

In a further aspect, the invention provides a polynucleotide molecule encoding an anti-BDCA2 antibody or antigen-binding fragment thereof described herein.

In a further aspect, the invention provides an expression vector comprising the polynucleotide molecule described herein, preferably the vector is a eukaryotic expression vector.

In a further aspect, the invention provides a host cell comprising the polynucleotide molecule described herein or the expression vector described herein, preferably the host cell is a eukaryotic cell, more preferably a mammalian cell.

In yet another aspect, the invention provides a method of making an anti-BDCA2 antibody or antigen-binding fragment thereof described herein, wherein the method comprises expressing the antibody or antigen-binding fragment thereof in a host cell described herein under conditions suitable for expression of the antibody or antigen-binding fragment thereof, and recovering the expressed antibody or antigen-binding fragment thereof from the host cell.

In a further aspect, the invention provides a pharmaceutical composition comprising an anti-BDCA2 antibody or antigen-binding fragment thereof described herein, and a pharmaceutically acceptable carrier or excipient.

In a further aspect, the invention provides a pharmaceutical combination comprising an antibody, or antigen binding fragment thereof, or a pharmaceutical composition as described herein, together with one or more additional therapeutic agents.

In yet another aspect, the invention provides a method of inhibiting cytokine IFNα release from immune cells of a subject, the method comprises contacting the anti-BDCA2 antibody or antigen-binding fragment thereof or pharmaceutical composition described herein with the immune cells of the subject, preferably, the immune cells are PBMC cells stimulated by CpG-A.

In a further aspect, the invention provides the use of an antibody or an antigen-binding fragment thereof as described herein, a pharmaceutical composition as described herein, or a pharmaceutical combination as described herein in the manufacture of a medicament for the treatment and/or prevention of a BDCA2-mediated disease, wherein preferably the disease is an inflammatory disease; more preferably, the inflammatory disease is selected from the group consisting of systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis and polymyositis.

In a further aspect, the invention provides an antibody or an antigen-binding fragment thereof as described herein, a pharmaceutical composition as described herein or a pharmaceutical combination as described herein for use in the treatment and/or prevention of a BDCA2-mediated disease, preferably the disease is an inflammatory disease; more preferably, the inflammatory disease is selected from the group consisting of systemic lupus erythematosus, discoid lupus, epidermal lupus erythematosus, lupus nephritis, rheumatoid arthritis, inflammatory bowel disease, systemic sclerosis (scleroderma), psoriasis, type I diabetes, dermatomyositis and polymyositis.

In yet another aspect, the invention provides a method of treating and/or preventing a BDCA2-mediated disease or disorder, comprising administering to a subject in need thereof a therapeutically or prophylactically effective amount of an antibody or antigen-binding fragment thereof described herein, a pharmaceutical composition described herein, or a pharmaceutical combination described herein, preferably the disease is an inflammatory disease; more preferably, the inflammatory disease is selected from the group consisting of systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis and polymyositis.

In a further aspect, the invention provides a kit comprising an antibody or an antigen binding fragment thereof described herein, a pharmaceutical composition described herein or a pharmaceutical combination described herein, and preferably further comprising an administration device.

In yet another aspect, the invention provides a method of detecting the presence of BDCA2 in a sample using an antibody or antigen-binding fragment thereof described herein or a detection composition containing the antibody or antigen-binding fragment thereof.

### Brief Description of the Figures

Figure 1 shows results of the FACS assays detecting binding of hybridoma antibodies to human CHOK1-hBDCA2 stably transfected cells which expresses human BDCA2 molecules. Panels a-e refer to antibodies from different hybridomas, respectively, as noted in legend.
Figure 2 shows results of the FACS assay detecting binding of hybridoma antibodies to stably transfected cells 293F-cynoBDCA2 which expresses cynomolgus BDCA2 molecules. Panels a-e refer to antibodies from different hybridomas, respectively, as noted in legend.
Figure 3 shows results of the binding assay of anti-BDCA2 chimeric antibodies to human CHOK1-hBDCA2 cells detected via FACS. Panels a to c refer to results of different chimeric antibodies, respectively, as noted in legend.
Figure 4 shows the detection of the internalization capacity of humanized antibodies. Panel a is result of humanized antibodies at the concentration of 100 nM in hu005 group. Panel b is result of humanized antibodies at the concentration of 10 nM in hu005 group. Panel c is result of humanized antibodies at the concentration of 100 nM in hu033 group. Panel d is result of humanized antibodies at the concentration of 10 nM in hu033 group.
FIG. 5 shows the binding ability of Fc-engineered antibodies to cells determined via FACS assays. Panel a is result of the assay of binding to CHOK1-Human hBDCA2 cells. Panel b is result of the assay of binding to 293-Cyno BDCA2 cells.

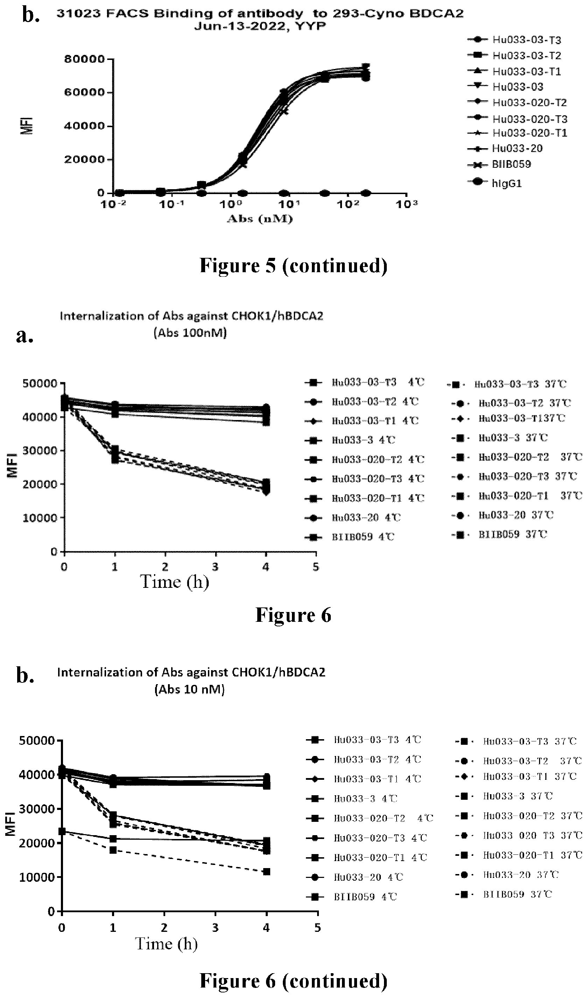

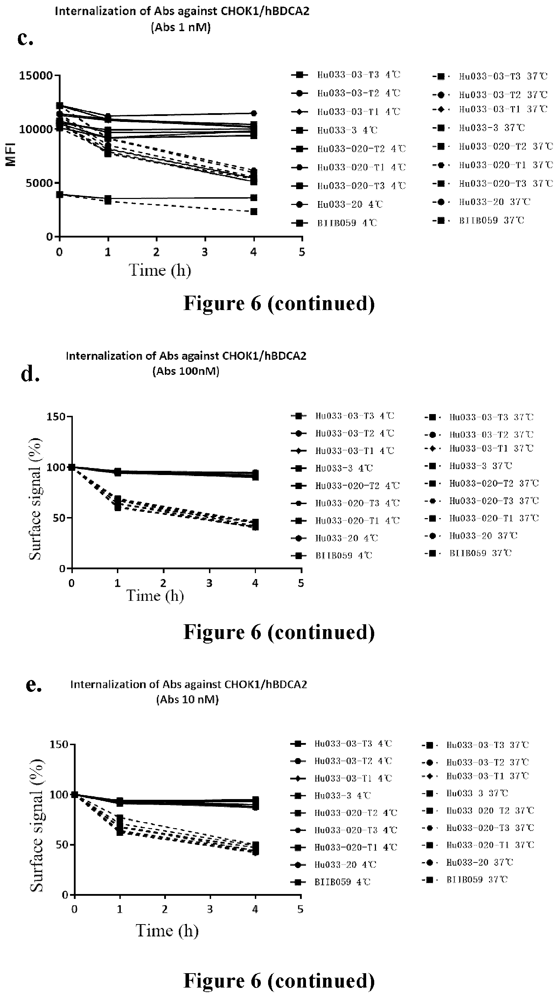

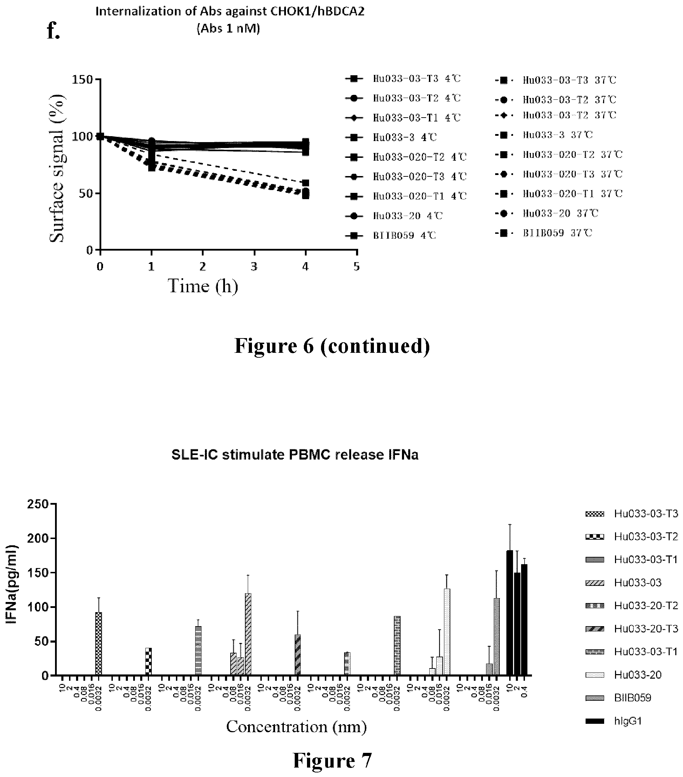

Figure 6 shows the FACS detection of internalization capacity of Fc-engineered humanized antibodies. Panel a shows the MFI of humanized antibodies determined at the concentration of 100 nM. Panel b shows the MFI of humanized antibodies determined at the concentration of 10 nM. Panel c shows the MFI of humanized antibodies determined at the concentration of 1 nM. Panel d shows the surface signal percentage of humanized antibodies determined at the concentration of 100 nM. Panel e shows the surface signal percentage of humanized antibodies determined at the concentration of 10 nM. Panel f shows the surface signal percentage of humanized antibodies determined at the concentration of 1 nM.
Figure 7 shows the Fc-engineered humanized antibodies mediated inhibition of the SLE-IC-induced IFNα secretion from PBMC.

### Detailed Description of Invention

### Definitions

The practice of the invention will employ, unless otherwise indicated, conventional techniques of molecular biology (including recombinant techniques), microbiology, cell biology, biochemistry and immunology, which are within the skill of the art.

Certain technical and scientific terms are specifically defined below in order that the invention may be more readily understood. The technical and scientific terms or expressions used herein have the meaning commonly understood by one of ordinary skill in the art to which this invention belongs, unless explicitly defined otherwise elsewhere herein. The practitioner may specifically refer, at least in part, to Current Protocols in Molecular Biology (Ausubel) with respect to definitions and terms in the art. Abbreviations for amino acid residues follow the standard 3-letter and/or 1-letter code used in the art to refer to one of the 20 commonly used L-amino acids. The singular forms as used herein, including the claims, include the corresponding plural forms thereof, unless the context clearly dictates otherwise.

The term "about" when used in conjunction with numerical value is meant to encompass numerical values within range having lower limit of 5% less than specified numerical value and upper limit of 5% greater than specified numerical value, including but not limited to ± 5%, ± 2%, ± 1%, and ± 0. 1%, as such variations are appropriate to perform disclosed methods.

The term "and/or" should be understood to mean any one of alternatives or combination of any two or more of alternatives.

As used herein, the term "or" should be understood to have the same meaning as "and/or" as defined above. For example, "or" or "and/or" should be interpreted as being inclusive when separating items in the list, that is, the inclusion of at least one, but also including more than one, of a number or list of elements, and, optionally, additional unlisted items. Only terms clearly indicated to the contrary, such as "only one of" or" exactly one of ", or "consisting of" used in the claims, will refer to only one number or only one element in the list.

The words "a" and "an," as used herein, unless clearly indicated to contrary by context, should be understood to mean "at least one."

The term "BDCA2" herein is an II-type and C-type lectin BDCA2 specifically expressed on pDC, which consists of a single extracellular carbohydrate recognition domain (CRD) at C-terminus, a transmembrane region from asparagine residue at position 45 to isoleucine residue at position 213, and a short cytoplasmic tail at N-terminus (without a signaling motif). BDCA2 transmits intracellular signals through an associated transmembrane adaptor, the FcεRIγ. Antibody-mediated binding of BDCA2 results in recruitment of spleen tyrosine kinase (SYK) to phosphorylated immunoreceptor tyrosine-based activation motif (ITAM) of FcRI. Activation of Syk results in activation of B cell linker (BLNK), Bruton tyrosine kinase (BTK), and phospholipase C2 (PLC2), thereby resulting in mobilization of Ca²⁺.

The term "BDCA2" includes variants, isoforms, species homologs of human BDCA2, or BDCA2 of other species and analogs having at least one common epitope of BDCA2. The term encompasses unprocessed full-length BDCA2 as well as any form of BDCA2 that results from processing in the cell, unless otherwise specified. The term encompasses "full-length," unprocessed BDCA2 as well as any form of BDCA2 that results from processing in the cell, or any fragment thereof, e.g. splice variants or allelic variants. In one embodiment, BDCA2 refers to full length from human or cynomolgus monkey BDCA2 or a fragment thereof (such as the mature fragment thereof lacking the signal peptide).

The term "immune response" refers to the effect of for example lymphocytes, antigen-presenting cells, phagocytes, granulocytes and production of soluble macromolecules, including antibodies, cytokines, and complement by the aforementioned cells or the liver, this effect results in selective damage, destruction or clearance from the human body of invading pathogens, cells or tissues infected with pathogens, cancer cells, or normal human cells or tissues in the case of autoimmunity or pathological inflammation.

The term "human plasmacytoid dendritic cells (pDC)" is a specialized population of myeloid-derived cells that secrete type I interferons (IFN) in response to toll-like receptor (TLR) ligands. PDCs are a key link of cellular immunity, involved in the induction and priming of immune responses and antigen tolerance. PDCs are different from ordinary DCs, e.g. they acquire antigen via receptor-mediated internalization, and so on. Type I interferons produced by pDC activated by pattern recognition receptors may also present antigens, thereby linking innate and acquired immune responses. At the same time, over-activation of pDC may also negatively affect the immune response process, e.g. may lead to autoimmune diseases.

The term "signal transduction pathway" or "signal transduction activity" refers to a biochemical causal relationship typically initiated by protein-protein interactions, such as the binding of growth factors to receptors, the relationship results in a signal being transmitted from one part of the cell to another part of the cell. **In** general, transfer includes the specific phosphorylation of one or more tyrosine, serine, or threonine residues on one or more proteins in a series of reactions that result in signal transduction. The penultimate process typically includes nuclear events, thereby resulting in changes in gene expression.

The term "activity" or "biological activity", or the term "biological property" or "biological characteristic" are used interchangeably herein, including but not limited to epitope/antigen affinity and specificity, ability to neutralize or antagonize BDCA2 activity in vivo or in vitro, IC₅₀, in vivo stability of the antibody, and immunogenic properties of the antibody. Other identifiable biological properties or characteristics of antibodies well known in the art include, for example, cross-reactivity (i.e., typically with a non-human homologue of the targeted peptide, or cross-reactivity with other proteins or tissues), and the ability to maintain high expression levels of the protein in mammalian cells. The aforementioned properties or characteristics are observed, determined or assessed using techniques well known in the art, such techniques include, but are not limited to, ELISA, FACS or BIACORE plasmon resonance assays, unrestricted in vitro or in vivo neutralization assays, receptor binding, production and/or secretion of cytokines or growth factors, signal transduction, and immunohistochemistry of tissue sections of different origins, including human, primate, or any other origin.

The term "antibody" refers to any form of antibody that possesses desired biological activity. Thus, it is used in broadest sense and specifically includes, but is not limited to, monoclonal antibodies (including full length monoclonal antibodies), polyclonal antibodies, multispecific antibodies (e.g., bispecific antibodies), humanized antibodies, fully human antibodies, chimeric antibodies, and camelized single domain antibodies.

The term "isolated antibody" refers to the purification state of the binding compound, and in this case means that the molecule is substantially free of other biomolecules, for example, nucleic acids, proteins, lipids, sugars or other materials such as cell debris and growth media. The term "isolated" does not mean the complete absence of such materials or the absence of water, buffers, or salts, unless they are present in amounts that would significantly interfere with experimental or therapeutic use of the binding compounds described herein.

The term "monoclonal antibody" refers to an antibody obtained from a population of substantially homogeneous antibodies, i.e. the individual antibodies comprising the population are identical except for possible naturally occurring mutations that may be present in minor amounts. The monoclonal antibody is highly specific, in contrast, conventional (polyclonal) antibody preparations typically include large amounts of antibodies directed against (or specific for) different epitopes. The modifier "monoclonal" indicates the character of the antibody as being obtained from a substantially homogeneous population of antibodies, and is not to be construed as requiring production of the antibody by any particular method.

The term "full length antibody" refers to an immunoglobulin molecule comprising at least four peptide chains when naturally occurring: two weight (H) chains and two light (L) chains are interconnected by disulfide bonds. Each heavy chain is comprised of a heavy chain variable region (abbreviated herein as VH) and a heavy chain constant region (abbreviated herein as CH). The heavy chain constant region consists of three domains, CH1, CH2 and CH3. Each light chain is comprised of a light chain variable region (abbreviated herein as VL) and a light chain constant region. The light chain constant region is comprised of one domain CL. The VH and VL regions can be further subdivided into complementarity determining regions (CDRs) with hypervariability and regions interspaced with more conservation, termed framework regions (FRs). Each VH or VL region is comprised of 3 CDRs and 4 FR in the following order from amino to carboxyl terminus: FR1, CDR1, FR2, CDR2, FR3, CDR3, FR4. The variable region of the heavy and light chains contains a binding domain that interacts with an antigen. The constant region of an antibody can mediate the binding of the immunoglobulin to host tissues or factors, including various cells of the immune system (e.g., effector cells) and the first component (Clq) of the classical complement system.

The term "antigen-binding fragment" of an antibody ("parent antibody") includes a fragment or derivative of an antibody, which retains at least some binding specificity of parent antibody. Examples of binding fragments of an antibody include, but are not limited to, Fab, Fab ', F(ab')₂, and Fv fragments; diabodies; linear antibodies; single chain antibody molecules, e.g., sc-Fv; nanobodies and multispecific antibodies formed from antibody fragments, as are well known in the art. **In** some preferred embodiments of the invention, the antigen-binding fragments of the invention are selected from the group consisting of Fab, Fab ', F(ab')₂ and Fv fragments; diabodies; linear antibodies; single chain antibody molecules, e.g., sc-Fv; nanobodies and multispecific antibodies formed from antibody fragments. The binding fragment or derivative typically retains at least 10% of its antigen binding activity when the binding activity of an antigen is expressed on a molar concentration basis. Preferably the binding fragment or derivative retains at least 20%, 50%, 70%, 80%, 90%, 95% or 100% or more of the antigen binding affinity of the parent antibody. It is also contemplated that antigen-binding fragments of antibodies can include conservative or non-conservative amino acid substitutions that do not significantly alter their biological activity (referred to as "conservative variants" or "function-conservative variants" of antibodies). The term "binding compound" refers to both antibodies and binding fragments thereof.

The term "single-chain Fv" or "scFv" antibody refers to antibody fragments comprising VH and VL domains of antibody, wherein these domains are present in single polypeptide chain. The Fv polypeptide generally further comprises polypeptide linker between VH and VL domains which enables scFv to form desired structure for antigen binding.

The term "domain antibody" is immunologically functional immunoglobulin fragment containing only heavy or light chain variable region. In certain instances, two or more VH regions are covalently linked to peptide linker to form bivalent domain antibody. The two VH regions of bivalent domain antibody may target same or different antigens.

The term "bivalent antibody" comprises two antigen binding sites. In certain instances, both binding sites have same antigen specificity. However, bivalent antibodies may be bispecific.

The term "diabodies" refers to small antibody fragments with two antigen-binding sites, the fragments comprise a heavy chain variable domain (VH) connected to a light chain variable domain (VL) in the same polypeptide chain (VH-VL or VL-VH). This domain is forced to pair with the complementary domains of another chain and create two antigen-binding sites by using a linker that is not short to allow pairing between the two domains of the same chain.

The term "mouse antibody" or "hybridoma antibody" in the present disclosure is a monoclonal antibody against BDCA2 prepared according to the knowledge and skill in the art. Test subjects were injected with BDCA2 antigen at time of preparation, and hybridomas expressing antibodies with desired sequence or functional properties were isolated. Hybridoma technology is by fusing two types of cells while also maintaining main features of both. These two types of cells are antigen-immunized mouse splenocytes and mouse myeloma cells, respectively. The main feature of a mouse splenocyte (B lymphocyte) immunized with a specific antigen is its antibody secreting function, but not continuous culture in vitro, and mouse myeloma cells can then divide and proliferate indefinitely under culture conditions, i.e. having so-called immortality. Only hybrid cells in which B cells are fused with myeloma cells can have the ability to continue to culture under the action of the selection medium, creating cell clones featured in both antibody secretion and preservation of cell immortality. In some embodiments, the invention obtains hybridoma cells capable of expressing positive antibodies by immunizing a mouse with BDCA2 protein and then fusing spleen cells and myeloma cells of the mouse.

The term "chimeric antibody" is an antibody having the variable domains of a first antibody and the constant domains of a second antibody, wherein the first antibody and the second antibody are from different species. Typically, variable domains are obtained from antibodies of rodents and the like ("parent antibodies"), whereas the constant domain sequences are obtained from human antibodies, such that the resulting chimeric antibody is less likely to induce an adverse immune response in a human subject compared to the parent rodent antibody. In some embodiments of the invention, the rodent is a mouse or rat. In some preferred embodiments of the invention, the affinity of the chimeric antibody for antigen is no lower or almost no lower than the parent mouse antibody.

The term "humanized antibody" refers to forms of antibodies that contain sequences from both human and non-human (e.g., mouse, rat) antibodies. In general, a humanized antibody comprises substantially all of at least one, and typically two, variable domains, in which all or substantially all of the hypervariable loops correspond to those of a non-human immunoglobulin and all or substantially all of the framework (FR) regions are those of a human immunoglobulin sequence. The humanized antibody optionally may comprise at least a portion of a human immunoglobulin constant region (Fc).

The term "fully human antibody" refers to an antibody that contains only human immunoglobulin protein sequences. A fully human antibody may contain mouse carbohydrate chains as produced in a mouse, in a mouse cell, or in a hybridoma derived from a mouse cell. Likewise, a "mouse antibody" refers to an antibody that contains only mouse immunoglobulin sequences. Alternatively, a fully human antibody may contain rat carbohydrate chains if produced in a rat, in a rat cell, or in a hybridoma derived from a rat cell. Likewise, a "rat antibody" refers to an antibody that contains only rat immunoglobulin sequences.

An "isotype" antibody refers to class of antibodies provided by heavy chain constant region genes (e.g., IgM, IgE, IgG, such as IgGl, IgG2, or IgG4). Isotype also includes modified versions of one of these classes, where modifications have been made to alter Fc function, e.g., to enhance or diminish effector function or binding to Fc receptors.

The term "Fc region" herein is used to define a C-terminal region of an immunoglobulin heavy chain that contains at least a portion of the constant region. The term includes native sequence Fc regions and variant Fc regions. In some embodiments, a human IgG heavy chain Fc region extends from Cys226, or from Pro230, to the carboxyl-terminus of the heavy chain. However, the C-terminal lysine (Lys447) of the Fc region may or may not be present (numbering in this paragraph is according to the EU numbering system, also referred to as the EU index, as described in Rabat et al., Sequences of Proteins of Immunological Interest, 5th Ed. Public Health Service, National Institutes of Health, Bethesda, MD, 1991).

The term "epitope" refers to a protein determinant capable of specific binding to an antibody. Epitopes usually consist of various chemically active surface molecules, such as amino acids or sugar side chains, and generally have specific three-dimensional structural characteristics, as well as specific charge characteristics. Conformational and non-conformational epitopes are distinguished in that the binding to the former but not to the latter is lost in the presence of denaturing solvents.

The term "cross-reaction" as described herein refers to binding to fragments of an antigen of the same target molecule of human, monkey, and/or mouse origin (mouse or rat) "Cross-reaction" should be understood as an interspecies reaction of an antigen binding molecule (e.g., an antibody) with a cognate molecule (e.g., BDCA2) expressed in a different species. The specificity of cross-reaction of monoclonal antibodies recognizing human BDCA2, monkey, and/or mouse BDCA2 (mouse or rat) can be determined by FACS analysis.

"Affinity" or "binding affinity" refers to intrinsic binding affinity reflecting an interaction between members of a binding pair. The affinity of a molecule X for its partner Y can generally be represented by the equilibrium dissociation constant (K_{D}), and the equilibrium dissociation constant is the ratio of the dissociation and association rate constants (k_{dis} and kₒₙ, respectively). Affinity can be measured by common methods known in the art. In some embodiments of the invention, affinity, e.g., between an antibody of the invention and antigen, is measured using surface plasmon resonance (SPR) technology. In some preferred embodiments of the invention, one particular method for measuring affinity is the BIAcore method herein.

The term "not binding" to protein or cell refers to not binding to protein or cell or not binding to it with high affinity, i.e. binding to protein or cell with K_{D} of 1.0×10⁻⁶ M or higher, more preferably 1.0×10⁻⁵ M or higher, more preferably 1.0×10⁻⁴ M or higher, 1.0×10⁻³ M or higher, more preferably 1.0×10⁻² M or higher.

The term "high affinity" for IgG antibodies refers to K_{D} for antigen of 1.0×10⁻⁶ M or less, preferably 5.0×10⁻⁸ M or less, more preferably 1.0×10⁻⁸ M or less, 5.0×10⁻⁹ M or less, more preferably 1.0×10⁻⁹ M or less. For other antibody isoforms," high affinity "binding may vary. For example," high affinity "binding for IgM isoforms refers to K_{D} of 10⁻⁶ M or less, preferably 10⁻⁷ M or less, more preferably 10⁻⁸ M or less.

The term "antibody-dependent cytotoxicity", "antibody-dependent cell-mediated cytotoxicity" or "ADCC" refers to cell-mediated immune defense in which immune system effector cell actively lyses target cells whose cell membrane surface antigen binds to antibody.

The term "complement dependent cytotoxicity" or "CDC" refers to effector function of IgG and IgM antibodies, eliciting classic complement pathway when bound to surface antigens, including formation of membrane attack complex and lysis of target cells.

The terms "nucleic acid", "polynucleotide", "nucleic acid molecule" and "polynucleotide molecule" are used interchangeably herein (unless the context indicates otherwise), and refers to deoxyribonucleic acids (DNA) or ribonucleic acids (RNA) and polymers thereof in single-or double-stranded form. Unless expressly limited, otherwise the term includes nucleic acids containing known analogs of natural nucleotides that have similar binding properties as the reference nucleic acid and are metabolized in a manner similar to naturally occurring nucleotides (see, U.S. Patent No. 8, 278, 036 to Kariko et al., which discloses an mRNA molecule in which uridine is replaced by pseudouridine, a method of synthesizing said mRNA molecule and a method for delivering a therapeutic protein in vivo). A particular nucleic acid sequence also implicitly encompasses conservatively modified variants thereof (e.g., degenerate codon substitutions), alleles, orthologs, SNPs, and complementary sequences as well as sequences explicitly indicated, unless otherwise noted. Specifically, degenerate codon substitutions can be achieved by generating sequences in which third position of one or more selected (or all) codons is substituted with mixed-base and/or deoxyinosine residues (Batzer et al., Nucleic Acid Res. 19: 5081 (1991); 260: 2605-2608 (1985); and Rossolini et al., Mol. Cell. Probes 8:91-98(1994)).

"Construct" refers to any recombinant polynucleotide molecule (such as a plasmid, cosmid, virus, autonomously replicating polynucleotide molecule, bacteriophage, or linear or circular single-stranded or double-stranded DNA or RNA polynucleotide molecule), which is derivable from any source, capable of integrating with the genome or autonomous replicating, in which comprising one or more polynucleotides that has been linked in a functionally operative manner (i.e., operably linked). In some preferred embodiments of the invention, recombinant constructs comprise a polynucleotide of the invention operably linked to transcription initiation regulatory sequences that will drive and/or direct transcription of a polynucleotide of the invention in a host cell. Both heterologous and non-heterologous (i.e., endogenous) promoters may be used to drive and/or direct expression of a polynucleotide of the invention.

"Vector" refers to any recombinant polynucleotide construct, the construct may be used for the purpose of transformation (i.e. to introduce heterologous DNA into a host cell). One type of vector is a "plasmid", refers to a circular double stranded DNA loop, and additional DNA segments may be ligated into this loop. Another type of vector is a viral vector, wherein additional DNA segments may be ligated into viral genome. Certain vectors are capable of autonomous replicating in a host cell into which they are introduced (e.g., bacterial vectors having a bacterial origin of replication and episomal mammalian vectors). Other vectors (e.g., non-episomal mammalian vectors) are integrated into the genome of a host cell upon introducing into a host cell, and thus are replicated along with the host genome. Moreover, certain vectors are capable of directing expression of operatively linked genes. Such vectors are referred to herein as "expression vectors".

The term "expression vector" as used herein refers to a nucleic acid molecule capable of replicating and expressing a gene of interest when transformed, transfected or transduced into a host cell. An expression vector comprises one or more phenotypic selectable markers and an origin of replication to ensure maintenance of vector and to provide amplification in host if required. In preferred embodiments of the invention, the expression vector of the invention comprises a construct of the invention and/or a polynucleotide of the invention.

Operation of "activation", "stimulation" and "treatment" for a cell or receptor can have the same meaning, e.g. a cell or receptor is activated, stimulated or treated with a ligand, unless otherwise or clearly dictated by context. "Ligand" includes natural and synthetic ligands, for example, cytokines, cytokine variants, analogs, muteins, and antibody-derived binding compounds. "Ligand" also includes small molecules, for example, peptidomimetics of cytokines and peptidomimetics of antibodies. "Activation" may refer to activation of a cell regulated by internal mechanisms as well as external or environmental factors. "Response/Reaction", e.g., a response of a cell, tissue, organ, or organism, including a change in biochemical or physiological behavior (such as concentration within a biological compartment, density, adhesion, or migration, rate of gene expression, or state of differentiation), wherein the change is related to activation, stimulation, or treatment, or related to an internal mechanism such as genetic programming.

As used herein, the term "BDCA2-mediated disease" refers to a disease in which there is an involvement of BDCA2 in the course of onset, progression, or migration, for example, involving activation (e.g., aberrant activation or excessive activation) of BDCA2, and the involvement of BDCA2 directly or indirectly results in at least one of the following effects: disease initiation/occurrence; increased/deepened pathological alterations of the disease; expansion of the site/extent of disease impact; exacerbation of body damage due to disease; increased suffering from disease; disease insensitivity/resistance to therapeutic measures; decreased self-healing propensity of the disease and worse prognosis of the disease. In some embodiments of the invention, the BDCA2-mediated disease is an inflammatory disease; in some particular embodiments of the invention, the BDCA2-mediated disease is systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis, and/or polymyositis.

As used herein, the term "treating" or "treatment" of any disease or disorder refers, in one embodiment, to ameliorating the disease or disorder (i.e., slowing or arresting or reducing the progression of the disease or at least one of the clinical symptoms thereof). In another embodiment, "treating" or "treatment" refers to alleviating or ameliorating at least one physical parameter, including those physical parameters that may not be discernible by the patient. In another embodiment, "treating" or "treatment" refers to modulating a disease or disorder, either physically (e.g., stabilization of a discernible symptom), physiologically (e.g., stabilization of a physical parameter), or both. Methods for assessing treatment and/or prevention of a disease are generally known in the art, unless specifically described herein.

A "subject" includes any human or non-human animal. The term "non-human animal" includes all vertebrates, e.g., mammals and non-mammals, such as non-human primates, sheep, dogs, cats, horses, cows, chickens, amphibians, reptiles, etc. As used herein, the term "cyno" or "cynomolgus monkey" refers to cynomolgus monkey or derived from cynomolgus monkey.

Administration "in combination with" one or more additional therapeutic agents include simultaneous (co) administration and consecutive administration in any order.

A "therapeutically effective amount," "therapeutically effective dose," and "effective amount" refer to an amount effective to prevent or ameliorate one or more symptoms of disease or condition, or development of disease or condition when the BDCA2 antibody or antigen-binding fragment thereof of the invention administered alone or in combination with other therapeutic agents to a cell, tissue, or subject. A therapeutically effective dose also refers to an amount of the antibody or antigen-binding fragment thereof sufficient to result in amelioration of symptoms, such as an amount to treat, cure, prevent, or ameliorate relevant medical conditions or to increase the rate of treatment, cure, prevention, or amelioration of such conditions. A therapeutically effective dose refers to that ingredient alone when active ingredient given alone is administered to subject. A therapeutically effective dose refers to the combined amount of the active ingredients that result in a therapeutic effect when administered in combination, whether in combination, sequentially or simultaneously. An effective amount of a therapeutic agent will result in an increase in a diagnostic criterion or parameter of at least 10%, usually at least 20%, preferably at least about 30%, more preferably at least 40%, most preferably at least 50%.

A "pharmaceutically acceptable carrier" refers to ingredient in pharmaceutical formulation or composition, other than active ingredient, which is nontoxic to subject. A pharmaceutically acceptable carrier includes, but is not limited to, buffer, excipient, stabilizer, or preservative.

### Anti-BDCA2 Antibodies

In one aspect, the invention provides an antibody or antigen-binding fragment thereof that specifically binds BDCA2. The term "anti-BDCA2 antibody", "anti-BDCA2", "BDCA2 antibody" or "an antibody that binds BDCA2" refers to an antibody that is capable of binding BDCA2 protein or a fragment thereof with sufficient avidity such that the antibody can be used as a diagnostic and/or therapeutic agent in targeting BDCA2.

In some embodiments, the antibodies of the invention bind to human or cynomolgus BDCA2 protein. In some embodiments, the antibodies of the invention bind to CHOK1-humanBDCA2 cells or 293F-cynoBDCA2 cells. In some embodiments, the antibodies of the invention inhibit CpG-A to stimulate human PBMC cells to release cytokine IFNα. In some embodiments, the antibodies of the invention have greater internalization capacity.

Any suitable method for producing antibodies can be used to produce the antibodies of the invention. Any suitable form of BDCA2 can be used as an immunogen (antigen) for producing antibodies. By way of example and not limitation, any BDCA2 variant or fragment thereof can be used as an immunogen. In some embodiments, hybridoma cells producing monoclonal anti-BDCA2 antibodies of mouse origin can be produced by methods well known in the art.

Antibodies derived from rodents, such as mice, may cause unwanted immunogenicity of the antibodies when used in vivo as therapeutic drugs, and repeated use results in generating an immune response against the therapeutic antibody in the human body, such immune response at least results in a loss of therapeutic efficacy, and severe results in potentially lethal anaphylaxis. One method to reduce the immunogenicity of rodent antibodies includes the generation of chimeric antibodies, wherein the mouse variable regions are fused to human constant regions (Liu et al. (1987) Proc. Natl. Acad. Sci. USA 84: 3439-43). However, retention of intact rodent variable regions in chimeric antibodies may still cause deleterious immunogenicity in patients. Grafting the complementarity determining region (CDR) loops of rodent variable domains onto human frameworks (i.e., humanization) has been used to further minimize rodent sequences (Jones et al. (1986) Nature 321: 522; Verhoeyen et al (1988) Science 239: 1534). In some embodiments, the antibody of the invention is a chimeric antibody. In some preferred embodiments, the antibody of the invention is a humanized antibody. In some preferred embodiments of the invention, the affinity of a mouse antibody, the chimeric antibody and/or the human-derived antibody of the invention to the antigen human BDCA2 or cynomolgus BDCA2 is embodied as a K_{D} value of 1.0×10⁻⁶ M or less, a KD value of 5.0×10⁻⁷ M or less, a KD value of 2.5×10⁻⁷ M or less, a KD value of 1.0×10⁻⁷ M or less, a KD value of 5.0×10⁻⁸ M or less, a KD value of 2.5×10⁻⁸ M or less, a KD value of 1.0×10⁻⁸ M or less, a KD value of 5.0×10⁻⁹ M or less, a KD value of 2.5×10⁻⁹ M or less, a KD value of 1.0×10⁻⁹ M or less, a KD value of 5.0×10⁻¹⁰ M or less, a KD value of 2.5×10⁻¹⁰ M or less, a KD value of 1.0×10⁻¹⁰ M or less.

The amino acid sequences of the light and heavy chain variable regions and CDRs of the anti-BDCA2 mouse antibodies of the invention are shown in Table 2.

In some embodiments, chimeric or humanized antibodies of the invention can be prepared based on the sequence of said prepared mouse monoclonal hybridoma antibody. DNA encoding the heavy and light chain immunoglobulins can be obtained from the mouse hybridoma of interest and engineered to contain non-mouse (e.g., human) immunoglobulin sequences using standard molecular biology techniques.

In some embodiments, the hybridoma-derived immunoglobulin heavy and light chain variable regions can be operably linked to human IgG constant regions using methods known in the art (see, e.g., U.S. Patent No. 4 to Cabilly et al.) to obtain chimeric heavy chains and chimeric light chains, thereby the chimeric BDCA2 antibody of the invention is produced. In some embodiments, the chimeric antibody of the invention comprises a constant region selected from any human IgG subtype, such as IgG1, IgG2, IgG3, IgG4, preferably IgG1.

In some embodiments, chimeric BDCA2 antibodies of the invention can be obtained from expression cells which are "mixed and matched" transfected by chimeric light chain and chimeric heavy chain expression plasmids, and BDCA2 binding of such "mixed and matched" antibodies can be tested using the binding assays described above and other conventional binding assays (e.g., ELISA).

The exact amino acid sequence boundaries of variable region CDRs of the antibodies of the invention may be determined using any of a number of well-known protocols, including Chothia based on the three-dimensional structure of the antibody and the topology of the CDR loops (Chothia et al. (1989) Nature 342: 877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (1999 Nucleic Acids Research, 27, 209-212), and the North CDR definition based on affinity propagation clustering utilizing a large number of crystal structures.

The boundaries of CDRs of the antibodies of the invention can be determined by one skilled in the art according to any protocol in the art (e.g. a different assignment system or a combination), unless otherwise indicated.

It should be noted that the boundaries of the CDRs of the variable region of the same antibody obtained based on different assignment systems may differ. That is, the CDR sequences of the variable region of the same antibody as defined under different assignment systems may differ. Thus, when referring to defining an antibody with specific CDR sequences defined herein, the scope of the antibody also encompasses antibodies whose variable region sequences comprise the specific CDR sequences but whose claimed CDR boundaries differ from the specific CDR boundaries defined herein due to the application of a different scheme (e.g. a different assignment system or a combination).

The antibodies having different specificities (i.e., different binding sites for different antigens) have different CDRs. However, only a limited number of amino acid positions within the CDRs are directly involved in antigen binding although CDRs differ from antibody to antibody. A minimum overlap area may be determined using at least two of Kabat, Chothia, AbM, Contact, and North methods, thereby providing a "minimal binding unit" for antigen binding. A minimal binding unit may be a subportion of a CDR. The residues in the remainder of the CDR sequence can be determined by the structure and protein folding of the antibody, ss will be apparent to those skilled in the art. Thus, variants of any of the CDRs given herein are also contemplated by the invention. For example, in variants of one CDR, the amino acid residues of the smallest binding unit may remain unchanged, while the remaining CDR residues according to the Kabat or Chothia definition may be replaced by conserved amino acid residues.

The mixed pairs of various chimeric heavy and light chain expression plasmids are used to transfect the expression cells, which is anti-BDCA2 chimeric antibodies.

The humanized antibodies described herein can have mouse CDR regions inserted into human germline framework regions using methods known in the art. See U.S. Patent No. 5,225,539 to Winter et al., and U.S. Patent No. 5,530,101; 5,585,089; 5,693,762 and 6,180,370to Queen et al.

The light and heavy chain amino acid sequences of the anti-BDCA2 humanized antibodies Hu033-03, Hu033-20 and Hu005-04 of the invention are shown below. The sequences outside the variable regions of the LC and HC of the remaining humanized antibodies are identical to the corresponding sequences of Hu033-03, Hu033-20 and Hu005-04.
Hu033-03-HC-IgG1: SEQ ID NO:183 (SEQ ID NOs:52, 39 and 53 for HCDR1-HCDR3, respectively) 
EVQLVESGGGLVKPGGSLRLSCAASGFTFSSYGMSWVRQAPGKGLEWVSTI
SSGDSYTYYPDSVKGRFTISRDNAKNSLYLQMNSLRAEDTAVYYCARQIYYDYAY
YFDFWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEAL
HNHYTQKSLSLSPGK
Hu033-03-LC: SEQ ID NO:186 (SEQ ID NO:54, 55 and 42 for LCDR1-LCDR3, respectively) 
DIVLTQSPASLAVSPGQRATITCRASESVSFRTSHLMHWYQQKPGQPPKLLIY
GASNLESGVPARFSGSGSETDFTLTINPVEAEDTANYFCQQSIEDPPTFGGGTKVEI
KRTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
Hu033-20-HC-IgG1: SEQ ID NO:184 (SEQ ID NO:52, 39 and 53 for HCDR1-HCDR3, respectively) 
EVQLVESGGGLVKPGGSLRLSCAASGFTFRSYGMSWVRQAPGKRLEWVSTI
SSGDSYTYYPDSVKGRFTISRDNAKNSLYLQMNSLKAEDTAVYYCARQIYYDYA
YYFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMH
EALHNHYTQKSLSLSPGK
Hu033-20-LC: SEQ ID NO:187 (SEQ ID NO:54, 55 and 42 for LCDR1-LCDR3, respectively) 
DIVMTQSPDSLAVSLGERATINCRASESVSFRTSHLMHWYQQKPGQPPKLLI
YGASNLESGVPDRFSGSGSGTDFTLTISSLQAEDVAVYYCQQSIEDPPTFGGGTKV
EIKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC
Hu005-04-HC-IgG1: SEQ ID NO:185 (SEQ ID NO:11, 12 and 13 for HCDR1-HCDR3, respectively) 
QVQLQQSGPGLVKPSETLSLTCTVSGGSLTNYGVHWIRQPPGKGLEWIGVIW
SGESTDYDAAFISRVTISKDNSKNQVSLKLSSVTAADTAVYYCARRRSHYYGYV
MDYWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHE
ALHNHYTQKSLSLSPGK
Hu005-04-LC: SEQ ID NO:188 (SEQ ID NO:86, 15 and 16 for LCDR1-LCDR3, respectively)

DIVLTQSPASLAVSPGQRATITCKASQSIDYdaIGYLNWYQQKPGQPPKLLIYA
ASNLESGVPARFSGSGSGTDFTLTINPVEANDTANYYCQQSNEDPPTFGQGTKLEI

KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

In some embodiments, amino acid changes include amino acid deletions, insertions, or substitutions. In some embodiments, the anti-BDCA2 antibodies or antigen-binding fragments thereof of the invention include those that have been mutated by amino acid deletion, insertion, or substitution, but still have an amino acid sequence that is at least about 90%, 91%, 92%, 93%, 94%, 95%, 96%, 97%, 98%, 99% or 100% identical to the above antibodies, particularly in the CDR regions depicted in the above sequences. In some embodiments, the antibodies of the invention have no more than 1, 2, 3, 4, or 5 amino acids mutated by amino acid deletion, insertion, or substitution in the CDR regions when compared to the CDR regions depicted in a particular sequence. In some embodiments, the antibodies of the invention have no more than 1, 2, 3, 4, or 5 amino acids mutated by amino acid deletion, insertion, or substitution in the framework regions when compared to the framework regions in a particular sequence.

In some embodiments, the Fc regions of the humanized antibodies Hu033-03 and Hu033-20 of the inventors were mutated, including S239D/I332E, G236A/S239D/I332E, and G236A/I332E, resulting in the following antibodies: Hu033-03-T1, Hu033-03-T2, Hu033-03-T3, Hu033-20-T1, Hu033-20-T2, Hu033-20-T3, whose light and heavy chain amino acid sequences are shown below:
Hu033-03-T1-HC: SEQ ID NO:191 
EVQLVESGGGLVKPGGSLRLSCAASGFTFSSYGMSWVRQAPGKGLEWVSTI
SSGDSYTYYPDSVKGRFTISRDNAKNSLYLQMNSLRAEDTAVYYCARQIYYDYA
YYFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPDVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPEEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
Hu033-03-T2-HC: SEQ ID NO:192 EVQLVESGGGLVKPGGSLRLSCAASGFTFSSYGMSWVRQAPGKGLEWVSTI 
SSGDSYTYYPDSVKGRFTISRDNAKNSLYLQMNSLRAEDTAVYYCARQIYYDYA
YYFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLAGPDVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPEEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
Hu033-03-T3-HC: SEQ ID NO:193 
EVQLVESGGGLVKPGGSLRLSCAASGFTFSSYGMSWVRQAPGKGLEWVSTI
SSGDSYTYYPDSVKGRFTISRDNAKNSLYLQMNSLRAEDTAVYYCARQIYYDYA
YYFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLAGPSVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPEEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
Hu033-03-T1/T2/T3-LC: SEQ ID NO:186 
DIVLTQSPASLAVSPGQRATITCRASESVSFRTSHLMHWYQQKPGQPPKLLIY
GASNLESGVPARFSGSGSETDFTLTINPVEAEDTANYFCQQSIEDPPTFGGGTKVEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
Hu033-20-T1-HC: SEQ ID NO:194 
EVQLVESGGGLVKPGGSLRLSCAASGFTFRSYGMSWVRQAPGKRLEWVSTI
SSGDSYTYYPDSVKGRFTISRDNAKNSLYLQMNSLKAEDTAVYYCARQIYYDYA
YYFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPDVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPEEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
Hu033-20-T2-HC: SEQ ID NO:195 
EVQLVESGGGLVKPGGSLRLSCAASGFTFRSYGMSWVRQAPGKRLEWVSTI
SSGDSYTYYPDSVKGRFTISRDNAKNSLYLQMNSLKAEDTAVYYCARQIYYDYA
YYFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLAGPDVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPEEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
Hu033-20-T3-HC: SEQ ID NO:196 
EVQLVESGGGLVKPGGSLRLSCAASGFTFRSYGMSWVRQAPGKRLEWVSTI
SSGDSYTYYPDSVKGRFTISRDNAKNSLYLQMNSLKAEDTAVYYCARQIYYDYA
YYFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLAGPSVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPEEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
Hu033-20-T1/T2/T3-LC: SEQ ID NO:187

DIVMTQSPDSLAVSLGERATINCRASESVSFRTSHLMHWYQQKPGQPPKLLI
YGASNLESGVPDRFSGSGSGTDFTLTISSLQAEDVAVYYCQQSIEDPPTFGGGTKV
EIKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC

The term "percent (%) of amino acid sequence identity" or simply "identity" is defined as percentage of amino acid residues in the candidate amino acid sequence that are identical in the reference amino acid sequence when amino acid sequences are aligned (and gaps introduced, if necessary) to achieve the maximum percent sequence identity, and not considering any conservative substitutions as part of sequence identity. The sequences can be aligned using various methods in the art in order to determine percent of amino acid sequence identity, for example, publicly available computer software such as BLAST, BLAST-2, ALIGN or MEGALIGN (DNASTAR) software can be used. Those skilled in the art can determine appropriate parameters for measuring alignment, including any algorithms needed to achieve maximal alignment over the full length of the sequences being compared.

Accordingly, in some embodiments, the antibodies of the invention comprise HCDR1, HCDR2, HCDR3, LCDR1, LCDR2 and LCDR3 as set forth in any combination in Table A below.

**Table A**

| Combination# | HCDR1, which comprises or consists of the following sequence (or sequence having 1, 2 or 3 amino acid differences) | HCDR2, which comprises or consists of the following sequence (or sequence having 1, 2 or 3 amino acid differences) | HCDR3, which comprises or consists of the following sequence (or sequence having 1, 2 or 3 amino acid differences) | LCDR1, which comprises or consists of the following sequence (or sequence having 1, 2 or 3 amino acid differences) | LCDR2, which comprises or consists of the following sequence (or sequence having 1, 2 or 3 amino acid differences) | LCDR3, which comprises or consists of the following sequence (or sequence having 1, 2 or 3 amino acid differences) |
|---|---|---|---|---|---|---|
| 1 | SEQ ID NO:1 | SEQ ID NO:2 | SEQ ID NO:3 | SEQ ID NO:4 | SEQ ID NO:5 | SEQ ID NO:6 |
| 2 | SEQ ID NO:7 | SEQ ID NO:2 | SEQ ID NO:8 | SEQ ID NO:9 | SEQ ID NO:10 | SEQ ID NO:6 |
| 3 | SEQ ID NO:11 | SEQ ID NO:12 | SEQ ID NO:13 | SEQ ID NO:14 | SEQ ID NO:15 | SEQ ID NO:16 |
| 4 | SEQ ID NO:17 | SEQ ID NO:12 | SEQ ID NO:13 | SEQ ID NO:18 | SEQ ID NO:15 | SEQ ID NO:16 |
| 5 | SEQ ID NO:19 | SEQ ID NO:20 | SEQ ID NO:21 | SEQ ID NO:22 | SEQ ID NO:23 | SEQ ID NO:24 |
| 6 | SEQ ID NO:25 | SEQ ID NO:26 | SEQ ID NO:27 | SEQ ID NO:28 | SEQ ID NO:29 | SEQ ID NO:30 |
| 7 | SEQ ID NO:31 | SEQ ID NO:32 | SEQ ID NO:33 | SEQ ID NO:34 | SEQ ID NO:35 | SEQ ID NO:36 |
| 8 | SEQ ID NO:25 | SEQ ID NO:26 | SEQ ID NO:37 | SEQ ID NO:28 | SEQ ID NO:29 | SEQ ID NO:30 |
| 9 | SEQ ID NO:38 | SEQ ID NO:39 | SEQ ID NO:40 | SEQ ID NO:41 | SEQ ID NO:15 | SEQ ID NO:42 |
| 10 | SEQ ID | SEQ ID | SEQ ID | SEQ ID | SEQ ID | SEQ ID |
| | NO:31 | NO:43 | NO:27 | NO:44 | NO:45 | NO:30 |
| 11 | SEQ ID NO:31 | SEQ ID NO:46 | SEQ ID NO:47 | SEQ ID NO:34 | SEQ ID NO:48 | SEQ ID NO:49 |
| 12 | SEQ ID NO:31 | SEQ ID NO:50 | SEQ ID NO:47 | SEQ ID NO:34 | SEQ ID NO:48 | SEQ ID NO:49 |
| 13 | SEQ ID NO:31 | SEQ ID NO:46 | SEQ ID NO:47 | SEQ ID NO:34 | SEQ ID NO:51 | SEQ ID NO:49 |
| 14 | SEQ ID NO:25 | SEQ ID NO:50 | SEQ ID NO:47 | SEQ ID NO:34 | SEQ ID NO:51 | SEQ ID NO:49 |
| 15 | SEQ ID NO:31 | SEQ ID NO:50 | SEQ ID NO:47 | SEQ ID NO:34 | SEQ ID NO:51 | SEQ ID NO:49 |
| 16 | SEQ ID NO:25 | SEQ ID NO:50 | SEQ ID NO:47 | SEQ ID NO:34 | SEQ ID NO:51 | SEQ ID NO:49 |
| 17 | SEQ ID NO:52 | SEQ ID NO:39 | SEQ ID NO:53 | SEQ ID NO:54 | SEQ ID NO:55 | SEQ ID NO:42 |
| 18 | SEQ ID NO:25 | SEQ ID NO:56 | SEQ ID NO:47 | SEQ ID NO:34 | SEQ ID NO:35 | SEQ ID NO:49 |
| 19 | SEQ ID NO:11 | SEQ ID NO:12 | SEQ ID NO:13 | SEQ ID NO:86 | SEQ ID NO:15 | SEQ ID NO:16 |
| 20 | SEQ ID NO:52 | SEQ ID NO:39 | SEQ ID NO:53 | SEQ ID NO:86 | SEQ ID NO:15 | SEQ ID NO:16 |
| 21 | SEQ ID NO:11 | SEQ ID NO:12 | SEQ ID NO:13 | SEQ ID NO:54 | SEQ ID NO:55 | SEQ ID NO:42 |

In some embodiments, an antibody of the invention comprises a VH and a VL as shown in any combination in Table B below.

**Table B**

| Combination# | VH, which comprises or consists of the following sequence (or sequence at least 95% identical thereto) | VL, which comprises or consists of the following sequence (or sequence at least 95% identical thereto) |
|---|---|---|
| 1 | SEQ ID NO:61 | SEQ ID NO:62 |
| 2 | SEQ ID NO:65 | SEQ ID NO:66 |
| 3 | SEQ ID NO:67 | SEQ ID NO:68 |
| 4 | SEQ ID NO:69 | SEQ ID NO:70 |
| 5 | SEQ ID NO:71 | SEQ ID NO:68 |
| 6 | SEQ ID NO:72 | SEQ ID NO:73 |
| 7 | SEQ ID NO:76 | SEQ ID NO:77 |
| 8 | SEQ ID NO:80 | SEQ ID NO:79 |
| 9 | SEQ ID NO:82 | SEQ ID NO:83 |
| 10 | Any of SEQ ID NO:87-110 | Any of SEQ ID NO:135-158 |
| 11 | Any of SEQ ID NO:111-134 | Any of SEQ ID NO:159-182 |
| 12 | SEQ ID NO:89 | SEQ ID NO:137 |
| 13 | SEQ ID NO:106 | SEQ ID NO:154 |
| 14 | SEQ ID NO:114 | SEQ ID NO:162 |

In some embodiments, an antibody of the invention comprises a VH and a VL as shown in any combination in Table C below.

**Table C**

| Combination # | VH, which comprises or consists of the following sequence (or sequence at least 95% identical thereto) | VL, which comprises or consists of the following sequence (or sequence at least 95% identical thereto) | Combination # | VH, which comprises or consists of the following sequence (or sequence at least 95% identical thereto) | VL, which comprises or consists of the following sequence (or sequence at least 95% identical thereto) |
|---|---|---|---|---|---|
| 15 | SEQ ID NO:87 | SEQ ID NO: 135 | 39 | SEQ ID NO:111 | SEQ ID NO:159 |
| 16 | SEQ ID NO:88 | SEQ ID NO: 136 | 40 | SEQ ID NO: 112 | SEQ ID NO: 160 |
| 17 | SEQ ID NO:89 | SEQ ID NO: 137 | 41 | SEQ ID NO:113 | SEQ ID NO:161 |
| 18 | SEQ ID NO:90 | SEQ ID NO: 138 | 42 | SEQ ID NO: 114 | SEQ ID NO: 162 |
| 19 | SEQ ID NO:91 | SEQ ID NO: 139 | 43 | SEQ ID NO:115 | SEQ ID NO: 163 |
| 20 | SEQ ID NO:92 | SEQ ID NO: 140 | 44 | SEQ ID NO: 116 | SEQ ID NO: 164 |
| 21 | SEQ ID NO:93 | SEQ ID NO: 141 | 45 | SEQ ID NO: 117 | SEQ ID NO: 165 |
| 22 | SEQ ID NO:94 | SEQ ID NO: 142 | 46 | SEQ ID NO:118 | SEQ ID NO: 166 |
| 23 | SEQ ID NO:95 | SEQ ID NO: 143 | 47 | SEQ ID NO:119 | SEQ ID NO: 167 |
| 24 | SEQ ID NO:96 | SEQ ID NO: 144 | 48 | SEQ ID NO: 120 | SEQ ID NO: 168 |
| 25 | SEQ ID NO:97 | SEQ ID NO:145 | 49 | SEQ ID NO:121 | SEQ ID NO: 169 |
| 26 | SEQ ID NO:98 | SEQ ID NO: 146 | 50 | SEQ ID NO: 122 | SEQ ID NO:170 |
| 27 | SEQ ID NO:99 | SEQ ID NO: 147 | 51 | SEQ ID NO: 123 | SEQ ID NO: 171 |
| 28 | SEQ ID NO:100 | SEQ ID NO: 148 | 52 | SEQ ID NO: 124 | SEQ ID NO:172 |
| 29 | SEQ ID NO:101 | SEQ ID NO: 149 | 53 | SEQ ID NO: 125 | SEQ ID NO:173 |
| 30 | SEQ ID NO:102 | SEQ ID NO:150 | 54 | SEQ ID NO: 126 | SEQ ID NO:174 |
| 31 | SEQ ID NO:103 | SEQ ID NO:151 | 55 | SEQ ID NO: 127 | SEQ ID NO:175 |
| 32 | SEQ ID NO:104 | SEQ ID NO:152 | 56 | SEQ ID NO: 128 | SEQ ID NO:176 |
| 33 | SEQ ID NO:105 | SEQ ID NO:153 | 57 | SEQ ID NO: 129 | SEQ ID NO:177 |
| 34 | SEQ ID NO:106 | SEQ ID NO:154 | 58 | SEQ ID NO: 130 | SEQ ID NO:178 |
| 35 | SEQ ID NO:107 | SEQ ID NO: 155 | 59 | SEQ ID NO:131 | SEQ ID NO:179 |
| 36 | SEQ ID | SEQ ID | 60 | SEQ ID | SEQ ID |
| | NO:108 | NO:156 | | NO: 132 | NO:180 |
| 37 | SEQ ID NO:109 | SEQ ID NO:157 | 61 | SEQ ID NO: 133 | SEQ ID NO:181 |
| 38 | SEQ ID NO:110 | SEQ ID NO:158 | 62 | SEQ ID NO:134 | SEQ ID NO:182 |

In some embodiments, BDCA2 antibody is IgG antibody, e.g., IgG1, IgG2, IgG3, or IgG4 antibody, or modified version thereof, as described in following section.

In some embodiments, Fc region variant may be generated by introducing one or more amino acid modifications into Fc region of antibody provided herein. The Fc region variant may comprise human Fc region sequence (e.g., human IgG1, IgG2, IgG3 or IgG4 Fc region) comprising amino acid modification (e.g. substitution) at one or more amino acid positions.

In some embodiments, it may be desirable to create cysteine engineered antibodies, e.g., "thioMAbs", in which one or more residues of antibody are substituted with cysteine residues.

In some embodiments, antibodies provided herein may be further modified to contain additional non-protein moieties that are known in the art and readily available. Moieties suitable for derivatization of antibodies include, but is not limited to, water-soluble polymers. Non-limiting examples of water-soluble polymers include, but is not limited to, polyethylene glycol (PEG), copolymers of ethylene glycol/propylene glycol, carboxymethylcellulose, dextran, polyvinyl alcohol, polyvinylpyrrolidone, poly-1, 3-dioxane, poly-1, 3, 6-trioxane, ethylene/maleic anhydride copolymer, polyaminoacids (either homopolymers or random copolymers), and dextran or poly (n-vinyl pyrrolidone) polyethylene glycol, propylene glycol homopolymers, polypropylene oxide/ethylene oxide copolymers, polyoxyethylene polyols (e.g., glycerol), polyvinyl alcohol, and mixtures thereof.

### Expression of Antibodies

In yet another aspect, the invention provides a polynucleotide molecule encoding the anti-BDCA2 antibody or antigen-binding fragment thereof described herein. The polynucleotide molecule can comprise a polynucleotide molecule that encodes the amino acid sequence of the light chain variable region and/or the heavy chain variable region of the antibody, or a polynucleotide molecule that comprises the amino acid sequence encoding the light chain and/or the heavy chain of the antibody.

In some embodiments, the polynucleotide molecules encoding antibodies of the invention include those that have been mutated by nucleotide deletion, insertion, or substitution, but still have at least about 60, 70, 80, 90, 95, or 100% identity with the corresponding coding regions of the CDRs depicted in the sequences described hereinabove.

In a further aspect, the invention provides an expression vector comprising the polynucleotide molecule as described herein, preferably the vector is a eukaryotic expression vector. In some embodiments, the polynucleotide molecule as described herein is comprised in one or more expression vectors.

In a further aspect, the invention provides a host cell comprising the polynucleotide molecule as described herein or the expression vector as described herein, preferably the host cell is a eukaryotic cell, more preferably a mammalian cell.

In yet another aspect, the invention provides a method for making an anti-BDCA2 antibody or antigen-binding fragment thereof as described herein, the method comprises expressing the antibody or antigen-binding fragment thereof in a host cell described herein under conditions suitable for expression of the antibody or antigen-binding fragment thereof, and recovering the expressed antibody or antigen-binding fragment thereof from the host cell.

The invention provides a mammalian host cell for expressing a recombinant antibody of the invention, including a number of immortalized cell lines available from the American Type Culture Collection (ATCC). These include, inter alia, Chinese Hamster Ovary (CHO), NS0, SP2/0 cells, HeLa cells, baby hamster kidney (BHK) cells, monkey kidney cells (COS), human hepatocellular carcinoma cells, A549 cells, 293T cells and numerous other cell lines. Mammalian host cells include human, mouse, rat, dog, monkey, pig, goat, cow, horse, and hamster cells. Particularly preferred cell lines are selected by determining which cell lines have high expression levels.

In one embodiment, the invention provides a method of making an anti-BDCA2 antibody, where the method comprises culturing the host cell for a period of time sufficient to allow for expression of the antibody in the host cell upon the expression vector is introduced into a mammalian host cell, or more preferably secretion of the antibody into the culture medium in which the host cell is grown, thereby producing the antibody.

The antibody can be recovered from the culture medium using standard protein purification methods. Antibody molecules prepared as described herein can be purified by known state of the art techniques, such as high performance liquid chromatography, ion exchange chromatography, gel electrophoresis, affinity chromatography, size exclusion chromatography, etc. The actual conditions used to purify a particular protein also depend on such factors as net charge, hydrophobicity, hydrophilicity, and the like. These will be readily apparent to those skilled in the art. The purity of an antibody molecule of the invention can be determined by any one of a number of well-known analytical methods, including size exclusion chromatography, gel electrophoresis, high performance liquid chromatography, and the like.

It is likely that antibodies expressed by different cell lines or expressed in transgenic animals have different glycosylation from each other. However, all antibodies encoded by the nucleic acid molecules provided herein or comprising the amino acid sequences provided herein are part of the invention, regardless of glycosylation of antibody. Again, in certain embodiments, non-fucosylated antibodies are advantageous because they generally have more potent efficacy than their fucosylated counterparts in vitro and in vivo, and are unlikely to be immunogenic because their glycostructures are normal components of native human serum IgG.

### Pharmaceutical Compositions and Pharmaceutical Formulations

In yet another aspect, the invention provides a pharmaceutical composition comprising an anti-BDCA2 antibody or antigen-binding fragment thereof as described herein, a polynucleotide molecule described herein, an expression vector described herein, or a host cell described herein, and pharmaceutically acceptable carriers or excipients. It will be understood that the anti-BDCA2 antibodies or pharmaceutical compositions thereof provided by the invention can integrate suitable carriers, excipients, and other agents in the formulation for co-administration, thereby providing improved transfer, delivery, tolerance, and the like.

The term "pharmaceutical composition" refers to preparation which permits biological activity of active ingredient contained therein to be present in such form as to be effective, and which contains no additional components which are unacceptably toxic to subject to which formulation would be administered.

Pharmaceutical formulations comprising an anti-BDCA2 antibody described herein, preferably in the form of aqueous solutions or lyophilized formulations, can be prepared by mixing an anti-BDCA2 antibody of the invention having the desired degree of purity with one or more optional pharmaceutically acceptable excipients (Remington's Pharmaceutical Sciences, 16th edition, Osol, A. Ed. (1980)).

The pharmaceutical composition or formulation of the invention may further comprise one or more further active ingredients, the active ingredient is as required for the particular indication being treated, preferably those active ingredients with complementary activities that do not adversely affect each other. In some embodiments, additional active ingredients are immune checkpoint inhibitors, growth inhibitory agents, suitably present in combination in amounts that are effective for the intended use. In some embodiments, the pharmaceutical compositions of the invention further comprise a composition of polynucleotide molecules encoding anti-BDCA2 antibodies.

In a further aspect, the invention provides a pharmaceutical combination comprising an antibody or antigen-binding fragment thereof described herein, a polynucleotide molecule described herein, an expression vector described herein, a host cell described herein, or a pharmaceutical composition described herein, and one or more additional therapeutic agents.

In a further aspect, the invention provides a kit comprising an antibody or antigen-binding fragment thereof described herein, a polynucleotide molecule described herein, an expression vector described herein, a host cell described herein, or a pharmaceutical composition described herein.

### Pharmaceutical Uses and Methods of Treatment

Any of the anti-BDCA2 antibodies or corresponding immunoconjugates provided herein can be used in methods of treatment. It is also understood that where "antibodies" are discussed, compositions comprising antibodies are also included. The anti-BDCA2 antibodies of the invention can be used in a therapeutically effective amount or a prophylactically effective amount in the methods of treatment or prevention described in any of the embodiments of the invention.

In a further aspect, the invention provides the use of an antibody or an antigen-binding fragment thereof as described herein, a pharmaceutical composition as described herein, or a pharmaceutical combination as described herein in the manufacture of a medicament for the treatment and/or prevention of a BDCA2-mediated disease, preferably the disease is an inflammatory disease; more preferably, the inflammatory disease is selected from systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis and polymyositis.

In a further aspect, the invention provides an antibody or an antigen-binding fragment thereof as described herein, a pharmaceutical composition as described herein or a pharmaceutical combination as described herein for use in the treatment and/or prevention of a BDCA2-mediated disease, preferably the disease is an inflammatory disease; more preferably, the inflammatory disease is selected from systemic lupus erythematosus, discoid lupus, lupus nephritis, rheumatoid arthritis, inflammatory bowel disease, systemic sclerosis (scleroderma), psoriasis, type I diabetes, dermatomyositis and polymyositis.

In yet another aspect, the invention provides a method of treating and/or preventing a BDCA2-mediated disease or disorder, comprising administering to a subject in need thereof a therapeutically or prophylactically effective amount of an antibody or antigen-binding fragment thereof described herein, a pharmaceutical composition described herein, or a pharmaceutical combination described herein, preferably the disease is an inflammatory disease; more preferably, the inflammatory disease is selected from systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis and polymyositis.

In some embodiments, the modes of administration of the invention include, but are not limited to, orally, intravenously, subcutaneously, intramuscularly, intra-arterially, intra-articularly (e.g., in arthritic joints), by inhalation, aerosol delivery, or focal local administration, and the like.

The term "treatment" refers to clinical intervention in an attempt to alter the natural course of disease in the individual being treated. Desirable effects of treatment include, but are not limited to, preventing occurrence or recurrence of disease, alleviation of symptoms, diminishment of any undesirable or direct or indirect pathological consequences of the disease, decreasing the rate of disease progression, amelioration or palliation of the disease state and remission or improvement of prognosis. The antibodies of the invention are capable of reducing the severity of the condition, i.e. exerting their therapeutic effect, in one or more aspects. In certain embodiments, one or more of the following are manifested: an increase in the average life (survival) of patients; delay in disease progression; reduced need for medical care.

The invention also provides co-administration of therapeutically effective amounts of one or more therapies (e.g., treatment modalities and/or other therapeutic agents) to a subject. Antibodies of the invention may be used alone or in combination with other therapeutic agents in a therapy. In some embodiments, the antibodies of the invention are co-administered with at least one additional therapeutic agent.

### Methods for Diagnosing and Detecting

In yet another aspect, the invention provides a method for detecting the presence of BDCA2 in a sample using the antibodies or antigen-binding fragments thereof described herein. The term "detecting" as used herein includes quantitative or qualitative detection. In some embodiments, the sample is a biological sample. In certain embodiments, the biological sample is blood, serum, or other liquid sample of biological origin. In certain embodiments, the biological sample comprises a cell or tissue. In certain embodiments, BDCA2 is human BDCA2 or cynomolgus BDCA2. The method comprises a step of contacting an antibody or antigen-binding fragment thereof described herein or a detection composition containing the antibody or antigen-binding fragment thereof with a sample, and a step of detecting the presence or absence of a conjugate or binding signal resulting from binding of the antibody or antigen-binding fragment thereof to BDCA2. For use in detection, the antibody or antigen-binding fragment thereof described herein can be labeled to indicate whether the conjugate has been formed. In certain embodiments, the method can be an in vitro or in vivo method.

In some embodiments, BDCA2 is detected prior to treatment, e.g., prior to initiation of treatment or prior to certain treatment after treatment interval. In one embodiment, anti-BDCA2 antibody or antigen-binding fragment thereof for use in method of diagnosis or detection is provided.

The positive progress of the invention lies in that:
Antibodies targeting BDCA2 of the invention involve one or more of the following advantages:
1. The antibodies of the invention shows stronger active binding to CHOK1-humanBDCA2 cells and/or 293F-cynoBDCA2 cells than the anti-BDCA2 antibody BIIB059 in the prior art;
2. The antibodies of the invention mediate a stronger inhibition of CpG-A stimulated release of cytokine IFNαfrom human PBMC cells than the anti-BDCA2 antibody BIIB059 in the prior art;
3. The antibodies of the invention result in higher internalization efficiency on cells expressing BDCA2;
4. The humanized antibody of the invention has a higher affinity for the human BDCA2 antigen than the anti-BDCA2 antibody BIIB059 in the prior art.
5. There is no expression of BDCA2 gene in non-primates such as mice, rats and rabbits, whereas the major diseases treated by BDCA2 antibodies (such as epidermal lupus erythematosus and systemic lupus erythematosus) have not been successfully established in any pharmacodynamic testing models in primates. According to the *in vitro* and clinical studies, the antibody BIIB059 exhibits an induction of BDCA2 internalization and an inhibitory effect on the function of secreting type I interferon of PBMC in *in vitro* experiments, while achieves a symptom-relieving effect in clinical experimental studies against epidermal lupus erythematosus and systemic lupus erythematosus (Werth VP et al. N Engl J Med 2022; 387: 321-31. Furie RA et al. N Engl J Med 2022; 387: 894-904.), so that it could be presumed that the anti-BDCA2 antibodies of the invention have a better therapeutic effect for the above-mentioned diseases or disorders clinically.

The invention includes all combinations of said specific embodiments. Further embodiments of the invention and the full scope of applicability will become apparent from the detailed description provided hereinafter. It should be understood that while the detailed description and the specific examples indicate the preferred embodiments of the invention, but these descriptions and examples are provided by way of illustration only, since various changes and modifications within the spirit and scope of the invention will become apparent to those skilled in the art from this detailed description. All publications, patents and patent applications cited herein, including citations, are incorporated herein by reference in their entirety for all purposes. The compounds of the invention can be prepared by a variety of synthetic methods well known to those skilled in the art, including the specific embodiments enumerated below, those formed by combining them with other methods, and equivalents thereof well known to those skilled in the art, and preferred embodiments include, but are not limited to, the examples of the invention.

The invention employs at least the following acronyms:
His-tag for histidine tag;
Fc tag for crystallizable fragment tag;
ECD for an extracellular domain;
PEI for polyethyleneimine;
BSA for bovine serum albumin;
PBS for phosphate buffered saline;
FBS for fetal bovine serum;
CFSE for carboxyfluorescein diacetate succinimidyl ester;
APC for ectopic phycocyanin;
NA-PE for phycoerythrin-labeled neutravidin;
PE for phycoerythrin;
TMB for 3, 3', 5, 5'-tetramethylbenzidine;
HEPES: hydroxyethyl piperazine ethanesulfonic acid buffer;
DTT: dithiothreitol.

### Examples

The invention is illustrated by the following examples, without intending to limit the invention in any way, the invention has been described in detail herein, with specific embodiments thereof also disclosed. It will be apparent to those skilled in the art that various changes and modifications to the specific embodiments of the invention will be made without departing from the spirit and scope of the invention.

### Example 1, Preparation of Recombinant Proteins for Activity Detection of Anti-BDCA2 Antibody

The cDNA sequence encoding the extracellular domain (Asn45-Ile213) of human BDCA2 (hBDCA2 or human BDCA2) was synthesized by Genscript Corporation following Genbank reference sequences The amplified fragment was cloned into an independently constructed eukaryotic expression plasmid system (pCP-EF1a-CMV-FcεRIγ) comprising a human-derived Fc (hIgG1) fragment using conventional cloning techniques, and a recombinant fusion protein expression plasmid Human BDCA2 Fc (pCP-EF1a-BDCA2-CMV-FcεRIγ) was generated. Human BDCA2 Fc recombinant protein (Fc region lies in BDCA2 extracellular domain in N-terminal direction, fused by linker) was obtained using expression cells 293E for the expression and size exclusion chromatography column for the purification, and then get identified by polyacrylamide gel electrophoresis.

The cDNA sequence encoding the extracellular domain (Asn45-Ile212) of cynomolgus BDCA2 (Cyno BDCA2) was synthesized by Genscript Corporation following Genbank reference sequences, and 6-His expression sequences were added to the N-terminus of cDNA. The fragment was cloned into an independently constructed eukaryotic expression plasmid system (pCP-EF1a-CMV-FcεRIγ) using conventional cloning techniques, and a recombinant fusion protein expression plasmid Cyno BDCA2 His (pCP-EF1a-Cyno BDCA2-CMV-FcεRIγ) was generated. Cyno BDCA2 His recombinant protein was obtained using expression cells 293E for the expression and size exclusion chromatography column for the purification, and then get identified by polyacrylamide gel electrophoresis.

It was identified that concentration, total amount and endotoxin content of both proteins obtained were suitable for subsequent applications.

The amino acid sequence of Human BDCA2 Fc as set forth in SEQ ID NO: 189, and NCBI accession number Q8WTT0 used as the protein sequence of Human BDCA2, the amino acid sequence of Cyno BDCA2 His as set forth in SEQ ID NO: 190, and NCBI accession number A0A2K5UWP4-1 used as the protein sequence of Cyno BDCA2, were used for the activity assay of the antibodies of the invention.

Reference antibody BIIB059 sequence to which BDCA2 specifically binds was derived from patent US9902775B2, wherein heavy chain sequence corresponds to patent sequence No. 4 and light chain sequence corresponds to patent sequence No. 3., and produced using conventional production processes for recombinant antibodies.

### Example 2, Construction of BDCA2-Expressing Cell Line for Activity Detection of Anti-BDCA2 Antibody

The cDNA sequence encoding human BDCA2 was synthesized by Genscript Corporation following Genbank reference sequences. The amplified fragment was cloned into an independently constructed lentiviral packaging plasmid system (PLvx-EF1a-CMV-hFcεRIγ-IRES-puro) using conventional cloning techniques, and a lentiviral packaging plasmid Human BDCA2 (PLvx-EF1a-hBDCA2-CMV-hFcεRIy-IRES-puro) was produced.

The cDNA sequence encoding cynomolgus BDCA2 was synthesized by Genscript Corporation following Genbank reference sequences. The amplified fragment was cloned into an independently constructed lentiviral packaging plasmid system (PLvx-EF1a-CMV-hFcεRIγ-IRES-puro) using conventional cloning techniques, and a lentiviral packaging plasmid Cyno BDCA2 (PLvx-EF1a-cynoBDCA2-CMV-cynoFcεRIγ-IRES-puro) was produced.

PLvx-EF1 a-hBDCA2-CMV-hFcεRIγ-IRES-puro plasmid or PLvx-EF1a-cynoBDCA2-CMV-cynoFcεRIγ-IRES-puro was transferred to 293T cells by transient method using 293T as the host cell. Cell culture supernatants containing human BDCA2 or Cyno BDCA2 expressing virus at 48 and 72 hours post-transfection was collected and concentrated to 1E8 EU/mL.

CHOK1 cells were cultured in advance till logarithmic growth phase and were then digested, plated 6-well cell culture plates at 1E5/well, cultured overnight to adhere. Subsequently, the concentrated human BDCA2 virus was added to pre-adherent 6-well plate cells at increasing amounts of infection (100µ1, 200µ1), cells infected were screened with puromycin-containing medium, then the amount of antigen expression was measured by flow cytometry, and the cells were subclonally treated. Flow cytometry was performed using the reference antibody BIIB059 and the results showed significant binding of BDCA2 relative to the control hlgG. Monoclones with high antigen expression were picked and expanded as later cell lines.

293F cells were cultured in advance till logarithmic growth phase and were then digested, plated 6-well cell culture plates at 1E5/well, cultured overnight to adhere. Subsequently, the concentrated cyno BDCA2 virus was added to pre-adherent 6-well plate cells at increasing amounts of infection (100µ1, 200µ1), cells infected were screened with puromycin-containing medium, then the amount of antigen expression was measured by flow cytometry, and the cells were subclonally treated. Flow cytometry was performed using the reference antibody BIIB059 and the results showed significant binding of BDCA2 relative to the control hlgG. Monoclones with high antigen expression were picked and expanded as later cell lines.

### Example 3, Preparation of Mouse Hybridoma Cells

### 3.1 Immunization of mice and detection of serum titers

### 3.1.1 Protocol for immunization of mice

A total of 3 groups of mice (5 per group) were immunized with 2 immunization routes, 14 days apart per immunization. Group 1 of 5 Balb/c mice (purchased from SLAC, BALB/c, female, 8 weeks) and Group 2 of 5 SJL mice (purchased from SLAC, BALB/c, female, 8 weeks) using a biolistic (pCP-EF1a-BDCA2-CMV-FcεRIγ) plus protein (humanBDCA2 Fc) mixing protocol for 5 times: the first 4 biolistic immunizations, at a dose of 4µg every time; the 5th protein immunization, at a dose 25g/mouse. Group 3 of 5 SJL mice (purchased from SLAC, female, 8 weeks) were immunized with pure protein (humanBDCA2 Fc) for a total of 3 immunizations at 50 g/dose for the first and 25 g/dose for the second. See Table 1.

**Table 1 Protocol for immunization of mice**

| Immunogen | Groupings of Animals | Strain | Route | Dose(µg/animal) | Adjuvant |
|---|---|---|---|---|---|
| Plasmid + Protein Mix | G1 (9731,9732,9733,9734,9735) | Balb/c | Gene gun | 4/4/4/4 ; 25 | GM-CSF & FLT3L |
| | G2 (9736,9737,9738,9739,9740) | SJL | | | |
| BDCA2-Fc protein | G3 (9946,9947,9948,9949,9950) | SJL | I.P. | 50/25/25 | CFA/IFA /IFA |

### 3.1. 2 Detection of immunization titers of mice

Serum samples from immunized mice were collected at a plurality of time points, ELISA and FACS assays were performed respectively. Based on the results of several assays (with the last mouse serum assay (TB) as the main reference factor, detailed results not shown), a total of 4 mice were selected and 2 fusions were made: Fusion # F0223 picked two mice with the highest fluorescence intensity of Balb/c # 9735 in G1 group and SJL # 9740 in G2 group; fusion # F0511 picked two mice with the highest fluorescence intensity of SJL # 9947 and SJL # 9948 in the G3 group.

### 3.2 Fusion of cells

The splenocytes were collected after sacrifice of mice on fourth day after last dose of boost and lymphocyte-enriched suspensions were taken after milling in normal saline and mixed with mouse myeloma cells Sp2/0 by conventional electro-transduction for cell fusion using high-efficiency electro-fusion.

The fused cells were diluted into DMEM medium containing HT (hypoxanthine, thymidine), plated to 96-well plates at a certain ratio and cultured in 5% CO₂ at 37°C incubator overnight, and DMEM medium containing 2*HAT (containing hypoxanthine, methotrexate and thymidine) was added after 24h to screen for successfully fused cells (hybridoma cells).

The formulation of DMEM complete medium was: 15% FBS (fetal bovine serum) + 1:50 L-glutamine + 100 U/mL Penicillin&Streptomycin + 1: 100 OPI (oxaloacetate, pyruvate, and insulin), and the conditions of incubator were 8% CO₂, 37°C.

### Example 4, Screening of Mouse Hybridoma Cells and Purification of Anti-BDCA2 Mouse Antibodies

The expression of BDCA2 antibodies in the supernatant was detected with an Acumen laser cell detector after culturing and screening of the fused cells for about 10 days, positive cells for detection were CHOK1-humanBDCA2, negative cells were CHOK1-blank, and positive control antibody was the reference antibody BIIB059. According to Acumen results, positive clones were picked into 24-well plate for expansion culture, and supernatants from the expansion culture in the 24-well plate were collected after 3 days of culture using the following method: 1) FACS for the binding activity to CHOK1-humanBDCA2 cells and 293F-cynoBDCA2 cells; 2) ELISA for the binding activity to humanBDCA2 Fc protein and cynoBDCA2 His protein; 3) detection of inhibitory activity in those experiments where CpG-A stimulates the production of cytokine IFNα by PBMC (inhibiting the production of IFNα).

189 hybridoma cells were screened by Acumen binding assay in the polyclonal hybridoma cells obtained from the fusion of F0223, and the secreted antibody can bind specifically to hBDCA2. A further scaling up to re-screening in 24-well plates of these 189 hybridoma cells positive for binding showed that antibodies expressed by 28 hybridoma cells could bind hBDCA2 and cyno BDCA2 in an ELISA assay, bind hBDCA2 and cyno BDCA2 expressed on cell surface in FACS, and inhibit CpG induction of IFNα secretion from PBMC cells in the IFNα-blocking experiments. The 28 hybridoma cells were then subcloned. 18 blocking monoclonal cell lines, numbered mAb001-mAb018, were screened out in the subclones.

168 hybridoma cells were screened by Acumen binding assay in the polyclonal hybridoma cells obtained from the fusion of F0511, and the secreted antibodies can bind specifically to hBDCA2. A further scaling up to re-screening in 24-well plates of these 168 hybridoma cells positive for binding showed that the antibodies expressed by 35 hybridoma cells can bind hBDCA2 and cyno BDCA2 in an ELISA assay, bind hBDCA2 and cyno BDCA2 expressed on cell surface in FACS, and inhibit CpG induction of IFNα secretion from PBMC cells in the IFNα-blocking experiments. The 35 hybridoma cells were then subcloned. 16 blocking monoclonal cell lines, numbered mAb019-mAb034, were screened out in the subclones.

A total of 34 positive monoclones were finally obtained via 2 fusions and screening. The secreted antibodies were purified and analyzed that the concentration, quality, purity and endotoxin content met the requirements of subsequent experiments.

### Example 5, Determination of the performance of anti-BDCA2 mouse antibody

The resulted mouse antibodies all had good binding activity to humanBDCA2 Fc protein, with EC₅₀ < 0.3 nM as measured by ELISA. Mouse antibodies had good binding to cynoBDCA2 His protein, and wherein most antibodies showed EC₅₀ < 0.5 nM as measured by ELISA.

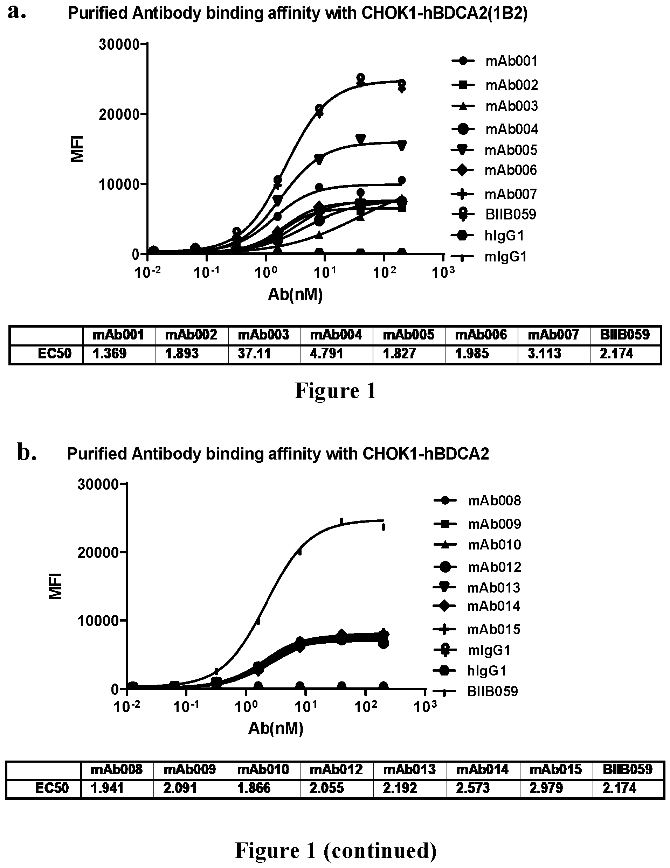

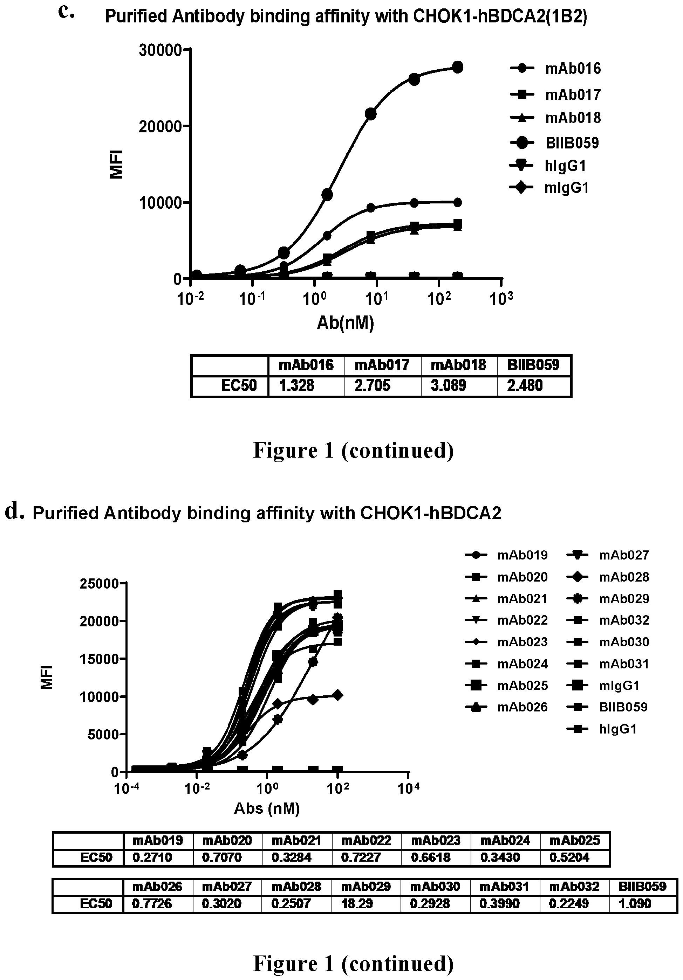

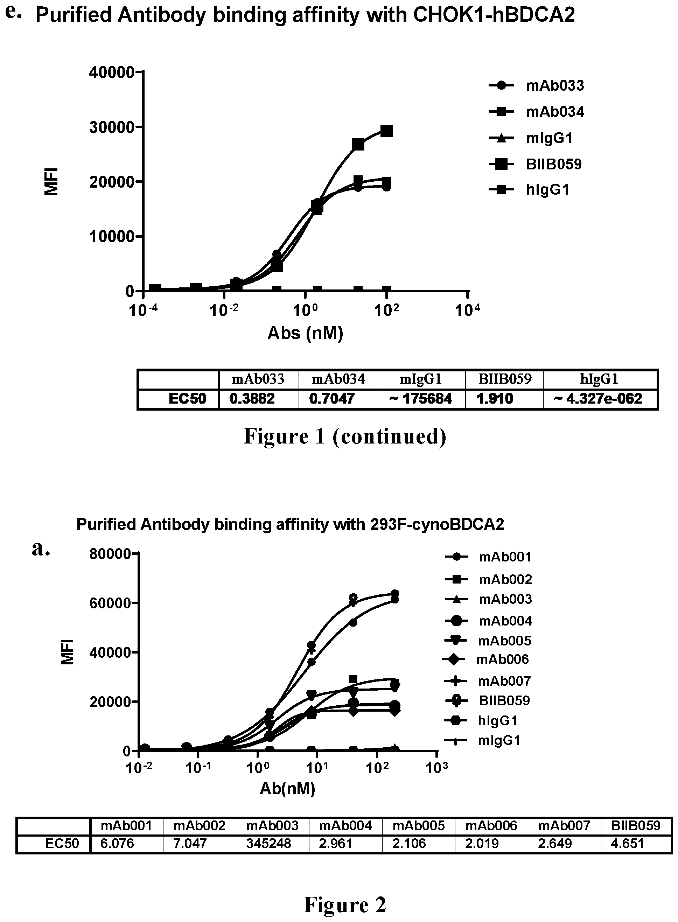

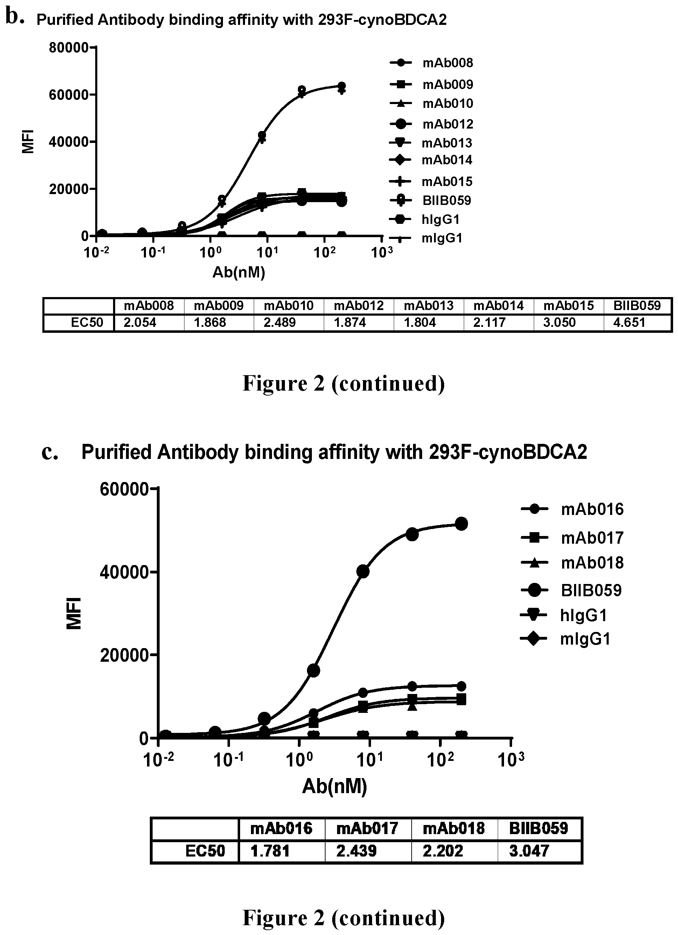

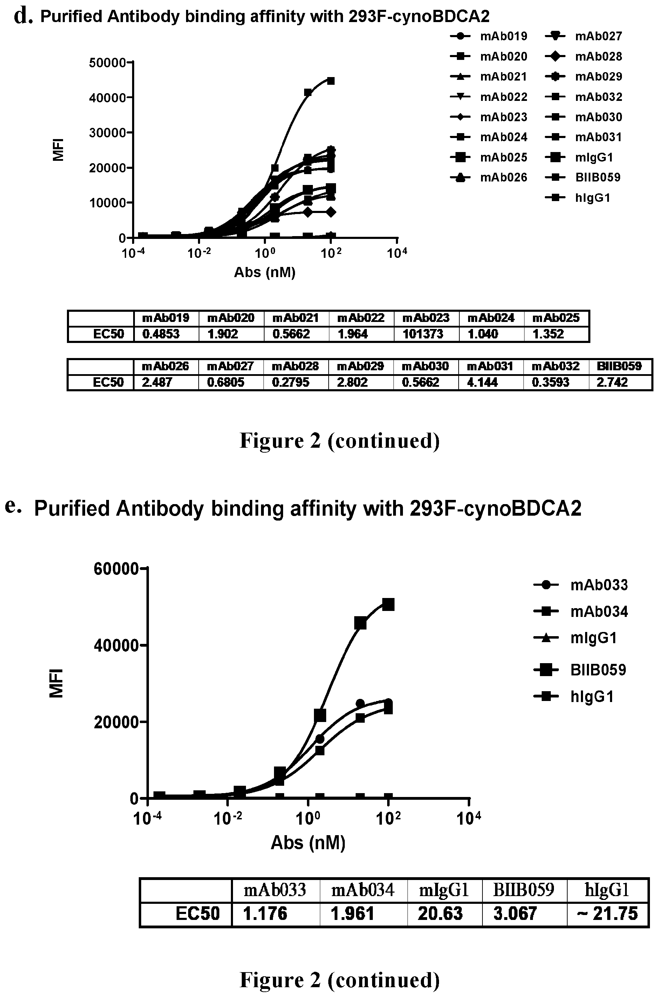

All mouse antibodies bound to CHOK1-humanBDCA2 cells (see results in Figures 1a-1e), with EC₅₀ superior or comparable to the control antibody BIIB059. Most mouse antibodies bound to 293F-cynoBDCA2 cells (see results in Figures 2a-2e), with EC₅₀ superior or comparable to the control antibody BIIB059.

Thirty-two antibodies binding to both CHOK1-humanBDCA2 and 293F-cynoBDCA2 cells were tested for their ability to inhibit cytokine IFNα release in CpG-A stimulated PBMC experiments. The antibodies all had some inhibitory activity. Of these, mAb005, mAb019, mAb020, mAb021, mAb022, mAb024, mAb027, mAb030, mAb033 had IC₅₀ for inhibiting cytokine release of 0.08667 nM, 0.003323 nM, 0.006146 nM, 0.004047 nM, 0.006232 nM, 0.002744 nM, 0.07004 nM, 0.004762 nM, and 0.004083 nM, respectively.

The above experimental procedure is shown below, wherein mAb001-mAb034 are mouse antibodies of the invention, BIIB059 is a reference antibody as described before, hIgG1 is a human IgG1 negative control and mIgG1 is a mouse IgG1 negative control.

### 5.1 Detection of Binding Activity of Mouse Antibodies to Proteins by ELISA

HumanBDCA2 Fc protein or cynoBDCA2 His protein was diluted with PBS to 1µg/mL and added to ELISA microplates at 100µL/well and incubated at 4°C overnight. The plates were blocked with ELISA blocking solution (PBS phosphate buffer with 1% BSA, pH 7.4, the percentages representing mass percentages) at 37°C for 2 hours. Sequential 10-fold serial dilutions of antibodies were added at 8 concentrations (0.0001 nM-100 nM, including 0 point control) in total at 100µL/well. The plates were incubated at 37°C for 1 hour, then washed 3 times and after anti-mouse IgG (Fab specific)-HRP (Sigma, A3682; 1: 5000 dilution) secondary antibodies were added at 100µL/well, were incubated at 37°C for 1h. The plates were washed 3 times, TMB development fluid (EL0009, Huzhou Ying Chuang Biotechnology) was added at 100 µL/well and 50µL 1N hydrochloric acid was added after incubation at 37°C for 10 min to stop the development reaction. OD450nm values were read with ELISA plate reader and the curve was fitted with GraphPad Prism6, and EC₅₀ values were calculated.

### 5.2 Detection of binding activity of mouse antibodies to cells by FACS method

CHOK1-humanBDCA2 and 293F-cynoBDCA2 cells cultured to logarithmic growth phase were trypsinized with TrypLE. The cells were recovered and resuspended to 2×10⁶/mL with FACS buffer (PBS + 2% FBS). The cells were added to 3799 cell plates (Corning) at 100µL/well, and the supernatant was discarded after centrifugation at 300 g. The antibodies were serially diluted with FACS buffer (Id.), the diluted antibodies were added to centrifuged cells and the cells were resuspended. The cells were incubated at 4°C for 1h. The incubated cells were then centrifuged at 300 g for 5 min and washed twice with FACS buffer. 100µL Donkey anti-Mouse IgG (H + L) Highly Cross-Adsorbed Secondary Antibody-Alexa Fluor 488 (Invitrogen, A21202; 1: 1000) secondary antibody was added to the cells, which were washed twice with FACS buffer after incubation at 4°C for 1h, resuspended with PBS, analyzed with FACS (BD FACS CantoTM II) instrument. The curve was fitted with GraphPad Prism6, and EC₅₀ values were calculated.

### 5.3. Capability of the mouse antibodies to inhibit CpG-A-stimulated release of cytokine IFNα from human PBMC cells

1) The cryopreserved human PBMC cells were recovered, incubated overnight with complete medium (1640 + 10% FBS + l×NEAA +l×L-Glutamine) for 18hrs. The PBMC cells were collected on following day, centrifuged at 600g for 8 minutes, and the supernatant was discarded. The cells were resuspended with media (1640 + 10% FBS) to 8×10⁶/mL, and added to 3799 cell culture plates at 100µL/well. The antibodies to be tested were serially diluted with medium (1640 + 10% FBS) in 10 levels (final concentration to be 0.0001 nM-10 nM), and diluted antibodies were added to PBMC at 50µL/well. The cells were resuspended and incubated at 37°C for 6 hrs. 50µL of 2µM CpG-A (Invitrogen, tlrl-2216-5) diluted with medium (1640 + 10% FBS) was added to each well and mixed and continued to incubate at 37°C for 20 hrs. The cell culture plates were centrifuged at 400 g for 5 minutes on the third day and the cell culture supernatants were collected and assayed for cytokine IFNα in the supernatants.
2) Detection of cytokine IFNα by ELISA

Antibody MT1/3/5 (Mabtech, 3425-1H-20) was diluted with PBS to 4µg/mL and added to CORNING ELISA plates at 100 µL per well, and were incubated at 4°C overnight to coat the plates. Then, the antibodies used to coat the plates were discarded, and the blocking solution (PBS + 1% BSA) was added to the plates at 300 µL per well, and incubated at 4°C overnight. The supernatants to be tested were diluted with blocking solution to the appropriate concentration (selected from 1: 3-1: 7 as the case may be), and were added to the coated ELISA plates at 100 µL per well. IFNα standards for detection were diluted in 2-fold series with blocking solution (top concentration was 1000 pg/mL, 8 concentration points, the last point was 0), and were added to the coated ELISA plates at 100 µL per well. The ELISA plates were incubated in incubator at 37°C for 1 hour. The plates were washed 3 times, and the detection antibody MT2/4/6 (1: 1000) was added at 100 µL per well and incubated I incubator at 37°C for 1 hour. The plates were washed 3 times, and the secondary antibody SA-HRP (1: 1000) was added at 100 µL per well followed by incubation in incubator at 37°C for 0.5h. The plates were washed 3 times, and the TMB development solution was added at 100 µL per well and the reaction was stopped at the appropriate time by the addition of 1M HC1 at 50 µL per well. OD450nm readings were measured with ELISA plate reader, then the inhibition rate was calculated; and the curve was fitted with GraphPad Prism6, and EC₅₀ values were calculated.

### Example 6, Determination of variable region sequences of anti-BDCA2 mouse hybridomas (monoclonal antibodies) (according to Kabat representation)

Sixteen hybridoma monoclones were selected for VH/VL sequence determination based on the results of ability of mouse antibodies to bind antigen at protein and cellular level and ability of antibodies to inhibit cytokine IFNα release caused by CpG-A stimulation in PBMC system.

The DNA coding sequences corresponding to the variable regions of anti-BDCA2 mouse antibodies were determined with a degenerate primer PCR-based method. The cells were collected by centrifugation at 1000 rpm, and total RNA was extracted by Trizol. The total RNA was used as template to synthesize a first strand cDNA which was used as a subsequent template to amplify the corresponding variable region DNA coding sequence by pCR. The primer sequences used in the amplification reactions are complementary to the first framework region of variable region and the constant region of the antibody (Larrick, J.W., et al., 1990, Scand. J. Immunol., 32, 121-128 and Coloma, J. J. et al., (1991) BioTechniques, 11, 152-156). cDNA 1 µ1, 10× PCR buffer 5µ1, upstream and downstream primers 1µ1 each (25 pmol), dNTP 1µ1, 25 mmol PL MgCl₂ 1µ1, H₂O 39µ1 were added into a 50 µI reaction system respectively, pre-denatured at 95 °C for 10 min, Taq enzyme 1µ1 was added, entered into temperature cycling for PCR amplification. Reaction conditions were 94°C denaturing 1 min, 58°C annealing 1 min, 72°C extension 15 s for a total of 32 cycles, then 72°C incubation 10 min. PCR products were recovered and purified. The amplified products were sequenced to give the amino acid sequences of heavy chain variable region and light chain variable region of anti-BDCA2 mouse antibody.

Consensus sequences were searched with NCBI Ig-Blast (http://www.ncbi.nlm.nih.gov/projects/igblast/) in germline and rearranged Ig variable region sequence databases. The amino acid sequences of complementarity determining region (CDR) were identified by sequence annotation and by Internet-based sequence analysis (http://www.imgt.org/IMGT_vquest/vquest and http://www.ncbi.nlm.nih.gov/igblast/) based on Kabat (Wu, T.T and Kabat, E.A. 1970 J. Exp. Med., 132: 211-250) and IMGT systems (Lefranc M.-P. et al., 1999 Nucleic Acids Research, 27, 209-212).

The amino acid sequences of the light and heavy chain variable regions and CDRs of the selected anti-BDCA2 mouse antibodies are shown in Table 2. Multiple sequence alignments and evolutionary tree classifications were performed, and the 16 monoclonal antibody sequences heavy chain variable region VH were mainly classified into 5 major classes according to the results: 1) mAb020, 022, 026; 2) mAb021, 027, 028, 030, 032, 034; 3) mAb019, 024, 033; 4) mAb001, 002; 5) mAb005, 016, where the heavy chain variable regions of mAb028 and mAb030 differ by only 2 amino acids and mAb028 and mAb032 differ by only 1 amino acid. The light chain variable region VL can be classified into 3 main classes: 1) mAb019; 2) mAb021, 027, 028, 030, 032, 034; 3) other antibodies. Where sequences of the light chain variable region of mAb028, 030, 032 are identical.

**Table 2 Amino acid sequences of CDR and variable region of anti-BDCA2 mouse antibodies (KABAT protocol)**

| Mouse antibody | HCDR1 | HCDR2 | HCDR3 | LCDR1 | LCDR2 | LCDR3 | VH | VL |
|---|---|---|---|---|---|---|---|---|
| mAb001 | SEQ ID NO:1 | SEQ ID NO:2 | SEQ ID NO:3 | SEQ ID NO:4 | SEQ ID NO:5 | SEQ ID NO:6 | SEQ ID NO:57 | SEQ ID NO:58 |
| mAb002 | SEQ ID NO:7 | SEQ ID NO:2 | SEQ ID NO:8 | SEQ ID NO:9 | SEQ ID NO:10 | SEQ ID NO:6 | SEQ ID NO:59 | SEQ ID NO:60 |
| mAb005 | SEQ ID | SEQ ID | SEQ ID | SEQ ID | SEQ ID | SEQ ID | SEQ ID | SEQ ID |
| | NO:11 | NO:12 | NO:13 | NO:14 | NO:15 | NO:16 | NO:61 | NO:62 |
| mAb016 | SEQ ID NO:17 | SEQ ID NO:12 | SEQ ID NO:13 | SEQ ID NO:18 | SEQ ID NO:15 | SEQ ID NO:16 | SEQ ID NO:63 | SEQ ID NO:64 |
| mAb019 | SEQ ID NO:19 | SEQ ID NO:20 | SEQ ID NO:21 | SEQ ID NO:22 | SEQ ID NO:23 | SEQ ID NO:24 | SEQ ID NO:65 | SEQ ID NO:66 |
| mAb020 | SEQ ID NO:25 | SEQ ID NO:26 | SEQ ID NO:27 | SEQ ID NO:28 | SEQ ID NO:29 | SEQ ID NO:30 | SEQ ID NO:67 | SEQ ID NO:68 |
| mAb021 | SEQ ID NO:31 | SEQ ID NO:32 | SEQ ID NO:33 | SEQ ID NO:34 | SEQ ID NO:35 | SEQ ID NO:36 | SEQ ID NO:69 | SEQ ID NO:70 |
| mAb022 | SEQ ID NO:25 | SEQ ID NO:26 | SEQ ID NO:37 | SEQ ID NO:28 | SEQ ID NO:29 | SEQ ID NO:30 | SEQ ID NO:71 | SEQ ID NO:68 |
| mAb024 | SEQ ID NO:38 | SEQ ID NO:39 | SEQ ID NO:40 | SEQ ID NO:41 | SEQ ID NO:15 | SEQ ID NO:42 | SEQ ID NO:72 | SEQ ID NO:73 |
| mAb026 | SEQ ID NO:31 | SEQ ID NO:43 | SEQ ID NO:27 | SEQ ID NO:44 | SEQ ID NO:45 | SEQ ID NO:30 | SEQ ID NO:74 | SEQ ID NO:75 |
| mAb027 | SEQ ID NO:31 | SEQ ID NO:46 | SEQ ID NO:47 | SEQ ID NO:34 | SEQ ID NO:48 | SEQ ID NO:49 | SEQ ID NO:76 | SEQ ID NO:77 |
| mAb028 | SEQ ID NO:25 | SEQ ID NO:50 | SEQ ID NO:47 | SEQ ID NO:34 | SEQ ID NO:51 | SEQ ID NO:49 | SEQ ID NO:78 | SEQ ID NO:79 |
| mAb030 | SEQ ID NO:31 | SEQ ID NO:50 | SEQ ID NO:47 | SEQ ID NO:34 | SEQ ID NO:51 | SEQ ID NO:49 | SEQ ID NO:80 | SEQ ID NO:79 |
| mAb032 | SEQ ID NO:25 | SEQ ID NO:50 | SEQ ID NO:47 | SEQ ID NO:34 | SEQ ID NO:51 | SEQ ID NO:49 | SEQ ID NO:81 | SEQ ID NO:79 |
| mAb033 | SEQ ID NO:52 | SEQ ID NO:39 | SEQ ID NO:53 | SEQ ID NO:54 | SEQ ID NO:55 | SEQ ID NO:42 | SEQ ID NO:82 | SEQ ID NO:83 |
| mAb034 | SEQ ID NO:25 | SEQ ID NO:56 | SEQ ID NO:47 | SEQ ID NO:34 | SEQ ID NO:35 | SEQ ID NO:49 | SEQ ID NO:84 | SEQ ID NO:85 |

### Example 7, Construction of anti-BDCA2 Chimeric Antibodies

A total of 12 monoclonal antibody sequences were picked for human-mouse chimeric antibody construction according to the sequence detection result and the sequence of hlgG1 Fc fragment was selected as human constant region sequence during plasmid construction. The coding sequences for heavy and light chain variable region of the anti-BDCA2 mouse antibody were synthesized by Genscript Corporation. Antibody expression was performed using expi 293F as host cells. The resulting chimeric antibodies were: mab001c, mab002c, mab005c, mab016c, mab019c, mab020c, mab021c, mab022c, mab024c, mab027c, mab030c, and mab033c, respectively, and expressed chimeric antibodies were characterized individually.

### Example 8, Screening of Chimeric Antibodies

### 8.1. Detection of binding activity of anti-BDCA2 chimeric antibodies to proteins by ELISA method

HumanBDCA2 Fc protein or cynoBDCA2 His protein was diluted with PBS to 1 µg/mL and added to ELISA microplates at 100 µL/well and incubated at 4 °C overnight. Blocking was performed with ELISA blocking solution (PBS phosphate buffer with 1% BSA, pH 7.4 the percentages are mass percentages) at 37°C for 2hrs. Sequential 10-fold serial dilutions of antibodies were added for 8 concentration points (0.0001 nM-100 nM, including 0 point control). The plates were incubated at 37°C for 1h at 100µL/well. The plates were washed 3 times and anti-Human IgG (Fab specific)-HRP (Sigma, A0293; 1: 5000 dilution) secondary antibodies was added at 100µL/well, incubated at 37°C for 1h. The plates were washed 3 times, TMB development fluid (EL0009, Huzhou Ying Chuang Biotechnology) was added at 100 µL/well and 50µL 1N hydrochloric acid was added after incubation at 37°C for 10 min to stop the development reaction. OD450nm values were read with ELISA plate reader and the curve was fitted with GraphPad Prism6, and EC₅₀ values were calculated.

### 8.2. Detection of binding activity of chimeric antibodies to cells by FACS method

CHOK1-humanBDCA2 and 293F-cynoBDCA2 cells cultured to logarithmic growth phase were trypsinized with TrypLE. The cells were recovered and resuspended to 2×10⁶/mL with FACS buffer (PBS + 2% FBS). The cells were added to 3799 cell plates (Corning) at 100µL/well, and the supernatant was discarded after centrifugation at 300 g. The antibodies were serially diluted with FACS buffer (Id.), the diluted antibodies were added to centrifuged cells and the cells were resuspended. The cells were incubated at 4 °C for 1h. The incubated cells were centrifuged at 300 g for 5 min and washed twice with FACS buffer. 100µL Donkey anti-Human IgG (H + L) Cross-Adsorbed Secondary Antibody-Alexa Fluor 488 (Sigma, A11013; 1:1000) secondary antibody was added, washed twice with FACS buffer after incubation at 4 °C for 1h, resuspended with PBS, analyzed with FACS (BD FACS CantoTM II) instrument and curve fitted with GraphPad Prism6, and EC₅₀ values were calculated.

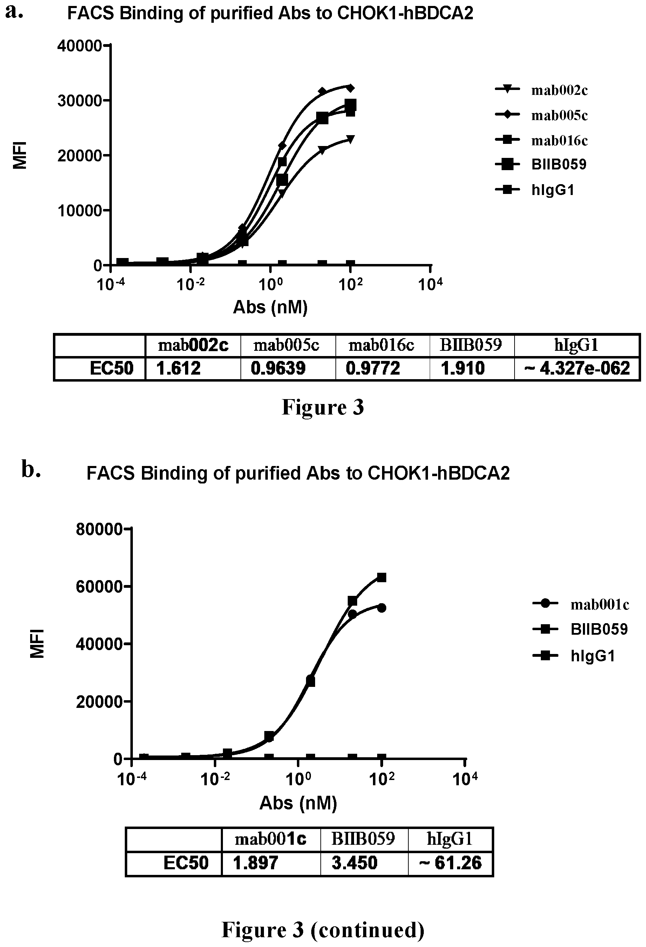

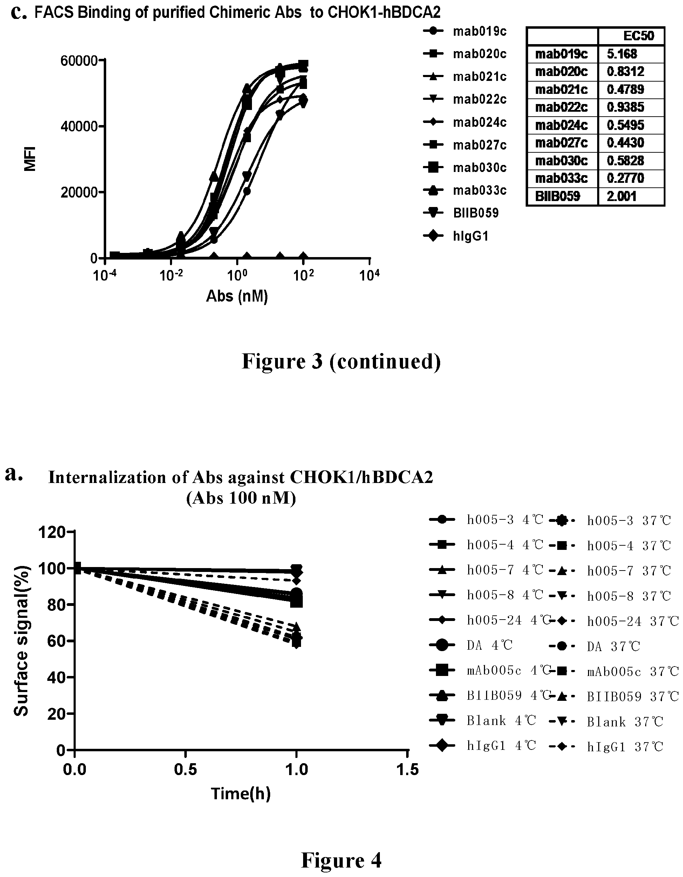

As shown in Figures 3 (3a-3c), all tested chimeric antibodies bound to CHOK1-humanBDCA2 cells with EC₅₀ superior or comparable to control antibody BIIB059. At same time, all tested chimeric antibodies bound to 293F-cynoBDCA2 cells with EC₅₀ of approximately 0.3-2 nM superior or comparable to control antibody BIIB059.

### 5.3. Detection of ability of chimeric antibodies to inhibit CpG-A to stimulate cytokine IFNα release from human PBMC cells

The cryopreserved human PBMC cells were recovered, incubated overnight with complete medium (1640 + 10% FBS + l×NEAA+l×L-Glutamine) for 18hrs. The PBMC cells were collected on following day, centrifuged at 600g for 8 minutes, and the supernatant was discarded. The cells were resuspended with media (1640 + 10% FBS) to 8×10⁶/mL, and added to 3799 cell culture plates at 100µL/well. The antibodies to be tested were serially diluted (final concentration, 0.0001 nM-10 nM) with medium (1640 + 10% FBS) in 10 series, and diluted antibodies were added to PBMC at 50µL/well. The cells were resuspended and incubated at 37°C for 6 hrs. 2µM CpG-A (Invitrogen, tlrl-2216-5) diluted with 50µL medium (1640 + 10% FBS) was added per well, mixed well and continued to incubate at 37°C for 20 hrs. The cell culture plates were centrifuged at 400 g for 5 minutes on the third day and the cell culture supernatants were collected and assayed for cytokine IFNα in the supernatants.

Antibody MT1/3/5 (Mabtech, 3425-1H-20) was diluted with PBS to 4µg/mL and added to CORNING ELISA plates at 100 µL per well, and the coated plates were incubated at 4°C overnight. Then, the antibodies coated the plates were discarded, and blocking solution (PBS + 1% BSA) was added to the plates at 300 µL per well, and incubated at 4°C overnight. The supernatants to be tested were diluted with blocking solution to the appropriate concentration (generally in range from 1: 3-1: 7), and were added to the coated ELISA plates at 100 µL per well. IFNα detection standards were diluted in 2-fold series with blocking solution (top concentration was 1000 pg/mL, 8 concentration points, the last point was 0), and were added to the coated ELISA plates at 100 µL per well. The ELISA plates were incubated in incubator at 37°C for 1 hour. The plates were washed 3 times, and the detection antibody MT2/4/6 (1: 1000) was added at 100 µL per well and incubated I incubator at 37°C for 1 hour. The plates were washed 3 times, the secondary antibody SA-HRP (1: 1000) was added at 100 µL per well, and incubated in incubator at 37°C for 0.5h. The plates were washed 3 times, and the TMB development solution was added at 100 µL per well and the reaction was stopped at the appropriate time with 1M HC1 added at 50 µL per well. OD450nm readings were read with ELISA plate reader, inhibition ratio was calculated and the curve was fitted with GraphPad Prism6, and EC₅₀ values were calculated.

All 12 chimeric antibodies tested were able to inhibit secretion of cytokine IFNα in experiments of CpG-A stimulated cytokine release from PBMCs, with IC₅₀ of mab005c, mab019c, mab020c, mab021c, mab022c, mab024c, mab027c, mab030c, and mab033c of 0.008359 nM, 0.01493 nM, 0.01853 nM, 0.01268 nM, 0.03239 nM, 0.01677 nM, 0.008976, nM, 0.01645 nM, and 0.008331 nM, respectively.

### 8.4. Detection of internalization capacity of chimeric antibodies

CHOK1-humanBDCA2 cells cultured to logarithmic growth phase were trypsinized with TrypLE. The cells were recovered and resuspended to 2× 106/mL with FACS buffer (PBS + 2% FBS). The cells were added to 3799 cell plates at 100µL/well, the supernatant was discarded after centrifugation at 300 g, and the cells were placed in ice box to cool. The antibody was serially diluted with FACS buffer (set at 3 concentrations: 100 nM, 10 nM, 1 nM; and 3 groups each: 0 h control; 4°C, 1 h; 37°C, 1 h), then placed in ice box to cool for 15 min, the antibodies were added to centrifuged cells after 15 min, the cells were resuspended and incubate at 4°C for 40 min. The incubated cells were centrifuged at 300 g for 5 min and washed twice with FACS buffer. The cells in 0h group were fixed with paraformaldehyde at room temperature for 10 min and washed twice with FACS buffer; two additional groups of cells were resuspended with 100µL FACS buffer and incubated at 4°C and 37°C for 1h, respectively. The cells in groups at 4°C for 1h and 37°C for 1h were fixed with paraformaldehyde at room temperature for 10 min, and washed twice with FACS buffer. The cells in 3 groups were simultaneously added with 100 µL of secondary antibody Goat anti-Human IgG (H + L) Cross-Adsorbed Secondary Antibody-Alexa Fluor 488 (Sigma, A11013; 1: 1000) and incubated at 4°C for 1h. The cells were washed twice with FAC buffer, and then resuspended with PBS, analyzed with a FACS instrument, and the percentage of surface signal was calculated.

Internalization of chimeric antibodies occurred at 100 nM, 10 nM, and 1 nM concentrations, with 12 chimeric antibodies mediating internalization capacities that were not as much different and slightly better than positive control antibody BIIB059. The cell surface signal values were 50%-60% upon internalization at 37°Cfor 1h at 100 nM.

### Example 9, Humanization of Variable Regions of Antibody

For humanization of variable regions of antibody, human germline IgG genes homologous to cDNA sequence of mouse antibodies were first searched at human immunoglobulin gene database at NCBI (http://www.ncbi.nlm.nih.gov/igblast/) website and then their exact boundaries were defined by the Kabat numbering system or the IMGT numbering system. In principle, human IGHV, which has high homology to mouse antibodies, is selected as the humanization template and humanization of variable regions of antibody is performed by CDR grafting.

mAb005 and mAb033 were selected for humanization from the sequences of the mouse antibodies obtained above. The steps of humanization process were as follows: A, the gene sequences of the mouse antibody were aligned with the gene sequences of the human germline antibody to find sequences with high homology; b, analysis to investigate HLA-DR affinity to select human germline framework sequences with low affinity; c. molecular docking was applied to analyze the framework amino acid sequences of the variable regions and their periphery utilizing in silico techniques to examine their spatial stereo bonding approach. Key amino acids in mouse antibody gene sequence that can act with human BDCA2 and maintain spatial framework were obtained by analysis with calculating electrostatic forces, van der Waals forces, hydrophilicity and entropy values, then these amino acids were grafted back to the human germline gene framework that has been selected, and amino acid sites of framework region that must be retained were identified on this basis, then humanized antibodies were synthesized. Based on this, various humanized antibody variable region sequences were obtained, and various combinations of designed variable regions of humanized anti-BDCA2 antibody were made to obtain humanized anti-BDCA2 antibodies whose variable region amino acid sequences are specified in Table 3.

CDRs of variable regions of the Hu005 series humanized antibody (KABAT scheme) are shown below:
HCDR1: NYGVH ( SEQ ID NO:11)
HCDR2: VIWSGESTDYDAAFIS ( SEQ ID NO:12)
HCDR3: RRSHYYGYVMDY ( SEQ ID NO:13 )
LCDR1: KASQSIDYDAIGYLN (SEQ ID NO:86)
LCDR2: AASNLES ( SEQ ID NO:15 )
LCDR3: QQSNEDPPT (SEQ ID NO:16)

CDRs of variable regions of the Hu003 series humanized antibody (KABAT scheme) are shown below:
HCDR1: SYGMS ( SEQ ID NO:52)
HCDR2: TISSGDSYTYYPDSVKG (SEQ ID NO:39)
HCDR3: QIYYDYAYYFDF ( SEQ ID NO:53 )
LCDR1: RASESVSFRTSHLMH (SEQ ID NO:54)
LCDR2: GASNLES (SEQ ID NO:55 )
LCDR3: QQSIEDPPT (SEQ ID NO:42)

**Table 3 Amino Acid Sequences of Variable Region of Anti-BDCA2 Humanized Antibodies**

| Antibodies | VH | VL | Antibodies | VH | VL | Antibodies | VH | VL |
|---|---|---|---|---|---|---|---|---|
| Hu033-01 | SEQ ID NO:87 | SEQ ID NO:135 | Hu033-17 | SEQ ID NO:103 | SEQ ID NO:151 | Hu005-09 | SEQ ID NO:119 | SEQ ID NO:167 |
| Hu033-02 | SEQ ID NO:88 | SEQ ID NO:136 | Hu033-18 | SEQ ID NO:104 | SEQ ID NO:152 | Hu005-10 | SEQ ID NO:120 | SEQ ID NO:168 |
| Hu033-03 | SEQ ID NO:89 | SEQ ID NO:137 | Hu033-19 | SEQ ID NO:105 | SEQ ID NO:153 | Hu005-11 | SEQ ID NO:121 | SEQ ID NO:169 |
| Hu033-04 | SEQ ID NO:90 | SEQ ID NO:138 | Hu033-20 | SEQ ID NO:106 | SEQ ID NO:154 | Hu005-12 | SEQ ID NO:122 | SEQ ID NO:170 |
| Hu033-05 | SEQ ID NO:91 | SEQ ID NO:139 | Hu033-21 | SEQ ID NO:107 | SEQ ID NO:155 | Hu005-13 | SEQ ID NO:123 | SEQ ID NO:171 |
| Hu033-06 | SEQ ID NO:92 | SEQ ID NO:140 | Hu033-22 | SEQ ID NO:108 | SEQ ID NO:156 | Hu005-14 | SEQ ID NO:124 | SEQ ID NO:172 |
| Hu033-07 | SEQ ID NO:93 | SEQ ID NO:141 | Hu033-23 | SEQ ID NO:109 | SEQ ID NO:157 | Hu005-15 | SEQ ID NO:125 | SEQ ID NO:173 |
| Hu033-08 | SEQ ID NO:94 | SEQ ID NO:142 | Hu033-24 | SEQ ID NO:110 | SEQ ID NO:158 | Hu005-16 | SEQ ID NO:126 | SEQ ID NO:174 |
| Hu033-09 | SEQ ID NO:95 | SEQ ID NO:143 | Hu005-01 | SEQ ID NO:111 | SEQ ID NO:159 | Hu005-17 | SEQ ID NO:127 | SEQ ID NO:175 |
| Hu033-10 | SEQ ID NO:96 | SEQ ID NO:144 | Hu005-02 | SEQ ID NO:112 | SEQ ID NO:160 | Hu005-18 | SEQ ID NO:128 | SEQ ID NO:176 |
| Hu033-11 | SEQ ID NO:97 | SEQ ID NO:145 | Hu005-03 | SEQ ID NO:113 | SEQ ID NO:161 | Hu005-19 | SEQ ID NO:129 | SEQ ID NO:177 |
| Hu033-12 | SEQ ID NO:98 | SEQ ID NO:146 | Hu005-04 | SEQ ID NO:114 | SEQ ID NO:162 | Hu005-20 | SEQ ID NO:130 | SEQ ID NO:178 |
| Hu033-13 | SEQ ID NO:99 | SEQ ID NO:147 | Hu005-05 | SEQ ID NO:115 | SEQ ID NO:163 | Hu005-21 | SEQ ID NO:131 | SEQ ID NO:179 |
| Hu033-14 | SEQ ID NO:100 | SEQ ID NO:148 | Hu005-06 | SEQ ID NO:116 | SEQ ID NO:164 | Hu005-22 | SEQ ID NO:132 | SEQ ID NO:180 |
| Hu033-15 | SEQ ID NO:101 | SEQ ID NO:149 | Hu005-07 | SEQ ID NO:117 | SEQ ID NO:165 | Hu005-23 | SEQ ID NO:133 | SEQ ID NO:181 |
| Hu033-16 | SEQ ID NO:102 | SEQ ID NO:150 | Hu005-08 | SEQ ID NO:118 | SEQ ID NO:166 | Hu005-24 | SEQ ID NO:134 | SEQ ID NO:182 |

### Example 10, Screening of Humanized Anti-BDCA2 Antibodies

### 10.1. Binding activity of humanized antibodies to BDCA2

CHOK1-humanBDCA2 and 293F-cynoBDCA2 cells cultured to logarithmic growth phase were trypsinized with TrypLE. The cells were recovered and resuspended to 2×10⁶/mL with FACS buffer (PBS + 2% FBS). The resuspended cells were added to 3799 cell plates at 100µL/well, and the supernatant was discarded after centrifugation at 300 g. The antibodies to be detected were diluted in 5-fold serials with FACS buffer (with final concentration at 0.012 nM-200 nM), the diluted antibodies were added to centrifuged cells and the cells were resuspended. The cells were incubated at 4°C for 1h. The incubated cells were centrifuged at 300g for 5min and washed twice with FACS buffer. 100µL Goat anti-Human IgG (H + L) Cross-Adsorbed Secondary Antibody, Alexa Fluor 488 (Sigma, A11013; 1:1000) secondary antibody was added, washed twice with FACS buffer after incubation at 4°C for 1h. The cells were resuspended with PBS, then the fluorescence signal of antibodies bound to cell surface was analyzed with FACS (BD FACS CantoTM II) instrument and curves were fitted with GraphPad Prism6 to calculate EC₅₀ values for antibodies binding to BDCA2 antigen. The BIIB059 antibody was selected as positive control and hlgG1 as negative control.

The candidate antibodies obtained after humanization of mAb033 sequence, whose activity of binding to CHOK1-humanBDCA2 and 293F-cynoBDCA2 cell was higher than positive control BIIB059 antibody, are shown in Table 4. Antibody affinity to human BDCA2 was determined using SPR and selected antibodies had stronger affinity to human BDCA2 than positive control BIIB059 antibody.

**Table 4 Binding activity of mAb033 series humanized antibodies to BDCA2**

| Number of Antibodies | CHOK1-hBDCA2 EC₅₀ (nM) | 293F-cynoBDCA2 EC₅₀ (nM) |
|---|---|---|
| BIIB059 | 1.30 | 1.63 |
| hIgG1 | - | - |
| mAb033c | 0.26 | 0.56 |
| Hu033-02(BM2) | 0.44 | 1.20 |
| Hu033-03(BM3) | 0.50 | 1.40 |
| Hu033-04(BM0) | 0.61 | 1.33 |
| Hu033-06(BM3) | 0.62 | 1.41 |
| Hu033-08(BM2) | 0.62 | 1.53 |
| Hu033-10(BM4) | 0.51 | 1.27 |
| Hu033-12(BM2) | 0.45 | 1.07 |
| Hu033-13(BM4) | 0.68 | 1.13 |
| Hu033-15(BM2) | 0.76 | 1.27 |
| Hu033-16(BM4) | 0.63 | 1.37 |
| Hu033-20(BM3) | 0.64 | 1.22 |
| Hu033-23(BM3) | 0.79 | 1.24 |

| | | |
|---|---|---|
| Note: "-" indicates not detected. | | |

The candidate antibodies obtained from humanization of mAb005 sequence bound CHOK1-humanBDCA2 cells with a better EC₅₀ value than positive control BIIB059 antibody, as shown in Table 5. At same time, the candidate antibodies obtained from humanization of mAb005 sequence also had better binding activity for 293F-cynoBDCA2 cells.

**Table 5 Binding activity of mAb005 series humanized antibodies to BDCA2**

| Number of Antibodies | CHOK1-hBDCA2 EC₅₀ (nM) |
|---|---|
| mAb005c | 2.2 |
| BIIB059 | 3.1 |
| hu005-03(BM4) | 2.1 |
| hu005-04(BM6) | 2.3 |
| hu005-07(BM5) | 2.0 |
| hu005-08(BM7) | 2.3 |
| hu005-24(BM6) | 1.9 |

### 10.2. Ability of humanized antibodies to inhibit CpG-A to stimulate cytokine IFNα release from human PBMC cells

A series of humanized candidate antibody molecules were selected for functional assays that inhibit CpG-A stimulation of cytokine IFNα release from human PBMC cells. The cryopreserved human PBMC cells were recovered, incubated overnight with complete medium (1640 + 10% FBS + l×NEAA+l×L-Glutamine) for 18hrs. The PBMC cells were collected on following day, centrifuged at 600g for 8 minutes, and the supernatant was discarded. The cells were resuspended with media (1640 + 10% FBS) to 8×10⁶/mL, and added to 3799 cell culture plates at 100µL/well. The antibodies to be tested were diluted in 10-fold series (0.0001 nM-10 nM and 0 point control) with medium (1640 + 10% FBS), and diluted antibodies were added to PBMC at 50µL/well. The cells were resuspended and incubated at 37°C for 6 hrs. 2µM CpG-A (Invitrogen, tlrl-2216-5) diluted with 50µL medium (1640 + 10% FBS) was added per well and mixed well and continued to incubate at 37°C for 20 hrs. The cell culture plates were centrifuged at 400g for 5 minutes on the third day and the cell culture supernatants were collected and assayed for cytokine IFNα in the supernatants. BIIB059 antibody was selected as positive control and hIgG1 as negative control.

Antibody MT1/3/5 (Mabtech, 3425-1H-20) was diluted with PBS to 4µg/mL and added to CORNING ELISA plates at 100µL per well, and the coated plates were incubated at 4°C overnight. Then, the antibodies coated the plates were discarded, and blocking solution (PBS + 1% BSA) was added to the plates at 300µL per well, and incubated at 4°C overnight. The supernatants to be tested were diluted with blocking solution to the appropriate concentration (generally in range from 1:3-1:7), and were added to the coated ELISA plates at 100µL per well. IFNα detection standards were diluted in 2-fold series with blocking solution (top concentration was 1000 pg/mL, 8 concentration points, the last point was 0), and were added to the coated ELISA plates at 100µL per well. The ELISA plates were incubated in incubator at 37°C for 1h. The plates were washed 3 times, and the detection antibody MT2/4/6 (1: 1000) was added at 100µL per well and incubated I incubator at 37°C for 1h. The plates were washed 3 times, and the secondary antibody SA-HRP (1: 1000) was added at 100µL per well followed by incubation in incubator at 37°C for 0.5h. The plates were washed 3 times, and the TMB development solution was added at 100µL per well and the reaction was stopped at the appropriate time with 1M HC1 added at 50µL per well. OD450nm readings were read with ELISA plate reader and the curve was fitted with GraphPad Prism6. The concentration of 10nM antibody was selected to correspond to OD value and maximum inhibition was calculated as inhibition rate = (OD (IgG1)-OD (Ab))/OD (IgG1).

All of humanized antibodies tested were able to inhibit cytokine IFNα secretion in CpG-A stimulated cytokine release from PBMC experiments, with EC₅₀ values less than positive control antibody BIIB059, and the results are further shown in Table 6.

**Table 6 Humanized antibodies inhibited that CpG-A stimulated cytokine IFNα release from human PBMC**

| Number of Antibodies | EC₅₀ (nM) |
|---|---|
| BIIB059 | 0.04 |
| hIgG1 | - |
| mAb033c | 0.02 |
| Hu033-02(BM2) | 0.02 |
| Hu033-03(BM3) | 0.01 |
| Hu033-04(BM0) | 0.03 |
| Hu033-06(BM3) | 0.02 |
| Hu033-08(BM2) | 0.02 |
| Hu033-10(BM4) | 0.01 |
| Hu033-12(BM2) | 0.02 |
| Hu033-13(BM4) | 0.01 |
| Hu033-15(BM2) | 0.02 |
| Hu033-16(BM4) | 0.01 |
| Hu033-20(BM3) | 0.01 |
| Hu033-23(BM3) | 0.03 |

| | |
|---|---|
| Note: "-" indicates not detected. | |

### 10.3. Detection of internalization capacity of humanized antibodies

CHOK1-humanBDCA2 cells cultured to logarithmic growth phase were trypsinized with TrypLE. The cells were recovered and resuspended to 2× 10⁶/mL with FACS buffer (PBS + 2% FBS). The cells were added to 3799 cell plates at 100µL/well, the supernatant was discarded after centrifugation at 300 g, and the cells were placed in ice box to cool. The antibody was serially diluted with FACS buffer (set at 2 concentrations: 100 nM, 10 nM; and 3 groups each: 0 h; 4°C, 1 h; 37°C, 1 h), then placed in ice box to cool for 15 min, the antibodies were added to centrifuged cells after 15 min, the cells were resuspended and incubate at 4°C for 40 min. The incubated cells were centrifuged at 300 g for 5 min and washed twice with FACS buffer. The cells in 0h group were fixed with paraformaldehyde at room temperature for 10 min and washed twice with FACS buffer; two additional groups of cells were resuspended with 100µL FACS buffer and incubated at 4°C and 37°C for 1h, respectively. The cells in groups at 4°C for 1h and 37°C for 1h were fixed with paraformaldehyde at room temperature for 10 min, and washed twice with FACS buffer. The cells in 3 groups were simultaneously added with 100 µL of secondary antibody Goat anti-Human IgG (H + L) Cross-Adsorbed Secondary Antibody-Alexa Fluor 488 (Sigma, A11013; 1: 1000) and incubated at 4°C for 1h. The cells were washed twice with FAC buffer, and then resuspended with PBS, analyzed with a FACS instrument, and the percentage of surface signal was calculated.

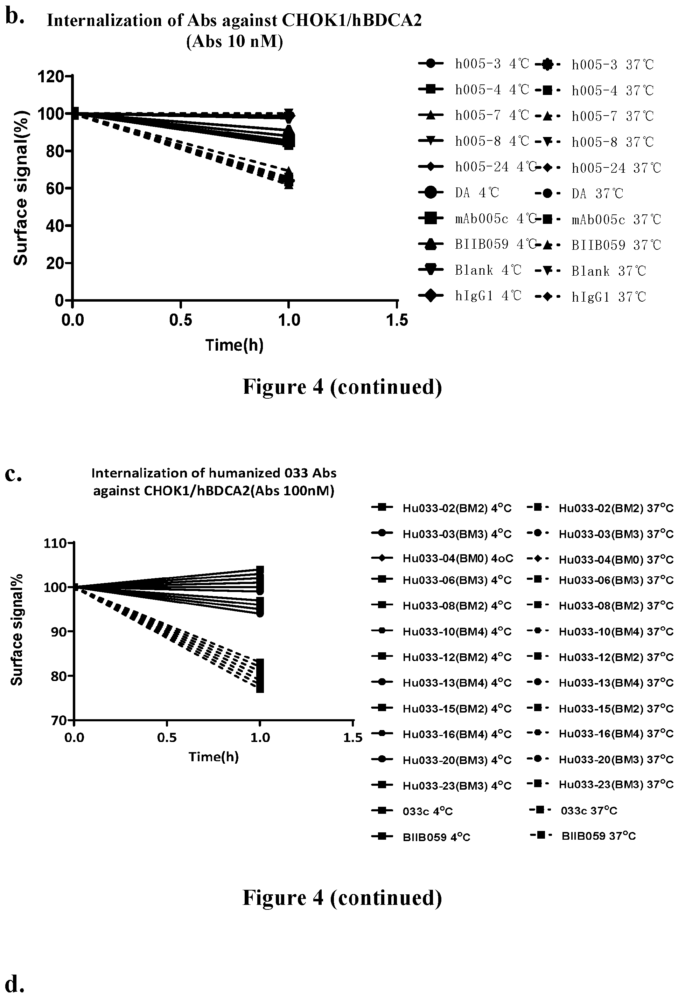

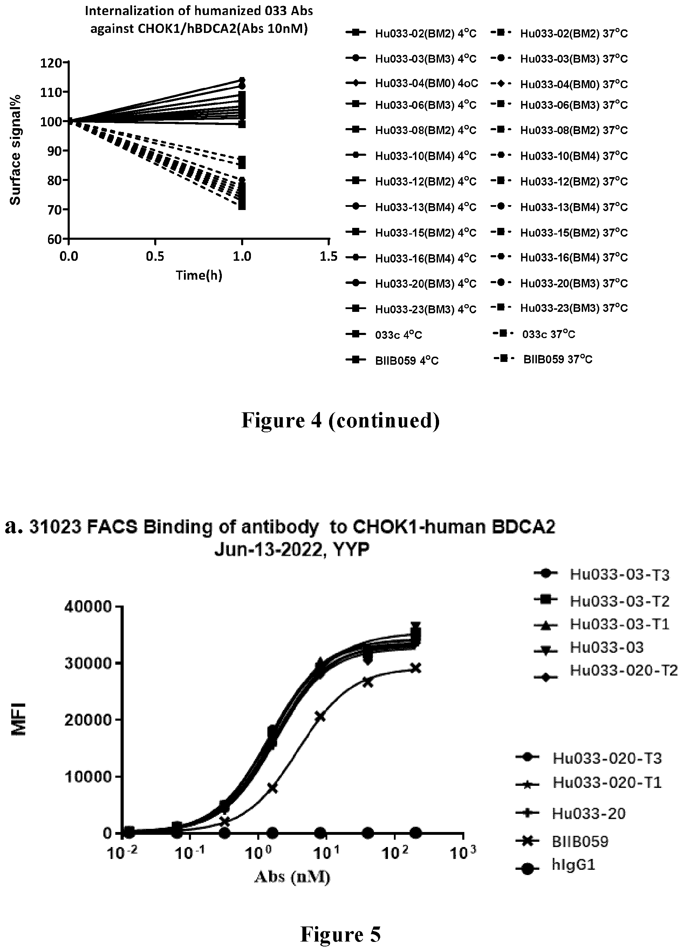

The results are shown in Figure 4, and both Hu005 and Hu033 groups of humanized antibodies developed internalization at 100 nM and 10 nM concentrations, with ability of humanized antibodies to mediate internalization comparable to positive control antibody BIIB059.

### 10.4. Detection of affinity of humanized antibodies

The affinity of the humanized antibodies of the invention were determined by BIAcore experiments: A CMS chip channel surface was activated with a 1:1 mix of 50mM NHS and 200mM EDC (NHS and EDC from amino coupling kit), the anti-human IgG (Fc) antibody (diluted in sodium acetate solution at pH 5.0, the concentration was 25 µ g/mL) was injected for 420 seconds. Then 1 M ethanolamine was injected at 10 L/min flow rate for 420 seconds, and the excess active carboxyl groups on chip were blocked. The humanized antibodies were injected into a detection channel flow cell at flow rate of 10 L/min for 60 s for capture after diluting to 1 ug/mL using running buffer. Recombinant Human BDCA2 was diluted to 50 or 100 nM with 1×HBS-EP + (pH 7.4), and diluted at 1:2 series to 0.39 nM. The sample hBDCA2 to be tested of each concentration gradient was injected into the detection channel at 30 µL/min flow rate, two samples at 0 concentration were used to remove background signal. The binding and dissociation times between antigen and antibody were 180 and 400 sec, respectively. An analysis of data was performed using Biacore Insight Evaluation Software (Version 2.0. 15.12933). A 1: 1 binding model was selected for curve fitting and kinetic parameters were calculated after subtraction of signal of the reference channel (flow cell 1) and at 0 concentration. The results of the assays are shown in Table 7 below.

**Table 7 Affinity of humanized antibodies**

| Number of Antibodies | SPR | | |
|---|---|---|---|
| Ab NO. | ka (1/Ms) | kd (1/s) | KD (M) |
| BIIB059 | 1.67E+05 | 1.79E-04 | 1.07E-09 |
| hIgG1 | | | |
| mAb033c | 6.98E+05 | 7.67E-05 | 1.10E-10 |
| Hu033-02(BM2) | 7.07E+05 | 1.69E-04 | 2.39E-10 |
| Hu033-03(BM3) | 5.76E+05 | 1.36E-04 | 2.36E-10 |
| Hu033-04(BM0) | 5.98E+05 | 1.93E-04 | 3.23E-10 |
| Hu033-06(BM3) | 7.33E+05 | 1.25E-04 | 1.70E-10 |
| Hu033-08(BM2) | 7.40E+05 | 1.69E-04 | 2.29E-10 |
| Hu033-10(BM4) | 5.89E+05 | 1.18E-04 | 2.00E-10 |
| Hu033-12(BM2) | 5.27E+05 | 1.49E-04 | 2.83E-10 |
| Hu033-13(BM4) | 4.54E+05 | 1.53E-04 | 3.38E-10 |
| Hu033-15(BM2) | 4.87E+05 | 2.27E-04 | 4.67E-10 |
| Hu033-16(BM4) | 5.69E+05 | 1.08E-04 | 1.91E-10 |
| Hu033-20(BM3) | 5.56E+05 | 1.49E-04 | 2.68E-10 |
| Hu033-23(BM3) | 4.66E+05 | 2.07E-04 | 4.44E-10 |

### Example 11 Fc mutation of humanized anti-BDCA2 antibodies and screening of antibodies

For the humanized antibodies Hu033-03 and Hu033-20 obtained in Example 10, Fc sites were selected for mutation to increase their affinity for FcγRIIA. A total of humanized antibodies Hu033-03-T1, Hu033-03-T2, Hu033-03-T3, Hu033-20-T1, Hu033-20-T2, and Hu033-20-T3 were designed, and the resulting heavy and light chain coding sequences were synthesized by Genscript. The amino acid sequences of heavy and light chains are shown in Table 8. 293F was selected as the host cell for antibody expression, and various characterization tests were performed on the purified antibodies after expression.

**Table 8: Amino acid sequences of variable region and full-length of mutant anti-BDCA2 humanized antibodies**

| Antibodies | VH | VL | HC | LC |
|---|---|---|---|---|
| Hu033-03-T1 | SEQ ID NO:89 | SEQ ID NO:137 | SEQ ID NO:191 | SEQ ID NO:186 |
| Hu033-03-T2 | SEQ ID NO:89 | SEQ ID NO:137 | SEQ ID NO:192 | SEQ ID NO:186 |
| Hu033-03-T3 | SEQ ID NO:89 | SEQ ID NO:137 | SEQ ID NO:193 | SEQ ID NO:186 |
| Hu033-20-T1 | SEQ ID NO:106 | SEQ ID NO:154 | SEQ ID NO:194 | SEQ ID NO:187 |
| Hu033-20-T2 | SEQ ID NO:106 | SEQ ID NO:154 | SEQ ID NO:195 | SEQ ID NO:187 |
| Hu033-20-T3 | SEQ ID NO:106 | SEQ ID NO:154 | SEQ ID NO:196 | SEQ ID NO:187 |

### 11.1. Fc mutation increases antibody affinity for FcγRIIA

Antibody kinetics or affinity parameters were measured using BIAcore 8K and freshly fixed biotinylated human Fc γ RIIA(R167)/Fc γ RIIA (H167) SA chips. The experiment was run at 25 °C with a running buffer of 1×HBS-EP+(10 mM HEPES pH 7.4, 150 mM NaCl, 3 mM EDTA, 0.05% Surfactant P20). The antibody was diluted with running buffer at a ratio of 1:2 to concentrations of 5000nM, 2500nM, 1250nM, 625nM, 321.5nM, 156.25nM, 78.13nM, 39.06nM, 19.53nM, or 9.77nM, and the tested antibody at each concentration gradient was injected into flow cell 1 and flow cell 2 at a flow rate of 30 µL/min. The binding and dissociation times of the antibody were 60 and 90 seconds, respectively. Two zero concentration samples are used to remove background signals. The sample was injected in a multi cycle manner, the analyte can be fully dissociated from the chip surface at the end of each cycle, and no regeneration step was applied. Data analysis was perform using Biacore Insight Evaluation Software (Version 2.0.15.12933). After deducting the reference channel (flow cell 1) and two zero concentration signals, affinity analysis was performed using a 1:1 binding model or steady-state model for curve fitting and calculation of kinetic parameters.

The affinity of the mutated antibody for both human Fcγ RIIA (R167) and Fcγ RIIA (H167) subtypes is significantly increased compared with the wild-type Fc. The results are shown in Tables 9-1 and 9-2.

**Table 9-1 Affinity of anti-BDCA2 humanized antibody for human FcγRIIA (H167) (1:1 Binding)**

| Antibodies | **ka (1/Ms)** | **kd (1/s)** | **KD (M)** | **Multiple of affinity** |
|---|---|---|---|---|
| Hu033-03-T1 | 4.70E+05 | 2.30E-01 | 4.90E-07 | 2.3 |
| Hu033-03-T2 | 5.70E+05 | 5.85E-02 | 1.03E-07 | 10.0 |
| Hu033-03-T3 | 5.51E+05 | 8.94E-02 | 1.62E-07 | 7.3 |
| Hu033-03 | 3.61E+05 | 3.39E-01 | 9.41E-07 | - |
| Hu033-20-T1 | 4.94E+05 | 2.42E-01 | 4.89E-07 | 2.5 |
| Hu033-20-T2 | 6.04E+05 | 5.77E-02 | 9.55E-08 | 12.1 |
| Hu033-20-T3 | 5.63E+05 | 8.70E-02 | 1.54E-07 | 8.7 |
| Hu033-20 | 4.39E+05 | 4.31E-01 | 9.81E-07 | - |

**Table 9-2 Affinity of anti-BDCA2 humanized antibody for human FcγRIIA (R167) (1:1 Binding)**

| Antibodies | **ka (1/Ms)** | **kd (1/s)** | **KD (M)** | **Multiple of affinity** |
|---|---|---|---|---|
| Hu033-03-T1 | 7.50E+05 | 2.32E-01 | 3.10E-07 | 4.0 |
| Hu033-03-T2 | 1.96E+06 | 8.99E-02 | 4.58E-08 | 13.7 |
| Hu033-03-T3 | 7.41E+05 | 2.25E-01 | 3.03E-07 | 4.4 |
| Hu033-03 | 1.53E+06 | 7.82E-01 | 5.10E-07 | = |
| Hu033-20-T1 | 7.98E+05 | 2.49E-01 | 3.12E-07 | 5.1 |
| Hu033-20-T2 | 2.00E+06 | 8.21E-02 | 4.11E-08 | 17.7 |
| Hu033-20-T3 | 6.83E+05 | 1.91E-01 | 2.79E-07 | 6.3 |
| Hu033-20 | 1.45E+06 | 8.38E-01 | 5.78E-07 | = |

### 11.2. The antibody with Fc mutation maintains unchanged binding affinity to BDCA2

The binding ability of Fc mutated antibodies to human BDCA2 and Cyno BDCA2 expressing cells was detected by FACA.

The experimental method is as follows.

CHOK1-humanBDCA2 and 293F-cynoBDCA2 cells were trypsinized with TrypLE. The cells were recovered and resuspended to 2× 10⁶/mL with FACS buffer (PBS + 2% FBS). The cells were added to 3799 cell plates (Corning) at 100µL/well, and the supernatant was discarded after centrifugation at 300 g. The antibody was diluted with FACS buffer at a starting concentration of 200nM in a 1:5 gradient, the diluted antibodies were added to centrifuged cells and the cells were resuspended. The cells were incubated at 4°C for 1h. The incubated cells were centrifuged at 300 g for 5 min and washed twice with FACS buffer. 100µL Donkey anti-Mouse IgG (H + L) Highly Cross-Adsorbed Secondary Antibody-Alexa Fluor 488 (Invitrogen, A21202; 1: 1000) secondary antibody was added, washed twice with FACS buffer after incubation at 4°C for 1h, resuspended with PBS, analyzed with FACS (BD FACS CantoTM II) instrument and curve fitted with GraphPad Prism6, and EC₅₀ values were calculated.

The binding ability of Fc mutated antibody to human BDCA2 is slightly better than that of wild-type Fc, and significantly better than the reference antibody BIIB059. The binding ability of Fc mutated antibodies to Cyno BDCA2 is superior to that of wild-type Fc and BIIB059. The results are shown in Figure 5a, Figure 5b, and Table 10.

**Table 10 Binding ability of humanized anti-BDCA2 antibodies to human BDCA2**

| Antibodies | EC₅₀ humanBDCA2 | EC₅₀ Cyno BDCA2 |
|---|---|---|
| Hu033-03-T1 | 1.625 | 3.321 |
| Hu033-03-T2 | 1.542 | 3.064 |
| Hu033-03-T3 | 1.422 | 2.728 |
| Hu033-03 | 1.823 | 4.002 |
| Hu033-20-T1 | 1.695 | 2.808 |
| Hu033-20-T2 | 1.718 | 2.861 |
| Hu033-20-T3 | 1.708 | 2.711 |
| Hu033-20 | 1.858 | 3.201 |
| BIIB059 | 3.811 | 4.382 |
| HIgG1 | ~146.6 | ~364250 |

### 11.3. The internalization capacity of antibodies remains unchanged after Fc mutation

The internalization capacity of each antibody was detected using FACS.

CHOK1-humanBDCA2 cells were trypsinized with TrypLE. The cells were recovered and resuspended to 2×10⁶/mL with FACS buffer (PBS + 2% FBS). The cells were added to 3799 cell plates at 100µL/well, the supernatant was discarded after centrifugation at 300 g, and the cells were placed in ice box to cool. The antibody was serially diluted with FACS buffer to 1nM, 10nM or 100nM (set 5 sets of detection conditions for each antibody concentration: 0 h; 4°C, 1 h; 37°C, 1 h; 4°C, 4h; 37°C, 4h), then placed in ice box to cool for 15 min, the antibodies were added to centrifuged cells after 15 min, the cells were resuspended and incubate at 4°C for 40 min. The incubated cells were centrifuged at 300 g for 5 min and washed twice with FACS buffer. The cells in 0h group were fixed with paraformaldehyde at room temperature for 10 min and washed twice with FACS buffer; four additional groups of cells were resuspended with 100µL FACS buffer and incubated at 4°C and 37°C for 1h or 4h, respectively. The cells in groups at 4°C and 37°C for said period were fixed with paraformaldehyde at room temperature for 10 min, and washed twice with FACS buffer. The cells in 5 groups were simultaneously added with 100 µL of secondary antibody Goat anti-Human IgG (H + L) Cross-Adsorbed Secondary Antibody-Alexa Fluor 488 (Sigma, A11013; 1: 1000) and incubated at 4°C for 1h. The cells were washed twice with FAC buffer, and then resuspended with PBS, analyzed with a FACS instrument, and the percentage of surface signal was calculated.

The Fc mutated antibody was found to be internalized at concentrations of 100nM, 10nM, and 1nM. The modification of three types of Fc has no effect on antibody internalization. See Figures 6a-6f for details.

### 11.4. Fc mutated antibodies have stronger inhibitory effects on antibody complex SLE-IC-stimulated release of cytokine IFNα from human PBMC cells

Function of Fc modified antibodies to inhibit antibody complexes-stimulated release of cytokine IFNα from human PBMC cells was detected. The cryopreserved human PBMC cells were recovered, incubated overnight with complete medium (1640 + 10% FBS + l×NEAA+l×L-Glutamine) for 18hrs. The PBMC cells were collected on following day, centrifuged at 600g for 8 minutes, and the supernatant was discarded. The cells were resuspended with media (1640 + 10% FBS) to 8×10⁶/mL, and added to 3799 cell culture plates at 100µL/well. The antibodies to be tested were diluted in a 5-fold gradient (0.0032 nM-10 nM, and 0 control) with medium (1640 + 10% FBS), and the diluted antibodies were added to PBMC at 50µL/well. The cells were resuspended and incubated at 37°C for 6 hrs. SLE-IC(2.5µl Sm/RNP-antigen (AROTEC, ART01-10) mixed with 10µl anti-RNP antibody (RayBiotech, MD-14-0513)) diluted with 50µL medium (1640 + 10% FBS) was added per well and mixed well and continued to incubate at 37°C for 20 hrs. The cell culture plates were centrifuged at 400 g for 5 minutes on the third day and the cell culture supernatants were collected and assayed for cytokine IFNα in the supernatants. BIIB059 antibody was selected as the positive control and hIgG1 as the negative control.

The cytokine IFNα in the supernatant was detected, antibody MT1/3/5 (Mabtech, 3425-1H-20) was diluted with PBS to 4µg/mL and added to CORNING ELISA plates at 100µL per well, and incubated at 4°C overnight. Then, the antibodies coated the plates were discarded, and blocking solution (PBS + 1% BSA) was added to the plates at 300 µL per well, and incubated at 4°C overnight. The supernatants to be tested were diluted with blocking solution to the appropriate concentration (selected from 1: 3-1: 7 as the case may be), and were added to the coated ELISA plates at 100 µL per well. IFNα detection standards were diluted in 2-fold series with blocking solution (top concentration was 1000 pg/mL, 8 concentration points, the last point was 0), and were added to the coated ELISA plates at 100 µL per well. The ELISA plates were incubated in incubator at 37°C for 1 hour. The plates were washed 3 times, and the detection antibody MT2/4/6 (1: 1000) was added at 100 µL per well and incubated I incubator at 37°C for 1 hour. The plates were washed 3 times, and the secondary antibody SA-HRP (1: 1000) was added at 100 µL per well followed by incubation in incubator at 37°C for 0.5h. The plates were washed 3 times, and the TMB development solution was added at 100 µL per well and 1M HC1 was added at the appropriate time at 50 µL per well. OD₄₅₀ₙₘ readings were read with ELISA plate reader, the curve was fitted with GraphPad Prism6 to calculate EC₅₀.

The Fc modified antibody has a stronger IFNα inhibitory effect under SLE-IC stimulation conditions compared with the wild-type Fc antibody and BIIB059 antibody, achieving complete IFNα secretion inhibition at 0.016nM. See Figure 7 and Table 11 for details.

**Table 11: Mutated humanized antibodies inhibit the release of cytokine IFNα from human PBMC cells stimulated by SLE-IC**

| Concentra tion of antibodies (nM) | Hu 033 -03-T3 | Hu 033 -03-T2 | **Hu** 033 -03-T1 | **Hu** 033 -03 | Hu 033 -20-T2 | Hu 033 -20-T3 | Hu03 3-03-T1 | Hu033 -20 | BIIB059 | hIgG1 |
|---|---|---|---|---|---|---|---|---|---|---|
| 10 | - | - | - | - | - | - | - | - | - | 182±38 |
| 2 | - | - | - | - | - | - | - | - | - | 150±31.6 |
| 0.4 | - | - | - | - | - | - | - | - | - | 162.4±8.5 |
| 0.08 | - | - | - | 33± 19. 6 | - | - | - | 11.2±1 5.8 | - | N.A. |
| 0.016 | - | - | - | 27± 20. 5 | - | - | - | 27.8±3 9.3 | 17.7±25 | N.A. |
| 0.0032 | 92. 6±2 1 | 40. 6±1 | 71. 5±1 0.1 | 119 .8± 26. 5 | 33. 8±0 .9 | 59. 8±3 4.3 | 86.8± 1 | 127±2 0 | 113.1±39. 7 | N.A. |

| | | | | | | | | | | |
|---|---|---|---|---|---|---|---|---|---|---|
| Note: "-" indicates below the detection limit; N.A. "indicates undetected. | | | | | | | | | | |

The various modifications and variations of the method and system described in the invention will be apparent to those skilled in the art without departing from the scope and spirit of the invention. Although The invention has been described in relation to specific preferred embodiments, it should be understood that the claimed invention should not be improperly limited to such specific embodiments. In fact, various modifications of the modes described for implementing the invention are obvious to those skilled in molecular biology, immunology, or related fields and are intended to fall within the scope of the appended claims.

Sequence list (all are amino acid sequences, see the previous section of the mention for the definition/explanation of sequences):

| SEQ ID NO | Sequence |
|---|---|
| SEQ ID NO:1 | DFSMH |
| SEQ ID NO:2 | WINTETGEPTYADDFKG |
| SEQ ID NO:3 | GGDGHPYWYFAV |
| SEQ ID NO:4 | RASQSVSTSSYSYMH |
| SEQ ID NO:5 | YASILES |
| SEQ ID NO:6 | QHSWEIPFT |
| SEQ ID NO:7 | DYSMH |
| SEQ ID NO:8 | GGDGFPYWYFNV |
| SEQ ID NO:9 | RASQSVSTSSNSYIH |
| SEQ ID NO:10 | YASNLDS |
| SEQ ID NO:11 | NYGVH |
| SEQ ID NO:12 | VIWSGESTDYDAAFIS |
| SEQ ID NO:13 | RRSHYYGYVMDY |
| SEQ ID NO:14 | KASQSIDYDGIGYLN |
| SEQ ID NO:15 | AASNLES |
| SEQ ID NO:16 | QQSNEDPPT |
| SEQ ID NO:17 | SYGVH |
| SEQ ID NO:18 | KASQSVDYDGVGYLN |
| SEQ ID NO:19 | DYYMY |
| SEQ ID NO:20 | YISYSGDSTYYPDTVKG |
| SEQ ID NO:21 | HSPSFPYYFDY |
| SEQ ID NO:22 | SASSSVSYMH |
| SEQ ID NO:23 | EISKLAS |
| SEQ ID NO:24 | QLWNYPLIT |
| SEQ ID NO:25 | SGYYWN |
| SEQ ID NO:26 | YISFDGTYNYNPSLKD |
| SEQ ID NO:27 | GVDYPNAIDW |
| SEQ ID NO:28 | RASESVSMHVTHLIH |
| SEQ ID NO:29 | VASNLES |
| SEQ ID NO:30 | QQSFEDPWT |
| SEQ ID NO:31 | SAYYWN |
| SEQ ID NO:32 | YITYDGNNYYSPSLKN |
| SEQ ID NO:33 | GGDYDDGGFAF |
| SEQ ID NO:34 | RASGNIHNYLA |
| SEQ ID NO:35 | NAKSLVD |
| SEQ ID NO:36 | QHFWIVPYT |
| SEQ ID NO:37 | GVDYPKAMDW |
| SEQ ID NO:38 | SFGMS |
| SEQ ID NO:39 | TISSGDSYTYYPDSV |
| SEQ ID NO:40 | QIYYDYAYYFDY |
| SEQ ID NO:41 | RASESVSFRSSHLMH |
| SEQ ID NO:42 | QQSIEDPPT |
| SEQ ID NO:43 | YISYDGSNNYNPSLKN |
| SEQ ID NO:44 | RASESVSIHGTHLMH |
| SEQ ID NO:45 | VASYLES |
| SEQ ID NO:46 | YITYDGSNNYNPSLKN |
| SEQ ID NO:47 | GGDYDDGGFAY |
| SEQ ID NO:48 | NAKTLVD |
| SEQ ID NO:49 | QHFWIIPYT |
| SEQ ID NO:50 | YISYDGSNYYNPSLKN |
| SEQ ID NO:51 | NAKTLAD |
| SEQ ID NO:52 | SYGMS |
| SEQ ID NO:53 | QIYYDYAYYFDF |
| SEQ ID NO:54 | RASESVSFRTSHLMH |
| SEQ ID NO:55 | GASNLES |
| SEQ ID NO:56 | YITYDGSNNYNPSLKD |
| SEQ ID NO:57 | QIQLVQSGPELKKPGETVKISCKASGYTFTDFSMHWVKQAPGKGLKW MGWINTETGEPTYADDFKGRFAFSLETSASTAFLQINNLKNDDTTTYFC SRGGDGHPYWYFAVWGAGTTVTVSS |
| SEQ ID NO:58 | DIVLTQSPASLAVSLGQRATISCRASQSVSTSSYSYMHWYQQKPGQPPK LLIKYASILESGVPARFSGSGSGTDFTLNIHPVEEEDTATYYCQHSWEIPF TFGSGTKLEIK |
| SEQ ID NO:59 | QIQLVQSGPELKKPGETVKISCRASGYSFTDYSMHWVKQAPGKGLKW VGWINTETGEPTYADDFKGRFALSLETSATSAYLQINNLKNEDTATYFCA RGGDGFPYWYFNVWGAGTTVTVSS |
| SEQ ID NO:60 | DIVLTQSPTSLAVSLGQRTNISCRASQSVSTSSNSYIHWYQQKPGQPPKL LIKYASNLDSGVPARFSGSGSGTDFTLNIHPVEEEDTSTYYCQHSWEIPF TFGSGTKLEIK |
| SEQ ID NO:61 | QVQLKQSGPGLVQPSQSLSITCTVSGFSLTNYGVHWVRQSPGKGLEWL GVIWSGESTDYDAAFISRLSISKDNSKSQVFFKMNSLQADDSAIYYCAR RRSHYYGYVMDYWGQGTSVTVSS |
| SEQ ID NO:62 | DIVLTQSPASLAVSLGQRATISCKASQSIDYDGIGYLNWYQHKPGQPPKL LIYAASNLESGIPARFSGSGSGTDFTLNIHPVEEEDAATYYCQQSNEDPPT FGSGTKLEIK |
| SEQ ID NO:63 | QVQLKQSGPGLVQPSQSLSITCTVSGFSLTSYGVHWVRQSPGKGLEWL GVIWSGESTDYDAAFISRLSISKDNSKSQVFFRMNSLQADDSAIYYCAR RRSHYYGYVMDYWGQGTSVTVSS |
| SEQ ID NO:64 | DIVLTQSPASLAVSLGQRATISCKASQSVDYDGVGYLNWYQHKPGQPP KLLIYAASNLESGIPARFSGSGSGTDFTLNIHPVEEEDAATYYCQQSNED PPTFGSGTKLEIK |
| SEQ ID NO:65 | EVKLVESGGDLVQPGGSLKLSCAASGFTFSDYYMYWVRQTPEKRLEW VAYISYSGDSTYYPDTVKGRFTISRDNAKNTLFLQMSRLKSEDTAMYYC ARHSPSFPYYFDYWGQGTALTVSS |
| SEQ ID NO:66 | EIVLTQSPAITAASLGQKVTITCSASSSVSYMHWYQQKSGTSPKPWIYEI SKLASGVPARFSGSGSGTSYSLTISSMEAEDAAIYYCQLWNYPLITFGGG TNLEIK |
| SEQ ID NO:67 | DVQLQESGPGLVKPSQSLSLTCSVTGYSIASGYYWNWIRQFPGNKLEW MGYISFDGTYNYNPSLKDRFSITRDTSKNQFFLKLNSVTTEDTATYYCA RGVDYPNAIDWWGQGTSVTVSS |
| SEQ ID NO:68 | DIVLTQSPASLAVSLGQRATISCRASESVSMHVTHLIHWYQQKPGQPPK LLIYVASNLESGVPARFSGSGSETDFTLNIHPVEEEDAATYFCQQSFEDP WTFGGGTKLEIK |
| SEQ ID NO:69 | DVQLQESGPGLVKPSQSLSLTCSVTGYSITSAYYWNWIRQFPGNKLECM GYITYDGNNYYSPSLKNRISITRDTSKNQFFLNLNSVTTEDTATYFCARG GDYDDGGFAFWGQGTLVTVSA |
| SEQ ID NO:70 | DIQMTQSPASLSASVGETVTITCRASGNIHNYLAWYHQKQGKSPQLLVY NAKSLVDGVPSRFSGSGSGTQYSLRINSLQPEDFGSYYCQHFWIVPYTF GGGTKLEII |
| SEQ ID NO:71 | DVQLQESGPGLVKPSQSLSLTCSVTGYSITSGYYWNWIRQFPGNKLEW MGYISFDGTYNYNPSLKDRFSITRDTSKNQFFLKLNSVTTEDTATYYCA RGVDYPKAMDWWGQGTSVTVSS |
| SEQ ID NO:72 | EVQLVESGGDLVKPGGSLKLSCAASGFTFSSFGMSWVRQTPDKRLDW VATISSGDSYTYYPDSVKGRFSISRDNAKNTLYLQMSSLKSEDTAVYYC ARQIYYDYAYYFDYWGQGTTLTVSS |
| SEQ ID NO:73 | DIVLTQSPASLAVSLGQRATISCRASESVSFRSSHLMHWYRQKPGQPPKL LIYAASNLESGVPARFSGSGSETDFTLNIHPVEEEDAAAYFCQQSIEDPPT FGGGTKLEIK |
| SEQ ID NO:74 | DVQLQESGPGLVKPSQSLSLTCSVTGYSITSAYYWNWIRQFPGHKLEW MGYISYDGSNNYNPSLKNRISITRDTSKNQFFLKLNSVTTEDTATYHCA RGVDYPNAIDWWGQGTSLTVSS |
| SEQ ID NO:75 | DIVLTQSPTSLTVSLGQRATISCRASESVSIHGTHLMHWYQQKPGQPPKL LIYVASYLESGVPARFSGSGSETDFSLNIHPVEEEDAATYFCQQSFEDPW TFGGGTKLEIK |
| SEQ ID NO:76 | DVQLQESGPGLVKPSQSLSLTCSVTGYSITSAYYWNWVRQFPGNQLEC MGYITYDGSNNYNPSLKNRISITRDTSKNQFFLKLNSVTTEDTATYYCV RGGDYDDGGFAYWGQGTLVTVSA |
| SEQ ID NO:77 | DIQMTQSPASLSASVGETVTITCRASGNIHNYLAWYQQKQGKSPQLLVY NAKTLVDGVPSRFSGSGSGTEYSLKINSLQPEDFGIYFCQHFWIIPYTFG GGTKLEIR |
| SEQ ID NO:78 | DVQLQESGPGLVKPSQSLSLTCSVTGYSISSGYYWNWIRQFPGNKLECM GYISYDGSNYYNPSLKNRISITRDTSKNQFFLSLNSVTTEDTATYFCVRG GDYDDGGFAYWGQGTLVTVSA |
| SEQ ID NO:79 | DIQMTQSPASLSASVGETVTITCRASGNIHNYLAWYQQKQGKSPQLLVY NAKTLADGVPSRFSGSGSETQYSLKINSLQPEDFGNYFCQHFWIIPYTFG GGTKLEIK |
| SEQ ID NO:80 | DVQLQESGPGLVKPSQSLSLTCSVTGYSITSAYYWNWIRQFPGNKLECM GYISYDGSNYYNPSLKNRISITRDTSKNQFFLSLNSVTTEDTATYFCVRG GDYDDGGFAYWGQGTLVTVSA |
| SEQ ID NO:81 | DVQLQESGPGLVKPSQSLSLTCSVTGYSITSGYYWNWIRQFPGNKLEC MGYISYDGSNYYNPSLKNRISITRDTSKNQFFLSLNSVTTEDTATYFCVR GGDYDDGGFAYWGQGTLVTVSA |
| SEQ ID NO:82 | EVQMVESGGDLVKPGDSLKLSCAASGFTFRSYGMSWVRQTPDKRLEW VATISSGDSYTYYPDSVKGRFTISRDNAKNTLYLQMSSLKSEDTALYYCA RQIYYDYAYYFDFWGQGTTLTVSS |
| SEQ ID NO:83 | DIVLTQSPASLAVSLGQRATISCRASESVSFRTSHLMHWYQQKPGQPPKL LIYGASNLESGVPARFSGSGSETDFTLNIHPVEEEDAAAYFCQQSIEDPPT FGGGTKLEIK |
| SEQ ID NO:84 | DVQLQESGPGLVKPSQSLSLTCSVTGYSITSGYYWNWIRQFPGNKLEC MGYITYDGSNNYNPSLKDRISITRDTSKNQFFLKLNSVTTEDTATYYCA RGGDYDDGGFAYWGHGTLVTVSA |
| SEQ ID NO:85 | DIQMTQSPVSLSASVGETVTITCRASGNIHNYLAWYQQKQGKSPQLLV YNAKSLVDGVPSRFSGSGSGTQYSLKINSLQPEDFGSYYCQHFWIIPYTF GGGTKLEIK |
| SEQ ID NO:86 | KASQSIDYDAIGYLN |
| SEQ ID NO:87 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:88 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:89 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:90 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:91 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:92 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:93 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:94 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSS |
| SEQ ID NO:95 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:96 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:97 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:98 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:99 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:100 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:101 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:102 | EVQLVESGGGLVKPGGSLRLSCAASGFTFRSYGMSWVRQAPGKRLEW VSTISSGDSYTYYPDSVKGRFTISRDNAKNSLYLQMNSLRAEDTAVYYC ARQIYYDYAYYFDFWGQGTTVTVSS |
| SEQ ID NO:103 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:104 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:105 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:106 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:107 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:108 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:109 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:110 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSS |
| SEQ ID NO:111 | QVQLQESGPGLVKPSETLSLTCTVSGGSISNYGVHWIRQPPGKGLEWIG VIWSGESTDYDAAFISRVTISVDTSKNQFSLKLSSVTAADTAVYYCARRR SHYYGYVMDYWGQGTTVTVSS |
| SEQ ID NO:112 | QVQLQESGPGLVKPSETLSLTCTVSGGSISNYGVHWIRQPPGKGLEWIG VIWSGESTDYDAAFISRVTISKDNSKNQFSLKLSSVTAADTAVYYCARR RSHYYGYVMDYWGQGTTVTVSS |
| SEQ ID NO:113 | QVQLQESGPGLVKPSETLSLTCTVSGGSITNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:114 | QVQLQQSGPGLVKPSETLSLTCTVSGGSLTNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:115 | QVQLQESGPGLVKPSETLSLTCTVSGGSISNYGVHWIRQPPGKGLEWIG VIWSGESTDYDAAFISRVTISVDTSKNQFSLKLSSVTAADTAVYYCARRR SHYYGYVMDYWGQGTTVTVSS |
| SEQ ID NO:116 | QVQLQESGPGLVKPSETLSLTCTVSGGSISNYGVHWIRQPPGKGLEWIG VIWSGESTDYDAAFISRVTISKDNSKNQFSLKLSSVTAADTAVYYCARR RSHYYGYVMDYWGQGTTVTVSS |
| SEQ ID NO:117 | QVQLQESGPGLVKPSETLSLTCTVSGGSITNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:118 | QVQLQQSGPGLVKPSETLSLTCTVSGGSLTNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:119 | QVQLQESGPGLVKPSETLSLTCTVSGGSISNYGVHWIRQPPGKGLEWIG VIWSGESTDYDAAFISRVTISVDTSKNQFSLKLSSVTAADTAVYYCARRR SHYYGYVMDYWGQGTTVTVSS |
| SEQ ID NO:120 | QVQLQESGPGLVKPSETLSLTCTVSGGSISNYGVHWIRQPPGKGLEWIG VIWSGESTDYDAAFISRVTISKDNSKNQFSLKLSSVTAADTAVYYCARR RSHYYGYVMDYWGQGTTVTVSS |
| SEQ ID NO:121 | QVQLQESGPGLVKPSETLSLTCTVSGGSITNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:122 | QVQLQQSGPGLVKPSETLSLTCTVSGGSLTNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:123 | QVQLQESGPGLVKPSETLSLTCTVSGGSISNYGVHWIRQPPGKGLEWIG VIWSGESTDYDAAFISRVTISVDTSKNQFSLKLSSVTAADTAVYYCARRR SHYYGYVMDYWGQGTTVTVSS |
| SEQ ID NO:124 | QVQLQESGPGLVKPSETLSLTCTVSGGSISNYGVHWIRQPPGKGLEWIG VIWSGESTDYDAAFISRVTISKDNSKNQFSLKLSSVTAADTAVYYCARR RSHYYGYVMDYWGQGTTVTVSS |
| SEQ ID NO:125 | QVQLQESGPGLVKPSETLSLTCTVSGGSITNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:126 | QVQLQQSGPGLVKPSETLSLTCTVSGGSLTNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:127 | QVQLQESGPGLVKPSETLSLTCTVSGGSISNYGVHWIRQPPGKGLEWIG VIWSGESTDYDAAFISRVTISVDTSKNQFSLKLSSVTAADTAVYYCARRR SHYYGYVMDYWGQGTTVTVSS |
| SEQ ID NO:128 | QVQLQESGPGLVKPSETLSLTCTVSGGSISNYGVHWIRQPPGKGLEWIG VIWSGESTDYDAAFISRVTISKDNSKNQFSLKLSSVTAADTAVYYCARR RSHYYGYVMDYWGQGTTVTVSS |
| SEQ ID NO:129 | QVQLQESGPGLVKPSETLSLTCTVSGGSITNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:130 | QVQLQQSGPGLVKPSETLSLTCTVSGGSLTNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:131 | QVQLQESGPGLVKPSETLSLTCTVSGGSISNYGVHWIRQPPGKGLEWIG VIWSGESTDYDAAFISRVTISVDTSKNQFSLKLSSVTAADTAVYYCARRR SHYYGYVMDYWGQGTTVTVSS |
| SEQ ID NO:132 | QVQLQESGPGLVKPSETLSLTCTVSGGSISNYGVHWIRQPPGKGLEWIG VIWSGESTDYDAAFISRVTISKDNSKNQFSLKLSSVTAADTAVYYCARR RSHYYGYVMDYWGQGTTVTVSS |
| SEQ ID NO:133 | QVQLQESGPGLVKPSETLSLTCTVSGGSITNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:134 | QVQLQQSGPGLVKPSETLSLTCTVSGGSLTNYGVHWIRQPPGKGLEWIG VIWSGESTDYDAAFISRVTISKDNSKNQVSLKLSSVTAADTAVYYCARR RSHYYGYVMDYWGQGTTVTVSS |
| SEQ ID NO:135 | DIVLTQSPASLAVSPGQRATITCRASESVSFRTSHLMHWYQQKPGQPPKL LIYGASNLESGVPARFSGSGSGTDFTLTINPVEANDTANYYCQQSIEDPP TFGGGTKVEIK |
| SEQ ID NO:136 | DIVLTQSPASLAVSPGQRATITCRASESVSFRTSHLMHWYQQKPGQPPKL LIYGASNLESGVPARFSGSGSETDFTLTINPVEAEDTANYYCQQSIEDPPT FGGGTKVEIK |
| SEQ ID NO:137 | DIVLTQSPASLAVSPGQRATITCRASESVSFRTSHLMHWYQQKPGQPPKL LIYGASNLESGVPARFSGSGSETDFTLTINPVEAEDTANYFCQQSIEDPPT FGGGTKVEIK |
| SEQ ID NO:138 | DIVMTQSPDSLAVSLGERATINCRASESVSFRTSHLMHWYQQKPGQPPK LLIYGASNLESGVPDRFSGSGSGTDFTLTISSLQAEDVAVYYCQQSIEDPP TFGGGTKVEIK |
| SEQ ID NO:139 | DIVLTQSPDSLAVSLGERATINCRASESVSFRTSHLMHWYQQKPGQPPK LLIYGASNLESGVPDRFSGSGSGTDFTLTISSLQAEDVAVYFCQQSIEDPP TFGGGTKVEIK |
| SEQ ID NO:140 | DIVLTQSPDSLAVSLGERATINCRASESVSFRTSHLMHWYQQKPGQPPK LLIYGASNLESGVPDRFSGSGSETDFTLTISPLQAEDVAVYFCQQSIEDPP TFGGGTKVEIK |
| SEQ ID NO:141 | EIVLTQSPATLSLSPGERATLSCRASESVSFRTSHLMHWYQQKPGQAPRL LIYGASNLESGIPARFSGSGSGTDFTLTISRLEPEDFAVYYCQQSIEDPPTF GGGTKVEIK |
| SEQ ID NO:142 | EIVLTQSPATLSLSPGERATLSCRASESVSFRTSHLMHWYQQKPGQAPRL LIYGASNLESGIPARFSGSGSETDFTLTISRLEPEDFAVYFCQQSIEDPPTF GGGTKVEIK |
| SEQ ID NO:143 | DIVLTQSPASLAVSPGQRATITCRASESVSFRTSHLMHWYQQKPGQPPKL LIYGASNLESGVPARFSGSGSGTDFTLTINPVEANDTANYYCQQSIEDPP TFGGGTKVEIK |
| SEQ ID NO:144 | DIVLTQSPASLAVSPGQRATITCRASESVSFRTSHLMHWYQQKPGQPPKL LIYGASNLESGVPARFSGSGSETDFTLTINPVEAEDTANYYCQQSIEDPPT FGGGTKVEIK |
| SEQ ID NO:145 | DIVLTQSPASLAVSPGQRATITCRASESVSFRTSHLMHWYQQKPGQPPKL LIYGASNLESGVPARFSGSGSETDFTLTINPVEAEDTANYFCQQSIEDPPT FGGGTKVEIK |
| SEQ ID NO:146 | DIVMTQSPDSLAVSLGERATINCRASESVSFRTSHLMHWYQQKPGQPPK LLIYGASNLESGVPDRFSGSGSGTDFTLTISSLQAEDVAVYYCQQSIEDPP TFGGGTKVEIK |
| SEQ ID NO:147 | DIVLTQSPDSLAVSLGERATINCRASESVSFRTSHLMHWYQQKPGQPPK LLIYGASNLESGVPDRFSGSGSGTDFTLTISSLQAEDVAVYFCQQSIEDPP TFGGGTKVEIK |
| SEQ ID NO:148 | DIVLTQSPDSLAVSLGERATINCRASESVSFRTSHLMHWYQQKPGQPPK LLIYGASNLESGVPDRFSGSGSETDFTLTISPLQAEDVAVYFCQQSIEDPP TFGGGTKVEIK |
| SEQ ID NO:149 | EIVLTQSPATLSLSPGERATLSCRASESVSFRTSHLMHWYQQKPGQAPRL LIYGASNLESGIPARFSGSGSGTDFTLTISRLEPEDFAVYYCQQSIEDPPTF GGGTKVEIK |
| SEQ ID NO:150 | EIVLTQSPATLSLSPGERATLSCRASESVSFRTSHLMHWYQQKPGQAPRL LIYGASNLESGIPARFSGSGSETDFTLTISRLEPEDFAVYFCQQSIEDPPTF GGGTKVEIK |
| SEQ ID NO:151 | DIVLTQSPASLAVSPGQRATITCRASESVSFRTSHLMHWYQQKPGQPPKL LIYGASNLESGVPARFSGSGSGTDFTLTINPVEANDTANYYCQQSIEDPP TFGGGTKVEIK |
| SEQ ID NO:152 | DIVLTQSPASLAVSPGQRATITCRASESVSFRTSHLMHWYQQKPGQPPKL LIYGASNLESGVPARFSGSGSETDFTLTINPVEAEDTANYYCQQSIEDPPT FGGGTKVEIK |
| SEQ ID NO:153 | DIVLTQSPASLAVSPGQRATITCRASESVSFRTSHLMHWYQQKPGQPPKL LIYGASNLESGVPARFSGSGSETDFTLTINPVEAEDTANYFCQQSIEDPPT FGGGTKVEIK |
| SEQ ID NO:154 | DIVMTQSPDSLAVSLGERATINCRASESVSFRTSHLMHWYQQKPGQPPK LLIYGASNLESGVPDRFSGSGSGTDFTLTISSLQAEDVAVYYCQQSIEDPP TFGGGTKVEIK |
| SEQ ID NO:155 | DIVLTQSPDSLAVSLGERATINCRASESVSFRTSHLMHWYQQKPGQPPK LLIYGASNLESGVPDRFSGSGSGTDFTLTISSLQAEDVAVYFCQQSIEDPP TFGGGTKVEIK |
| SEQ ID NO:156 | DIVLTQSPDSLAVSLGERATINCRASESVSFRTSHLMHWYQQKPGQPPK LLIYGASNLESGVPDRFSGSGSETDFTLTISPLQAEDVAVYFCQQSIEDPP TFGGGTKVEIK |
| SEQ ID NO:157 | EIVLTQSPATLSLSPGERATLSCRASESVSFRTSHLMHWYQQKPGQAPRL LIYGASNLESGIPARFSGSGSGTDFTLTISRLEPEDFAVYYCQQSIEDPPTF GGGTKVEIK |
| SEQ ID NO:158 | EIVLTQSPATLSLSPGERATLSCRASESVSFRTSHLMHWYQQKPGQAPRL LIYGASNLESGIPARFSGSGSETDFTLTISRLEPEDFAVYFCQQSIEDPPTF GGGTKVEIK |
| SEQ ID NO:159 | DIVLTQSPASLAVSPGQRATITCKASQSIDYdaIGYLNWYQQKPGQPPKL LIYAASNLESGVPARFSGSGSGTDFTLTINPVEANDTANYYCQQSNEDPP TFGQGTKLEIK |
| SEQ ID NO:160 | DIVLTQSPASLAVSPGQRATITCKASQSIDYdaIGYLNWYQQKPGQPPKL LIYAASNLESGVPARFSGSGSGTDFTLTINPVEANDTANYYCQQSNEDPP TFGQGTKLEIK |
| SEQ ID NO:161 | DIVLTQSPASLAVSPGQRATITCKASQSIDYdaIGYLNWYQQKPGQPPKL LIYAASNLESGVPARFSGSGSGTDFTLTINPVEANDTANYYCQQSNEDPP TFGQGTKLEIK |
| SEQ ID NO:162 | DIVLTQSPASLAVSPGQRATITCKASQSIDYdaIGYLNWYQQKPGQPPKL LIYAASNLESGVPARFSGSGSGTDFTLTINPVEANDTANYYCQQSNEDPP TFGQGTKLEIK |
| SEQ ID NO:163 | DIVLTQSPASLAVSPGQRATITCKASQSIDYdaIGYLNWYQHKPGQPPKL LIYAASNLESGVPARFSGSGSGTDFTLTINPVEANDTANYYCQQSNEDPP TFGQGTKLEIK |
| SEQ ID NO:164 | DIVLTQSPASLAVSPGQRATITCKASQSIDYdaIGYLNWYQHKPGQPPKL LIYAASNLESGVPARFSGSGSGTDFTLTINPVEANDTANYYCQQSNEDPP TFGQGTKLEIK |
| SEQ ID NO:165 | DIVLTQSPASLAVSPGQRATITCKASQSIDYdaIGYLNWYQHKPGQPPKL LIYAASNLESGVPARFSGSGSGTDFTLTINPVEANDTANYYCQQSNEDPP TFGQGTKLEIK |
| SEQ ID NO:166 | DIVLTQSPASLAVSPGQRATITCKASQSIDYdaIGYLNWYQHKPGQPPKL LIYAASNLESGVPARFSGSGSGTDFTLTINPVEANDTANYYCQQSNEDPP TFGQGTKLEIK |
| SEQ ID NO:167 | EIVLTQSPATLSLSPGERATLSCKASQSIDYdaIGYLNWYQQKPGQAPRLL IYAASNLESGIPARFSGSGSGTDFTLTISSLEPEDFAVYYCQQSNEDPPTFG QGTKLEIK |
| SEQ ID NO:168 | EIVLTQSPATLSLSPGERATLSCKASQSIDYdaIGYLNWYQQKPGQAPRLL IYAASNLESGIPARFSGSGSGTDFTLTISSLEPEDFAVYYCQQSNEDPPTFG QGTKLEIK |
| SEQ ID NO:169 | EIVLTQSPATLSLSPGERATLSCKASQSIDYdaIGYLNWYQQKPGQAPRLL IYAASNLESGIPARFSGSGSGTDFTLTISSLEPEDFAVYYCQQSNEDPPTFG QGTKLEIK |
| SEQ ID NO:170 | EIVLTQSPATLSLSPGERATLSCKASQSIDYdaIGYLNWYQQKPGQAPRLL IYAASNLESGIPARFSGSGSGTDFTLTISSLEPEDFAVYYCQQSNEDPPTFG QGTKLEIK |
| SEQ ID NO:171 | EIVLTQSPATLSLSPGERATLSCKASQSIDYdaIGYLNWYQHKPGQAPRLL IYAASNLESGIPARFSGSGSGTDFTLTISSLEPEDFAVYYCQQSNEDPPTFG QGTKLEIK |
| SEQ ID NO:172 | EIVLTQSPATLSLSPGERATLSCKASQSIDYdaIGYLNWYQHKPGQAPRLL IYAASNLESGIPARFSGSGSGTDFTLTISSLEPEDFAVYYCQQSNEDPPTFG QGTKLEIK |
| SEQ ID NO:173 | EIVLTQSPATLSLSPGERATLSCKASQSIDYdaIGYLNWYQHKPGQAPRLL IYAASNLESGIPARFSGSGSGTDFTLTISSLEPEDFAVYYCQQSNEDPPTFG QGTKLEIK |
| SEQ ID NO:174 | EIVLTQSPATLSLSPGERATLSCKASQSIDYdaIGYLNWYQHKPGQAPRLL IYAASNLESGIPARFSGSGSGTDFTLTISSLEPEDFAVYYCQQSNEDPPTFG QGTKLEIK |
| SEQ ID NO:175 | DIVMTQSPDSLAVSLGERATINCKASQSIDYdaIGYLNWYQQKPGQPPKL LIYAASNLESGVPDRFSGSGSGTDFTLTISSLQAEDVAVYYCQQSNEDPP TFGQGTKLEIK |
| SEQ ID NO:176 | DIVMTQSPDSLAVSLGERATINCKASQSIDYdaIGYLNWYQQKPGQPPKL LIYAASNLESGVPDRFSGSGSGTDFTLTISSLQAEDVAVYYCQQSNEDPP TFGQGTKLEIK |
| SEQ ID NO:177 | DIVMTQSPDSLAVSLGERATINCKASQSIDYdaIGYLNWYQQKPGQPPKL LIYAASNLESGVPDRFSGSGSGTDFTLTISSLQAEDVAVYYCQQSNEDPP TFGQGTKLEIK |
| SEQ ID NO:178 | DIVMTQSPDSLAVSLGERATINCKASQSIDYdaIGYLNWYQQKPGQPPKL LIYAASNLESGVPDRFSGSGSGTDFTLTISSLQAEDVAVYYCQQSNEDPP TFGQGTKLEIK |
| SEQ ID NO:179 | DIQMTQSPSSLSASVGDRVTITCKASQSIDYdaIGYLNWYQQKPGKAPK LLIYAASNLESGVPSRFSGSGSGTDFTLTISSLQPEDFATYYCQQSNEDPP TFGQGTKLEIK |
| SEQ ID NO:180 | DIQMTQSPSSLSASVGDRVTITCKASQSIDYdaIGYLNWYQQKPGKAPK LLIYAASNLESGVPSRFSGSGSGTDFTLTISSLQPEDFATYYCQQSNEDPP TFGQGTKLEIK |
| SEQ ID NO:181 | DIQMTQSPSSLSASVGDRVTITCKASQSIDYdaIGYLNWYQQKPGKAPK LLIYAASNLESGVPSRFSGSGSGTDFTLTISSLQPEDFATYYCQQSNEDPP TFGQGTKLEIK |
| SEQ ID NO:182 | DIQMTQSPSSLSASVGDRVTITCKASQSIDYdaIGYLNWYQQKPGKAPK LLIYAASNLESGVPSRFSGSGSGTDFTLTISSLQPEDFATYYCQQSNEDPP TFGQGTKLEIK |
| SEQ ID NO:183 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHN HYTQKSLSLSPGK |
| SEQ ID NO:184 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC |
| | ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHN HYTQKSLSLSPGK |
| SEQ ID NO:185 | QVQLQQSGPGLVKPSETLSLTCTVSGGSLTNYGVHWIRQPPGKGLEWIG VIWSGESTDYDAAFISRVTISKDNSKNQVSLKLSSVTAADTAVYYCARR RSHYYGYVMDYWGQGTTVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGK |
| SEQ ID NO:186 | DIVLTQSPASLAVSPGQRATITCRASESVSFRTSHLMHWYQQKPGQPPKL LIYGASNLESGVPARFSGSGSETDFTLTINPVEAEDTANYFCQQSIEDPPT FGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACE VTHQGLSSPVTKSFNRGEC |
| SEQ ID NO:187 | DIVMTQSPDSLAVSLGERATINCRASESVSFRTSHLMHWYQQKPGQPPK LLIYGASNLESGVPDRFSGSGSGTDFTLTISSLQAEDVAVYYCQQSIEDPP TFGGGTKVEIKRTVAAPSVFIFPPSDEQLKSGTASVVCLLNNFYPREAKV QWKVDNALQSGNSQESVTEQDSKDSTYSLSSTLTLSKADYEKHKVYAC EVTHQGLSSPVTKSFNRGEC |
| SEQ ID NO:188 | DIVLTQSPASLAVSPGQRATITCKASQSIDYdaIGYLNWYQQKPGQPPKL LIYAASNLESGVPARFSGSGSGTDFTLTINPVEANDTANYYCQQSNEDPP TFGQGTKLEIKRTVAAPSVFIFPPSDEQLKSGTASVVCLLNNFYPREAKV QWKVDNALQSGNSQESVTEQDSKDSTYSLSSTLTLSKADYEKHKVYAC EVTHQGLSSPVTKSFNRGEC |
| SEQ ID NO:189 | MDMRVPAQLLGLLLLWFPGSRCPKSCDKTHTCPPCPAPELLGGPSVFLF PPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKA KGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGKIEGRNFMYSKTVKRLSKLREYQQYHPSLTCVMEGKDIE DWSCCPTPWTSFQSSCYFISTGMQSWTKSQKNCSVMGADLVVINTREE QDFIIQNLKRNSSYFLGLSDPGGRRHWQWVDQTPYNENVTFWHSGEPN NLDERCAIINFRSSEEWGWNDIHCHVPQKSICKMKKIYI |
| SEQ ID NO:190 | MDMRVPAQLLGLLLLWFPGSRCHHHHHHNFMYSKTVKRLSKLQEYQ QYYPSLTCVMEGKDMEDWSCCPTPWTSFQSSCYFISTVMQSWTKSQN NCSVMGADLVVINTKEEQDFITQNLKINSAYFLGLSDPKGWRHWQWV DQTPYNKNVTFWHSGEPNSPDERCAIINFRSEEWGWNDVHCHVPQKSI CKMKKIYI |
| SEQ ID NO:191 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPDV FLFPPKPKDTLMISRTPEVTCVVVDVSHEDPEVKFNWYVDGVEVHNAK TKPREEQYNSTYRVVSVLTVLHQDWLNGKEYKCKVSNKALPAPEEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK |
| SEQ ID NO:192 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLAGPDV FLFPPKPKDTLMISRTPEVTCVVVDVSHEDPEVKFNWYVDGVEVHNAK TKPREEQYNSTYRVVSVLTVLHQDWLNGKEYKCKVSNKALPAPEEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK |
| SEQ ID NO:193 | EVQLVESGGGLVKPGGSLRLSCAASGFTFSSYGMSWVRQAPGKGLEW VSTISSGDSYTYYPDSVKGRFTISRDNAKNSLYLQMNSLRAEDTAVYYC ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLAGPSV FLFPPKPKDTLMISRTPEVTCVVVDVSHEDPEVKFNWYVDGVEVHNAK TKPREEQYNSTYRVVSVLTVLHQDWLNGKEYKCKVSNKALPAPEEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK |
| SEQ ID NO:194 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPDV FLFPPKPKDTLMISRTPEVTCVVVDVSHEDPEVKFNWYVDGVEVHNAK TKPREEQYNSTYRVVSVLTVLHQDWLNGKEYKCKVSNKALPAPEEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK |
| SEQ ID NO:195 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLAGPDV FLFPPKPKDTLMISRTPEVTCVVVDVSHEDPEVKFNWYVDGVEVHNAK TKPREEQYNSTYRVVSVLTVLHQDWLNGKEYKCKVSNKALPAPEEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK |
| SEQ ID NO:196 | EVQLVESGGGLVKPGGSLRLSCAASGFTFRSYGMSWVRQAPGKRLEW VSTISSGDSYTYYPDSVKGRFTISRDNAKNSLYLQMNSLKAEDTAVYYC ARQIYYDYAYYFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLAGPSV FLFPPKPKDTLMISRTPEVTCVVVDVSHEDPEVKFNWYVDGVEVHNAK TKPREEQYNSTYRVVSVLTVLHQDWLNGKEYKCKVSNKALPAPEEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALH NHYTQKSLSLSPGK |

## Claims

1. An anti-BDCA2 antibody or antigen-binding fragment thereof, wherein the anti-BDCA2 antibody or antigen-binding fragment thereof comprises a heavy chain variable region and/or a light chain variable region:
The heavy chain variable region comprises:
(I) HCDR1, HCDR2, and HCDR3 having amino acid sequences as set forth in SEQ ID NO: 11, SEQ ID NO: 12, and SEQ ID NO: 13, respectively; or HCDR1, HCDR2 and HCDR3 having 1, 2 or 3 amino acid differences from the amino acid sequences as set forth in SEQ ID NO: 11, SEQ ID NO: 12 and SEQ ID NO: 13, respectively; or
(II) HCDR1, HCDR2 and HCDR3 having amino acid sequences as set forth in SEQ ID NO: 19, SEQ ID NO: 20 and SEQ ID NO: 21, respectively; or HCDR1, HCDR2 and HCDR3 having 1, 2 or 3 amino acid differences from the amino acid sequences as set forth in SEQ ID NO: 19, SEQ ID NO: 20 and SEQ ID NO: 21, respectively; or
(III) HCDR1, HCDR2 and HCDR3 having amino acid sequences as set forth in SEQ ID NO: 25, SEQ ID NO: 26 and SEQ ID NO: 27, respectively; or HCDR1, HCDR2, and HCDR3 having 1, 2, or 3 amino acid differences from the amino acid sequences as set forth in SEQ ID NO: 25, SEQ ID NO: 26, and SEQ ID NO: 27, respectively; or
(IV) HCDR1, HCDR2 and HCDR3 having amino acid sequences as set forth in SEQ ID NO: 31, SEQ ID NO: 32 and SEQ ID NO: 33, respectively; or HCDR1, HCDR2, and HCDR3 having 1, 2, or 3 amino acid differences from the amino acid sequences as set forth in SEQ ID NO: 31, SEQ ID NO: 32, and SEQ ID NO: 33, respectively; or
(V) HCDR1, HCDR2 and HCDR3 having amino acid sequences as set forth in SEQ ID NO: 38, SEQ ID NO: 39 and SEQ ID NO: 40, respectively; or HCDR1, HCDR2, and HCDR3 having 1, 2, or 3 amino acid differences from the amino acid sequences as set forth in SEQ ID NO: 38, SEQ ID NO: 39, and SEQ ID NO: 40, respectively; or
(VI) HCDR1, HCDR2 and HCDR3 having amino acid sequences as set forth in SEQ ID NO: 31, SEQ ID NO: 46 and SEQ ID NO: 47, respectively; or HCDR1, HCDR2, and HCDR3 having 1, 2, or 3 amino acid differences from the amino acid sequences as set forth in SEQ ID NO: 31, SEQ ID NO: 46, and SEQ ID NO: 47, respectively; or
(VII) HCDR1, HCDR2 and HCDR3 having amino acid sequences as set forth in SEQ ID NO: 31, SEQ ID NO: 50 and SEQ ID NO: 47, respectively; or HCDR1, HCDR2, and HCDR3 having 1, 2, or 3 amino acid differences from the amino acid sequences as set forth in SEQ ID NO: 31, SEQ ID NO: 50, and SEQ ID NO: 47, respectively; or
(VIII) HCDR1, HCDR2 and HCDR3 having amino acid sequences as set forth in SEQ ID NO: 52, SEQ ID NO: 39 and SEQ ID NO: 53, respectively; or HCDR1, HCDR2, and HCDR3 having 1, 2, or 3 amino acid differences from the amino acid sequences as set forth in SEQ ID NO: 52, SEQ ID NO: 39, and SEQ ID NO: 53, respectively; or
(IX) HCDR1, HCDR2 and HCDR3 having amino acid sequences as set forth in SEQ ID NO: 25, SEQ ID NO: 26 and SEQ ID NO: 37, respectively; or HCDR1, HCDR2, and HCDR3 having 1, 2, or 3 amino acid differences from the amino acid sequences as set forth in SEQ ID NO: 25, SEQ ID NO: 26, and SEQ ID NO: 37, respectively; and/or
the light chain variable region comprises:
(I) LCDR1, LCDR2, and LCDR3 having amino acid sequences as set forth in SEQ ID NO: 14, SEQ ID NO: 15, and SEQ ID NO: 16, respectively; or LCDR1, LCDR2 and LCDR3 having 1, 2 or 3 amino acid differences from the amino acid sequences as set forth in SEQ ID NO: 14, SEQ ID NO: 15 and SEQ ID NO: 16, respectively; or
(II) LCDR1, LCDR2, and LCDR3 having amino acid sequences as set forth in SEQ ID NO: 22, SEQ ID NO: 23, and SEQ ID NO: 24, respectively; or LCDR1, LCDR2 and LCDR3 having 1, 2 or 3 amino acid differences from the amino acid sequences as set forth in SEQ ID NO: 22, SEQ ID NO: 23 and SEQ ID NO: 24, respectively; or
(III) LCDR1, LCDR2, and LCDR3 having amino acid sequences as set forth in SEQ ID NO: 28, SEQ ID NO: 29, and SEQ ID NO: 30, respectively; or LCDR1, LCDR2 and LCDR3 having 1, 2 or 3 amino acid differences from the amino acid sequences as set forth in SEQ ID NO: 28, SEQ ID NO: 29 and SEQ ID NO: 30, respectively; or
(IV) LCDR1, LCDR2, and LCDR3 having amino acid sequences as set forth in SEQ ID NO: 34, SEQ ID NO: 35, and SEQ ID NO: 36, respectively; or LCDR1, LCDR2 and LCDR3 having 1, 2 or 3 amino acid differences from the amino acid sequences as set forth in SEQ ID NO: 34, SEQ ID NO: 35 and SEQ ID NO: 36, respectively; or
(V) LCDR1, LCDR2, and LCDR3 having amino acid sequences as set forth in SEQ ID NO: 41, SEQ ID NO: 15, and SEQ ID NO: 42, respectively; or LCDR1, LCDR2 and LCDR3 having 1, 2 or 3 amino acid differences from the amino acid sequences as set forth in SEQ ID NO: 41, SEQ ID NO: 15 and SEQ ID NO: 42, respectively; or
(VI) LCDR1, LCDR2, and LCDR3 having amino acid sequences as set forth in SEQ ID NO: 34, SEQ ID NO: 48, and SEQ ID NO: 49, respectively; or LCDR1, LCDR2 and LCDR3 having 1, 2 or 3 amino acid differences from the amino acid sequences as set forth in SEQ ID NO: 34, SEQ ID NO: 48 and SEQ ID NO: 49, respectively; or
(VII) LCDR1, LCDR2, and LCDR3 having amino acid sequences as set forth in SEQ ID NO: 34, SEQ ID NO: 51, and SEQ ID NO: 49 respectively; or LCDR1, LCDR2 and LCDR3 having 1, 2 or 3 amino acid differences from the amino acid sequences as set forth in SEQ ID NO: 34, SEQ ID NO: 51 and SEQ ID NO: 49, respectively; or
(VIII) LCDR1, LCDR2, and LCDR3 having amino acid sequences as set forth in SEQ ID NO: 54, SEQ ID NO: 55, and SEQ ID NO: 42, respectively; or LCDR1, LCDR2 and LCDR3 having 1, 2 or 3 amino acid differences from the amino acid sequences as set forth in SEQ ID NO: 54, SEQ ID NO: 55 and SEQ ID NO: 42, respectively; or
(IX) LCDR1, LCDR2, and LCDR3 having amino acid sequences as set forth in SEQ ID NO: 86, SEQ ID NO: 15, and SEQ ID NO: 16, respectively; or LCDR1, LCDR2 and LCDR3 having 1, 2 or 3 amino acid differences from the amino acid sequences as set forth in SEQ ID NO: 86, SEQ ID NO: 15 and SEQ ID NO: 16, respectively.

2. The antibody or antigen binding fragment thereof as claimed in claim 1, wherein the antibody or antigen-binding fragment thereof comprises:
(I) a heavy chain variable region comprising HCDR1, HCDR2, and HCDR3 having amino acid sequences as set forth in SEQ ID NO: 52, SEQ ID NO: 39, and SEQ ID NO: 53, respectively; and a light chain variable region comprising LCDR1, LCDR2, and LCDR3 having amino acid sequences as set forth in SEQ ID NO: 54, SEQ ID NO: 55, and SEQ ID NO: 42, respectively; or
(II) a heavy chain variable region comprising HCDR1, HCDR2, and HCDR3 having amino acid sequences as set forth in SEQ ID NO: 19, SEQ ID NO: 20, and SEQ ID NO: 21, respectively; and a light chain variable region comprising LCDR1, LCDR2, and LCDR3 having amino acid sequences as set forth in SEQ ID NO: 22, SEQ ID NO: 23, and SEQ ID NO: 24, respectively; or
(III) a heavy chain variable region comprising HCDR1 and HCDR2 having amino acid sequences as set forth in SEQ ID NO: 25, SEQ ID NO: 26, respectively, and HCDR3 as set forth in SEQ ID NO: 27 or SEQ ID NO: 37, respectively; and a light chain variable region comprising LCDR1, LCDR2, and LCDR3 having amino acid sequences as set forth in SEQ ID NO: 28, SEQ ID NO: 29, and SEQ ID NO: 30, respectively; or
(IV) a heavy chain variable region comprising HCDR1, HCDR2, and HCDR3 having amino acid sequences as set forth in SEQ ID NO: 31, SEQ ID NO: 32, and SEQ ID NO: 33, respectively; and a light chain variable region comprising LCDR1, LCDR2, and LCDR3 having amino acid sequences as set forth in SEQ ID NO: 34, SEQ ID NO: 35, and SEQ ID NO: 36, respectively; or
(V) a heavy chain variable region comprising HCDR1, HCDR2, and HCDR3 having amino acid sequences as set forth in SEQ ID NO: 38, SEQ ID NO: 39, and SEQ ID NO: 40, respectively; and a light chain variable region comprising LCDR1, LCDR2, and LCDR3 having amino acid sequences as set forth in SEQ ID NO: 41, SEQ ID NO: 15, and SEQ ID NO: 42, respectively; or
(VI) a heavy chain variable region comprising HCDR1 having amino acid sequences as set forth in SEQ ID NO: 31, HCDR2 as set forth in SEQ ID NO: 46 or SEQ ID NO: 50, and HCDR3 as set forth in SEQ ID NO:47, respectively; and a light chain variable region comprising LCDR1 having amino acid sequences as set forth in SEQ ID NO: 34, LCDR2 as set forth in SEQ ID NO: 48 or SEQ ID NO: 51, and LCDR3 as set forth in SEQ ID NO:49, respectively; or
(VII) a heavy chain variable region comprising HCDR1, HCDR2, and HCDR3 having amino acid sequences as set forth in SEQ ID NO: 11, SEQ ID NO: 12, and SEQ ID NO: 13, respectively; and a light chain variable region comprising LCDR1 having amino acid sequences as set forth in SEQ ID NO: 14 or SEQ ID NO:86, LCDR2 as set forth in SEQ ID NO: 15, and LCDR3 as set forth in SEQ ID NO: 16, respectively.

3. The antibody or antigen binding fragment thereof as claimed in claim 1 or 2, wherein the antibody or its antigen binding fragment comprises a heavy chain variable region and a light chain variable region:
(I) the heavy chain variable region comprises the amino acid sequence as set forth in SEQ ID NO: 61, or comprises an amino acid sequence having at least 95%, 96%, 97%, 98%, or 99% sequence identity to the amino acid sequence as set forth in SEQ ID NO: 61; and the light chain variable region comprising the amino acid sequence as set forth in SEQ ID NO: 62, or comprising an amino acid sequence having at least 95%, 96%, 97%, 98%, or 99% sequence identity to the amino acid sequence as set forth in SEQ ID NO: 62; or
(II) the heavy chain variable region comprises the amino acid sequence as set forth in SEQ ID NO: 65, or comprises an amino acid sequence having at least 95%, 96%, 97%, 98%, or 99% sequence identity to the amino acid sequence as set forth in SEQ ID NO: 65; and the light chain variable region comprising the amino acid sequence as set forth in SEQ ID NO: 66, or comprising an amino acid sequence having at least 95%, 96%, 97%, 98%, or 99% sequence identity to the amino acid sequence as set forth in SEQ ID NO: 66; or
(III) the heavy chain variable region comprises the amino acid sequence as set forth in SEQ ID NO: 67, or comprises an amino acid sequence having at least 95%, 96%, 97%, 98%, or 99% sequence identity to the amino acid sequence as set forth in SEQ ID NO: 67; and the light chain variable region comprising the amino acid sequence as set forth in SEQ ID NO: 68, or comprising an amino acid sequence having at least 95%, 96%, 97%, 98%, or 99% sequence identity to the amino acid sequence as set forth in SEQ ID NO: 68; or
(IV) the heavy chain variable region comprises the amino acid sequence as set forth in SEQ ID NO: 69, or comprises an amino acid sequence having at least 95%, 96%, 97%, 98%, or 99% sequence identity to the amino acid sequence as set forth in SEQ ID NO: 69; and the light chain variable region comprising the amino acid sequence as set forth in SEQ ID NO: 70, or comprising an amino acid sequence having at least 95%, 96%, 97%, 98%, or 99% sequence identity to the amino acid sequence as set forth in SEQ ID NO: 70; or
(V) the heavy chain variable region comprises the amino acid sequence as set forth in SEQ ID NO: 71, or comprises an amino acid sequence having at least 95%, 96%, 97%, 98%, or 99% sequence identity to the amino acid sequence as set forth in SEQ ID NO: 71; and the light chain variable region comprising the amino acid sequence as set forth in SEQ ID NO: 68, or comprising an amino acid sequence having at least 95%, 96%, 97%, 98%, or 99% sequence identity to the amino acid sequence as set forth in SEQ ID NO: 68; or
(VI) the heavy chain variable region comprises the amino acid sequence as set forth in SEQ ID NO: 72, or comprises an amino acid sequence having at least 95%, 96%, 97%, 98%, or 99% sequence identity to the amino acid sequence as set forth in SEQ ID NO: 72; and the light chain variable region comprising the amino acid sequence as set forth in SEQ ID NO: 73, or comprising an amino acid sequence having at least 95%, 96%, 97%, 98%, or 99% sequence identity to the amino acid sequence as set forth in SEQ ID NO: 73; or
(VII) the heavy chain variable region comprises the amino acid sequence as set forth in SEQ ID NO: 76, or comprises an amino acid sequence having at least 95%, 96%, 97%, 98%, or 99% sequence identity to the amino acid sequence as set forth in SEQ ID NO: 76; and the light chain variable region comprising the amino acid sequence as set forth in SEQ ID NO: 77, or comprising an amino acid sequence having at least 95%, 96%, 97%, 98%, or 99% sequence identity to the amino acid sequence as set forth in SEQ ID NO: 77; or
(VIII) the heavy chain variable region comprises the amino acid sequence as set forth in SEQ ID NO: 80, or comprises an amino acid sequence having at least 95%, 96%, 97%, 98%, or 99% sequence identity to the amino acid sequence as set forth in SEQ ID NO: 80; and the light chain variable region comprising the amino acid sequence as set forth in SEQ ID NO: 79, or comprising an amino acid sequence having at least 95%, 96%, 97%, 98%, or 99% sequence identity to the amino acid sequence as set forth in SEQ ID NO: 79; or
(IX) the heavy chain variable region comprises the amino acid sequence as set forth in SEQ ID NO: 82, or comprises an amino acid sequence having at least 95%, 96%, 97%, 98%, or 99% sequence identity to the amino acid sequence as set forth in SEQ ID NO: 82; and the light chain variable region comprising the amino acid sequence as set forth in SEQ ID NO: 83, or comprising an amino acid sequence having at least 95%, 96%, 97%, 98%, or 99% sequence identity to the amino acid sequence as set forth in SEQ ID NO: 83.

4. The antibody or antigen binding fragment thereof as claimed in claim 1 or 2, wherein the antibody or its antigen binding fragment comprises:
(I) a heavy chain variable region comprising the amino acid sequence as set forth in any one of SEQ ID NOs: 87-110, or comprising an amino acid sequence having at least 95%, 96%, 97%, 98%, or 99% sequence identity to the amino acid sequence as set forth in any one of SEQ ID NOs: 87-110; and a light chain variable region comprising the amino acid sequence as set forth in any one of SEQ ID NOs: 135-158, or comprising an amino acid sequence having at least 95%, 96%, 97%, 98%, or 99% sequence identity to an amino acid sequence as set forth in any one of SEQ ID NOs: 135-158; or
(II) a heavy chain variable region comprising the amino acid sequence as set forth in any one of SEQ ID NOs: 111-134, or comprising an amino acid sequence having at least 95%, 96%, 97%, 98%, or 99% sequence identity to the amino acid sequence as set forth in any one of SEQ ID NOs: 111-134; and a light chain variable region comprising the amino acid sequence as set forth in any one of SEQ ID NOs: 159-182, or comprising an amino acid sequence having at least 95%, 96%, 97%, 98%, or 99% sequence identity to an amino acid sequence as set forth in any one of SEQ ID NOs: 159-182; or
(III) a heavy chain variable region comprising the amino acid sequence as set forth in SEQ ID NO: 89, or comprising an amino acid sequence having at least 95%, 96%, 97%, 98%, or 99% sequence identity to the amino acid sequence as set forth in SEQ ID NO: 89; and a light chain variable region comprising the amino acid sequence as set forth in SEQ ID NO: 137, or comprising an amino acid sequence having at least 95%, 96%, 97%, 98%, or 99% sequence identity to an amino acid sequence as set forth in SEQ ID NO: 137.
(IV) a heavy chain variable region comprising the amino acid sequence as set forth in SEQ ID NO: 106, or comprising an amino acid sequence having at least 95%, 96%, 97%, 98%, or 99% sequence identity to the amino acid sequence as set forth in SEQ ID NO: 106; and a light chain variable region comprising the amino acid sequence as set forth in SEQ ID NO: 154, or comprising an amino acid sequence having at least 95%, 96%, 97%, 98%, or 99% sequence identity to an amino acid sequence as set forth in SEQ ID NO: 154.
(V) a heavy chain variable region comprising the amino acid sequence as set forth in SEQ ID NO: 114, or comprising an amino acid sequence having at least 95%, 96%, 97%, 98%, or 99% sequence identity to the amino acid sequence as set forth in SEQ ID NO: 114; and a light chain variable region comprising the amino acid sequence as set forth in SEQ ID NO: 162, or comprising an amino acid sequence having at least 95%, 96%, 97%, 98%, or 99% sequence identity to an amino acid sequence as set forth in SEQ ID NO: 162; or
(VI) a heavy chain variable region and a light chain variable region, wherein the heavy chain variable region and light chain variable region comprise any combination of amino acid sequences as shown in Table C.

5. The antibody or antigen binding fragment thereof as claimed in any one of claims 1-4, wherein the antibody is selected from the group consisting of mouse derived antibodies, chimeric antibodies, humanized antibodies, and fully human antibodies.

6. The antibody or antigen binding fragment thereof as claimed in any one of claims 1-4, wherein the antigen binding fragment is selected from the group consisting of Fab, Fab', F(ab')2, Fv, scFv, and sdAb.

7. The antibody or antigen binding fragment thereof as claimed in any one of claims 1-4, wherein the antibody or antigen binding fragment thereof is of any IgG subtype, such as IgG1, IgG2, IgG3, or IgG4, preferably IgG1 subtype.

8. The antibody or antigen binding fragment thereof as claimed in claim 4, wherein the antibody comprises or consists of the following sequences:
(I) a heavy chain having amino acid sequence as set forth in SEQ ID NO: 183 or having at least 95%, 96%, 97%, 98% or 99% sequence identity thereto, and a light chain having amino acid sequence as set forth in SEQ ID NO: 186 or having at least 95%, 96%, 97%, 98% or 99% sequence identity thereto; or
(II) a heavy chain having amino acid sequence as set forth in SEQ ID NO: 184 or having at least 95%, 96%, 97%, 98% or 99% sequence identity thereto, and a light chain having amino acid sequence as set forth in SEQ ID NO: 187 or having at least 95%, 96%, 97%, 98% or 99% sequence identity thereto; or
(III) a heavy chain having amino acid sequence as set forth in SEQ ID NO: 185 or having at least 95%, 96%, 97%, 98% or 99% sequence identity thereto, and a light chain having amino acid sequence as set forth in SEQ ID NO: 188 or having at least 95%, 96%, 97%, 98% or 99% sequence identity thereto.

9. The antibody or antigen binding fragment thereof as claimed in claim 8, wherein the antibody comprises or consists of the following sequence:
(I) a heavy chain having 1, 2, or 3 amino acid differences from amino acid sequence as set forth in SEQ ID NO: 183, and a light chain having amino acid sequence as set forth in SEQ ID NO: 186; or
(II) a heavy chain having 1, 2, or 3 amino acid differences from amino acid sequence as set forth in SEQ ID NO: 184, and a light chain having amino acid sequence as set forth in SEQ ID NO: 187; or
(III) a heavy chain having 1, 2, or 3 amino acid differences from amino acid sequence as set forth in SEQ ID NO: 185, and a light chain having amino acid sequence as set forth in SEQ ID NO: 188.

10. The antibody or antigen binding fragment thereof as claimed in claim 8, wherein the antibody comprises or consists of the following sequence:
(I) a heavy chain having amino acid sequence as set forth in SEQ ID NO: 191, SEQ ID NO: 192 or SEQ ID NO: 193 and a light chain having amino acid sequence as set forth in SEQ ID NO: 186; or
(II) a heavy chain having amino acid sequence as set forth in SEQ ID NO: 194, SEQ ID NO: 195 or SEQ ID NO: 196 and a light chain having amino acid sequence as set forth in SEQ ID NO: 187.

11. A polynucleotide molecule encoding an anti-BDCA2 antibody or antigen binding fragment thereof as claimed in any one of claims 1-10.

12. An expression vector comprising a polynucleotide molecule as claimed in claim 11, preferably the expression vector is a eukaryotic expression vector.

13. A host cell comprising the polynucleotide molecule as claimed in claim 11 or the expression vector as claimed in claim 12, preferably the host cell is a eukaryotic cell, more preferably a mammalian cell.

14. A method for preparing an anti-BDCA2 antibody or antigen binding fragment thereof as claimed in any one of claims 1-10, wherein the method comprises expressing the antibody or antigen binding fragment thereof in a host cell as claimed in claim 13 under conditions suitable for the expression of the antibody or antigen binding fragment thereof, and recovering the expressed antibody or antigen binding fragment thereof from the host cell.

15. An immunoconjugate comprising an anti-BDCA2 antibody or antigen binding fragment thereof as claimed in any one of claims 1-10, wherein the antibody or antigen binding fragment thereof is conjugated with at least one therapeutic or diagnostic agent, preferably an anti-inflammatory drug or immunosuppressant.

16. A pharmaceutical composition comprising an anti-BDCA2 antibody or antigen binding fragment thereof as claimed in any one of claims 1-10, a polynucleotide molecule as claimed in claim 11, an expression vector as claimed in claim 13, a host cell as claimed in claim 11, an immunoconjugate as claimed in claim 13, and a pharmaceutically acceptable carrier or excipient.

17. A method for inhibiting the over-release of cytokines from immune cells of a subject, comprising contacting the immune cells of the subject with an anti-BDCA2 antibody or an antigen binding fragment thereof as claimed in any one of claims 1-10, an immunoconjugate as claimed in claim 15, or a pharmaceutical composition as claimed in claim 16, preferably the immune cells are CpG-A-stimulated PBMC cells.

18. The use of an antibody or antigen binding fragment thereof as claimed in any one of claims 1-10, an immunoconjugate as claimed in claim 15, or a pharmaceutical composition as claimed in claim 16 in the preparation of a medicament for the treatment and/or prevention of BDCA2 mediated diseases, preferably inflammatory diseases; and more preferably, the inflammatory disease is selected from systemic lupus erythematosus, discoid lupus, lupus nephritis, epidermal lupus erythematosus, rheumatoid arthritis, inflammatory bowel disease, systemic sclerosis (scleroderma), psoriasis, type I diabetes, dermatomyositis, and polymyositis.

19. A kit comprising an antibody or antigen binding fragment thereof as claimed in any one of claims 1-10, an immunoconjugate as claimed in claim 15, or a pharmaceutical composition as claimed in claim 16.

20. A method for detecting the presence of BDCA2 in a sample using an antibody or antigen binding fragment thereof as claimed in any one of claims 1-10 or a detection composition containing said antibody or antigen binding fragment thereof, comprising the step of contacting the antibody or antigen binding fragment thereof or the detection composition containing said antibody or antigen binding fragment thereof with the sample.
